(19) Europäisches Patentamt / European Patent Office / Office européen des brevets

(11) EP 1 409 024 B1

(12) # EUROPÄISCHE PATENTSCHRIFT

(45) Veröffentlichungstag und Bekanntmachung des Hinweises auf die Patenterteilung:
**06.08.2008 Patentblatt 2008/32**

(21) Anmeldenummer: **02754904.7**

(22) Anmeldetag: **18.07.2002**

(51) Int Cl.:
***A61K 47/48*** *(2006.01)* ***A61K 49/00*** *(2006.01)*
***C07D 257/02*** *(2006.01)* ***C07F 5/00*** *(2006.01)*

(86) Internationale Anmeldenummer:
**PCT/EP2002/007999**

(87) Internationale Veröffentlichungsnummer:
**WO 2003/009874 (06.02.2003 Gazette 2003/06)**

(54) **MAKROCYCLISCHE METALLKOMPLEXE UND DEREN VERWENDUNG ZUR HERSTELLUNG VON KONJUGATEN MIT BIOMOLEKÜLEN**

MACROCYCLIC METAL COMPLEXES AND USE THEREOF FOR PRODUCTION OF CONJUGATES WITH BIOMOLECULES

COMPLEXES METALLIQUES MACROCYCLIQUES ET LEUR UTILISATION POUR LA PRODUCTION DE COMPOSES CONJUGUES AVEC DES BIOMOLECULES

(84) Benannte Vertragsstaaten:
**AT BE BG CH CY CZ DE DK EE ES FI FR GB GR IE IT LI LU MC NL PT SE SK TR**

(30) Priorität: **20.07.2001 DE 10135356**

(43) Veröffentlichungstag der Anmeldung:
**21.04.2004 Patentblatt 2004/17**

(73) Patentinhaber: **Bayer Schering Pharma Aktiengesellschaft**
**13353 Berlin (DE)**

(72) Erfinder:
- **PLATZEK, Johannes**
  **12621 Berlin (DE)**
- **SCHMITT-WILLICH, Heribert**
  **12161 Berlin (DE)**
- **MICHL, Günther**
  **15562 Rüdersdorf (DE)**
- **FRENZEL, Thomas**
  **12247 Berlin (DE)**
- **SÜLZLE, Detlev**
  **10589 Berlin (DE)**
- **BAUER, Hans**
  **12279 Berlin (DE)**
- **RADÜCHEL, Bernd**
  **13465 Berlin (DE)**
- **WEINMANN, Hanns-Joachim**
  **14129 Berlin (DE)**
- **SCHIRMER, Heiko**
  **13403 Berlin (DE)**

(74) Vertreter: **Teipel, Stephan et al**
**Lederer & Keller**
**Patentanwälte**
**Prinzregentenstrasse 16**
**80538 München (DE)**

(56) Entgegenhaltungen:
**EP-A- 0 353 450** **EP-A- 0 481 420**
**WO-A-01/52900** **WO-A-89/01476**
**WO-A-92/21379** **WO-A1-92/21379**
**WO-A1-95/31444** **WO-A1-99/05128**
**WO-A1-99/05145** **WO-A2-95/17910**
**WO-A2-03/013617** **DE-A- 19 603 033**
**US-A- 5 006 643** **US-A- 5 972 307**

- **COX J P L ET AL: "SYNTHESIS OF A KINETICALLY STABLE YTTRIUM-90 LABELLED MACROCYCLE-ANTIBODY CONJUGATE" JOURNAL OF THE CHEMICAL SOCIETY, CHEMICAL COMMUNICATIONS, CHEMICAL SOCIETY. LETCHWORTH, GB, Nr. 12, 1989, Seiten 797-798, XP001031170 ISSN: 0022-4936**
- **MISHRA A K ET AL: "A Convenient, Novel Approach for the Synthesis of Polyaza Macrocyclic Bifunctional Chelating Agents" TETRAHEDRON LETTERS, ELSEVIER SCIENCE PUBLISHERS, AMSTERDAM, NL, Bd. 37, Nr. 42, 14. Oktober 1996 (1996-10-14), Seiten 7515-7518, XP004068836 ISSN: 0040-4039**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- ALBERT R ET AL: "Direct Synthesis of [DOTA-DPhe]-Octreotide and [DOTA-DPhe, Tyr]-Octreotide (SMT487): Two Conjugates for Systemic Delivery of Radiotherapeutical Nuclides to Somatostatin Receptor Positive Tumors in Man" BIOORGANIC & MEDICINAL CHEMISTRY LETTERS, OXFORD, GB, Bd. 8, Nr. 10, 19. Mai 1998 (1998-05-19), Seiten 1207-1210, XP004137048 ISSN: 0960-894X
- LI ET AL: "A Calcium-Sensitive Magnetic Resonance Imaging Contrast Agent" JOURNAL OF THE AMERICAN CHEMICAL SOCIETY, AMERICAN CHEMICAL SOCIETY, WASHINGTON, DC, US, Bd. 121, Nr. 6, 1999, Seiten 1413-1414, XP002115324 ISSN: 0002-7863
- HEPPELER A ET AL: "RADIOMETAL-LABELLED MACROCYCLIC CHELATOR-DERIVATISED SOMATOSTATIN ANALOGUE WITH SUPERB TUMOUR-TARGETING PRORPERTIES AND POTENTIAL FOR RECEPTOR-MEDIATED INTERNAL RADIOTHERAPY" CHEMISTRY - A EUROPEAN JOURNAL, VCH PUBLISHERS, US, Bd. 5, Nr. 7, Juli 1999 (1999-07), Seiten 1974-1981, XP000829499 ISSN: 0947-6539
- PULUKKODY K P: "Synthesis of Charged and Uncharged Complexes of Gadolinium and Yttrium with Cyclic polyazaphosphinic Acid Ligands for in vivo Applications" JOURNAL OF THE CHEMICAL SOCIETY, PERKIN TRANSACTIONS 2, CHEMICAL SOCIETY. LETCHWORTH, GB, Nr. 4, 1993, Seiten 605-620, XP002146570 ISSN: 1472-779X
- GARRITY M L ET AL: "A NEW SYNTHETIC ROUTE TO 2-(P-NITROBENZYL)-1,4,7,10-TETRAAZACYCLODODECANE" TETRAHEDRON LETTERS, ELSEVIER SCIENCE PUBLISHERS, AMSTERDAM, NL, Bd. 34, Nr. 35, 1993, Seiten 5531-5534, XP001042259 ISSN: 0040-4039
- AIME S ET AL: "Non-covalent conjugates between cationic polyamino acids and GdIII chelates: a route for seeking accumulation of MRI-contrast agents at tumor targeting sites." CHEMISTRY (WEINHEIM AN DER BERGSTRASSE, GERMANY) GERMANY 14 JUL 2000, Bd. 6, Nr. 14, 14. Juli 2000 (2000-07-14), Seiten 2609-2617, XP002220623 ISSN: 0947-6539
- CHAPPELL L L ET AL: "Improved synthesis of the bifunctional chelating agent 1,4,7,10-tetraaza-N-(1-carboxy-3-(4-nitrop henyl)propyl)-N',N'', N'''- tri s(acetic acid)cyclododecane (PA-DOTA)." BIOORGANIC & MEDICINAL CHEMISTRY. ENGLAND NOV 1999, Bd. 7, Nr. 11, November 1999 (1999-11), Seiten 2313-2320, XP002220624 ISSN: 0968-0896
- CORSI D M ET AL: "Inulin as a carrier for contrast agents in magnetic resonance imaging." CHEMISTRY (WEINHEIM AN DER BERGSTRASSE, GERMANY) GERMANY 5 JAN 2001, Bd. 7, Nr. 1, 5. Januar 2001 (2001-01-05), Seiten 64-71, XP002220625 ISSN: 0947-6539
- REANY OFER ET AL: "A model system using modulation of lanthanide luminescence to signal Znin competitive aqueous media" J CHEM SOC PERKIN TRANS 2;JOURNAL OF THE CHEMICAL SOCIETY. PERKIN TRANSACTIONS 2 SEPTEMBER 2000, Nr. 9, September 2000 (2000-09), Seiten 1819-1831, XP002220626
- BEEBY ANDREW ET AL: "The efficient intramolecular sensitisation of terbium(III) and europium(III) by benzophenone-containing ligands" J CHEM SOC PERKIN TRANS 2; JOURNAL OF THE CHEMICAL SOCIETY. PERKIN TRANSACTIONS 2 JULY 2000, Nr. 7, Juli 2000 (2000-07), Seiten 1281-1283, XP002220627
- HOVLAND RAGNAR ET AL: "Gadolinium DO3A derivatives mimicking phospholipids; Preparation and in vitro evaluation as pH responsive MRI contrast agents" J CHEM SOC PERKIN TRANS 2;JOURNAL OF THE CHEMICAL SOCIETY. PERKIN TRANSACTIONS 2 2001, Nr. 6, 2001, Seiten 929-933, XP002220628
- COX J P L ET AL: "SYNTHESIS OF C AND N-FUNCTIONALIZED DERIVATIVES OF 1 4 7 TRIAZACYCLONONANE-1 4 7-TRIYLTRIACETIC ACID NOTA 1 4 7 10 TETRAAZACYCLODODECANE-1 4 7 10-TETRAYLTETRAACETIC ACID DOTA AND DIETHYLENETRIAMINEPENTAACETIC ACID DTPA BIFUNCTIONAL COMPLEXING AGENTS FOR THE DERIVATIZATION OF ANTIBODIES" JOURNAL OF THE CHEMICAL SOCIETY PERKIN TRANSACTIONS I, Nr. 9, 1990, Seiten 2567-2576, XP001119344 ISSN: 0300-922X
- OGAN M D ET AL: "A specific radioimmunoassay for the measurement of gadoteridol, a contrast agent for magnetic resonance imaging in biological fluids." JOURNAL OF PHARMACEUTICAL SCIENCES. UNITED STATES MAY 1993, Bd. 82, Nr. 5, Mai 1993 (1993-05), Seiten 475-479, XP001119429 ISSN: 0022-3549
- GRIERSON JOHN R ET AL: "Synthesis of a DOTA conjugated peptide and its labeling with Ce-141." JOURNAL OF LABELLED COMPOUNDS AND RADIOPHARMACEUTICALS, Bd. 40, Dezember 1997 (1997-12), Seiten 512-514, XP001119556 XIIth International Symposium on Radiopharmaceutical Chemistry;Uppsala, Sweden; June 15-19, 1997 ISSN: 0362-4803
- MIER W ET AL: "Synthesis and isolation of active esters of DOTA." JOURNAL OF LABELLED COMPOUNDS AND RADIOPHARMACEUTICALS, Bd. 44, Nr. Supplement 1, Mai 2001 (2001-05), Seiten S814-S816, XP001119526 Fourteenth International Symposium on Radiopharmaceutical Chemistry;Interlaken, Switzerland; June 10-15, 2001 ISSN: 0362-4803




(Forts. nächste Seite)

- CHANG C A: "Selectivity of macrocyclic aminocarboxylates for alkaline-earth metal ions and stability of their complexes" JOURNAL OF THE CHEMICAL SOCIETY, DALTON TRANSACTIONS, 1996, Seiten 2347-2350, XP001119339
- FORMANOVSKY A A ET AL: "One-stage monosubstitution in cyclen - two novel examples" SYNTHETIC COMMUNICATIONS, Bd. 26, Nr. 8, 1996, Seiten 1595-1603, XP001119430
- ANELLI P L ET AL: "A new approach to hepatospecific MRI contrast agents: gadolinium complexes conjugated to iodinated synthons" GAZZETTA CHIMICA ITALIANA, Bd. 126, Nr. 2, 1996, Seiten 89-97, XP001119427
- REANY O ET AL: "Selective signalling of zinc ions by modulation of terbium luminescence" CHEMICAL COMMUNICATIONS, 2000, Seiten 473-474, XP002220629
- LI M ET AL: "Luminescent polyaminocarboxylate chelates of terbium and europium: the effect of chelate structure" JOURNAL OF THE AMERICAN CHEMICAL SOCIETY, Bd. 117, Nr. 31, 1995, Seiten 8132-8138, XP002220630 ISSN: 0002-7863
- LOWE M.P. ET AL: 'PH-DEPENDENT MODULATION OF RELAXIVITY AND LUMINESCENCE IN MACROCYCLIC GADOLINIUM AND EUROPIUM COMPLEXES BASED ON REVERSIBLE INTRAMOLECULAR SULFONAMIDE LIGATION' JOURNAL OF THE AMERICAN CHEMICAL SOCIETY Bd. 123, Nr. 31, 17 Juli 2001, Seiten 7601 - 7609, XP001147822 ISSN: 0002-7863
- LOWE M.P. ET AL: 'pH Switched sensitisation of europium(III) by a dansyl group' INORGANICA CHIMICA ACTA Bd. 317, Nr. 1,2, 28 Mai 2001, Seiten 163 - 173, XP001042289 ISSN: 0020-1693
- WOODS M. ET AL: 'CORRELATION OF WATER EXCHANGE RATE WITH ISOMERIC COMPOSITION IN DIASTEREOISOMERIC GADOLINIUM COMPLEXES OF TETRA (CARBOXYETHYL)DOTA AND RELATED MACROCYCLIC LIGANDS' JOURNAL OF THE AMERICAN CHEMICAL SOCIETY Bd. 122, Nr. 40, 11 Oktober 2000, Seiten 9781 - 9792, XP009058154 ISSN: 0002-7863
- CHEN D.-F. ET AL: 'SYNTHESIS AND CHARACTERIZATION OF RARE EARTH COMPLEX WITH POLYAZA POLYCARBOXYLIC MACROCYCLE NA[LN(DOTMA)(H2O)].2H2O' GAODENG XUEXIAO HUAXUE XUEBAO (CHEMICAL JOURNAL OF CHINESE UNIVERSITIES) Bd. 17, Nr. 8, August 1996, Seiten 1179 - 1181, XP009058156 ISSN: 0251-0790
- KANG S.I. ET AL: 'SYNTHESIS, CHARACTERIZATION, AND CRYSTAL STRUCTURE OF THE GADOLINIUM(III) CHELATE OF (IR,4R,7R)-ALPHA,ALPHA',ALPHA-TRIMETHYL-1,4 ,7,10-TETRAAZACYCLODODECANE-1,4,7-TRIACETIC ACID (DO3MA)' INORGANIC CHEMISTRY Bd. 32, Nr. 13, 1993, Seiten 2912 - 2918, XP009058169 ISSN: 0020-1669

## Beschreibung

Die Erfindung betrifft den in den Patentansprüchen gekennzeichneten Gegenstand, d.h. makrocyclische Metallkomplexe sowie deren Verwendung zur Herstellung von Konjugaten mit Biomolekülen. Die Konjugate eignen sich für die Herstellung von Kontrastmitteln für die NMR- und Radiodiagnostik sowie von Mitteln für die Radiotherapie.

Voraussetzung für eine gezielte und erfolgreiche Therapie ist eine exakte Diagnose. Gerade auf dem diagnostischen Gebiet haben die Möglichkeiten in den vergangenen Jahren sehr stark zugenommen, wobei beispielsweise die NMR-Diagnostik in der Lage ist, praktisch jedes anatomische Detail selektiv und mit großer Genauigkeit darzustellen. In vielen Fällen werden die entsprechenden Strukturen aber erst durch die Anwendung von Kontrastmitteln sichtbar. Darüber hinaus besteht die Möglichkeit, die Kontrastmittel so auszugestalten, daß sie sich selektiv in den gewünschten Zielstrukturen anreichern. Hierdurch läßt sich die Genauigkeit der Bildgebung bei gleichzeitiger Verringerung der benötigten Kontrastmittelmenge erhöhen.

Als Kontrastmittel für die NMR-Diagnostik eigenen sich Chelatkomplexe von paramagnetischen Metallen. Theorie und Anwendung von Gadolinium(III) Chelaten als NMR-Kontrastmittel sind in einem Übersichtsartikel von P. Caravan et al. in Chem. Rev. 1999, 99, 2293-2352 ausführlich erläutert.

Die Bildintensität im Protonen-NMR wird im wesentlichen durch die Wasserprotonen bestimmt. Sie hängt von den Kernrelaxationszeiten ab. Komplexe von paramagnetischen Übergangsmetallen und Lanthanoiden verkürzen die Relaxationszeiten von benachbarten Protonen durch dipolare Wechselwirkungen. Die paramagnetischen Kontrastmittel werden nicht direkt detektiert, sondern es erfolgt eine indirekte Detektion auf der Grundlage der Tatsache, daß die Kontrastmittel Relaxationszeiten von benachbarten Protonen wie Wasserprotonen verändern können. Aufgrund ihrer hohen magnetischen Momente und Relaxationseffizienz sind $Gd^{3+}$, $Fe^{3+}$ und $Mn^{2+}$ bevorzugte paramagnetische Metallkationen in der NMR-Diagnostik.

Eine wichtige physikalische Größe, welche das Relaxationsverhalten von Protonen beschreibt, ist die longitudinale Relaxationszeit $T_1$. Gewebe mit kurzen Relaxationszeiten $T_1$ liefern im allgemeinen Bilder höherer Intensität als solche mit längeren Relaxationszeiten. Trägt man für ein bestimmtes paramagnetisches Ion den Reziprokwert der gemessenen Relaxationszeit $T_1$ in Abhängigkeit von der Konzentration c auf, so erhält man Geraden der Steigung R. Diese Steigung nennt man auch Relaxivität, welche ein Maß für das Vermögen des entsprechenden paramagnetischen Ions ist, die Relaxationszeit der benachbarten Protonen zu verkürzen.

Die Anwendung von Radiopharmaka für diagnostische und therapeutische Zwecke ist ebenfalls seit langem im Bereich der biologischen und medizinischen Forschung bekannt. Insbesondere werden Radiopharmaka dazu benutzt, um bestimmte Strukturen wie beispielsweise das Skelett, Organe oder Gewebe darzustellen. Die diagnostische Anwendung setzt den Gebrauch solcher radioaktiver Mittel voraus, die sich nach Applikation spezifisch in den Strukturen im Patienten anreichern, die untersucht werden sollen. Diese sich lokal anreichernden radioaktiven Mittel können dann mittels geeigneter Detektoren, wie beispielsweise Szintilations-Kameras oder anderer geeigneter Aufnahmeverfahren, aufgespürt, geplottet oder szintigraphiert werden. Die Verteilung und relative Intensität des detektierten radioaktiven Mittels kennzeichnet den Ort einer Struktur, in dem sich das radioaktive Mittel befindet, und kann die Anwesenheit von Anomalien in Strukturen und Funktionen, pathologische Veränderungen etc. darstellen.

In ähnlicher Weise können Radiopharmaka als therapeutische Mittel angewendet werden, um bestimmte krankhafte Gewebe oder Bereiche zu bestrahlen. Solche Behandlung erfordert die Herstellung radioaktiver therapeutischer Mittel, die sich in bestimmten Strukturen, Organen oder Geweben anreichern.

Aufgrund ihrer zum Teil relativ hohen Giftigkeit werden die benötigten Ionen im Regelfall nicht in Form von wasserlöslichen Salzen verabreicht, sondern in Form von Chelatkomplexen. Diese können praktisch unverändert vom Körper ausgeschieden werden. Je kleiner die Komplexe in Lösung sind, desto geringer ist ihr Trägheitsmoment und desto schneller drehen sie sich in Lösung (Tumbling Motion Time). Je schneller ein Komplex rotiert, desto geringer ist seine Relaxivität. Die Relaxivität steigt somit mit der Molekularmasse des gesamten Komplexes. Eine hohe Molekularmasse kann man durch Anbindung an Makromoleküle erreichen. Ein gutes NMR-Kontrastmittel zeichnet sich unter anderem dadurch aus, daß es einen großen Wert für die Relaxivität hat.

Konjugate aus Gd-DTPA (Diethylentriaminpentaessigsäure) mit Albumin sind beispielsweise von M.D. Ogan et al. in Invest. Radiol. 1987, 22, 665-671 und U. Schmiedl et al. in Radiology 1987, 162. 205-210 beschrieben. Konjugate aus makrocyclischem Metallkomplexen und Biomolekülen sind in der WO 95/31444 offenbart. Zur Verbesserung der Selektivität von Kontrastmitteln schlägt die WO 01/08712 ein Kontrastmittel vor, das mindestens zwei Metallchelateinheiten als bildverbessernde Gruppen und mindestens zwei "Zielbindungseinheiten" zur Bindung des Kontrastmittelmoleküls an das gewünschte Zielmolekül oder Zielorgan im Körper umfaßt.

Große Kontrastmittelmoleküle mit hoher Molmasse werden gemäß der WO 97/02051 durch Einbau von makrocyclischen Metallkomplexen in Kaskadenpolymere erhalten.

Tetraazacyclododecantetraessigsäurederivate hoher Stabilität und guter Löslichkeit aufgrund fehlender Ladung, die sich zur Anbindung an Biomoleküle eignen, sind in dem EP-A-0 565 930 beschrieben.

M. P. Lowe et al. offenbaren in J. Am. Chem. Soc. 2001, 123, 7601 makrocyclische Gadolinium- und Europiumkomplexe mit einer Arylsulfonamidseitengruppe.

M. P. Lowe et al. offenbaren in Inorganica Chemica Acta 2001, 317, 163 makrocyclische Europiumkomplexe, die ebenfalls eine Arylsulfonamidseitengruppe enthalten.

M. Woods et al. offenbaren in J. Am. Chem. Soc. 2000, 122, 9781 Komplexe von Tetra(carboxyethyl)-Derivaten des 1,4,7,10-Tetraazacyclododecans.

Die Synthese von $\alpha,\alpha',\alpha''$-Tris(methyl)-1,4,7,10-tetraazacyclododecan-1,4,7,10-tetraessigsäure wird in WO 99/05145 beschrieben.

Die WO 99/05128 offenbart die Synthese von $\alpha,\alpha',\alpha'',\alpha'''$-Tetramethyl-1,4,7,10-tetraazacyclododecan-1,4,7,10-tetraessigsäure.

Chemical Journal of Chinese Universities 1996, 17, 1179 beschreibt $Na[Ln(DOTMA)(H_2O)]\cdot 2H_2O$.

Die WO 95/17910 offenbart Yb(III)-Komplexe von $\alpha,\alpha',\alpha'',\alpha'''$-Tetramethyl-1,4,7,10-tetraazacyclododecan-1,4,7,10-tetraessigsäure (DOTMA).

S.I. Kang et al. beschreiben in Inorg. Chem. 1993, 32, 2912 Gadoliniumkomplexe von DOTMA.

Die WO 92/21379 offenbart 1-{$\alpha$-[2-(4-Aminophenyl)ethyl]essigsäure}-1,4,7,10-tetraazacyclododecan-4,7,10-tris(methylessigsäure) (PA-DOTMA).

Die EP-A-0 481 420 offenbart Gd-DOTMA.

Die EP-A-0 353 450 offenbart makrocyclische funktionalisierte Polyaminocarboxylatkomplexbildner.

Die vorstehend beschriebene Anbindung von makrocyclischen Metallkomplexen an Biomoleküle ermöglicht sowohl eine Erhöhung der Relaxivität als auch der Selektivität des Kontrastmittels. Je höher die Relaxivität des Kontrastmittels ist, desto weniger Kontrastmittel muß dem Patienten verabreicht werden und desto höher ist die Kontrastgebung im Bild. Aus diesem Grund ist es weiterhin wünschenswert, NMR-Kontrastmittel mit möglichst hoher Relaxivität zur Verfügung zu stellen.

Eine Aufgabe der vorliegenden Erfindung besteht somit darin, verbesserte Kontrastmittel für die NMR- und Radiodiagnostik sowie Mittel für die Radiotherapie zur Verfügung zu stellen. Insbesondere sollen diese NMR-Kontrastmittel eine möglichst hohe Relaxivität aufweisen und sich möglichst selektiv an einem gewünschten Ort im Körper anreichern.

Es wurde nun gefunden, daß diese Aufgabe überraschend dadurch gelöst werden kann, daß ein 1,4,7,10-Tetraazacyclododecanmakrocyclus mit speziellen Liganden versehen wird. Die neuen Qualitäten der erfindungsgemäßen Verbindungen werden deutlich, wenn sie an Biomoleküle gebunden werden. Durch die spezielle Ligandierung des Makrocyclus wird die Relaxivität des erhaltenen Kontrastmittels erhöht und außerdem wird ein Feintuning der Relaxivität für eine gewünschte Anwendung möglich.

Die vorliegende Erfindung betrifft somit Verbindungen der Formel I

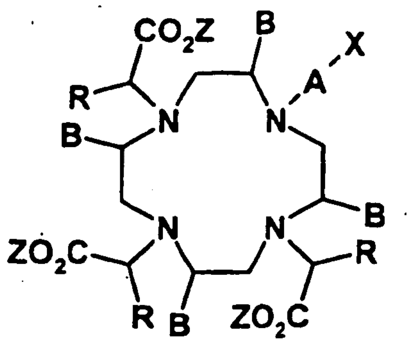

I

worin

Z ein Wasserstoffatom darstellt oder mindestens zwei Z ein Metallionenäquivalent darstellen,

B ein Wasserstoffatom oder einen $C_{1-4}$-Alkylrest darstellt,

R ein Wasserstoffatom oder einen geraden, verzweigten oder cyclischen, gesättigten oder ungesättigten $C_{1-10}$-Alkyl- oder Arylrest darstellt, der gegebenenfalls mit einer Carboxylgruppe, $-SO_3H$ oder $-PO_3H_2$ substituiert ist, und wobei die Alkylkette des $C_{1-10}$-Alkylrestes gegebenenfalls eine Arylgruppe und/oder 1-2 Sauerstoffatome enthält, mit der Maßgabe, daß die Reste B und R nicht beide gleichzeitig Wasserstoffatome darstellen.

A einen Rest A'-U darstellt, worin A' an das Stickstoffatom des makrocyclischen Rings und U an X gebunden ist, und wobei A'

a) eine Gruppe der Formel

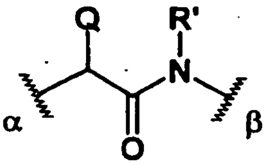

worin Q ein Wasserstoffatom, einen $C_{1-10}$-Alkylrest, der gegebenenfalls mit einer Carboxylgruppe substituiert ist, oder einen Arylrest darstellt, der gegebenenfalls mit einer Carboxylgruppe, einer $C_{1-15}$-Alkoxygruppe, einer Aryloxygruppe oder einem Halogenatom substituiert ist, und R' wie vorstehend R definiert ist, aber unabhängig gewählt werden kann, oder

b) eine Gruppe der Formel

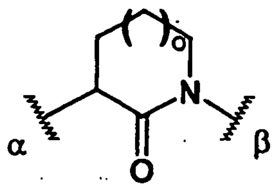

worin o 0 oder 1 ist, und der Ring gegebenenfalls mit einem Benzolring anelliert ist, wobei dieser Benzolring, falls vorhanden, mit einer Methoxy- oder Carboxylgruppe, $-SO_3H$ oder - $PO_3H_2$ substituiert sein kann, darstellt, wobei in den Gruppen unter a) und b) die an den mit 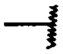 gekennzeichneten Positionen an die benachbarten Gruppen gebunden sind und worin die Position α an ein Stickstoffatom des makrocyclischen Rings und die Position β an U gebunden ist und

U eine gerade oder verzweigte, gesättigte oder ungesättigte $C_{1-30}$-Kohlenwasserstoffkette darstellt, die gegebenenfalls 1-3 Sauerstoffatome, 1-3 Stickstoffatome und/oder 1-3 -NR''-Reste, worin R'' wie vorstehend R definiert ist, aber unabhängig gewählt werden kann, enthält und bei der gegebenenfalls 1-3 Kohlenstoffatome als Carbonylgruppen vorliegen, wobei die Kette oder ein Teil der Kette ringförmig angeordnet sein kann mit der Maßgabe, daß A' und U zusammen so ausgestaltet sind, daß X über mindestens 3 Atome mit dem Stickstoffatom, an das A' gebunden ist, verbunden ist; und

X eine Gruppe darstellt, die mit einem Biomolekül eine Reaktion eingehen kann, sowie deren Salze

und deren Verwendung zur Herstellung eines Konjugats mit einem Biomolekül.

Eine entsprechende makrocyclische Verbindung worin die vier Stickstoffatome des makrocyclischen Rings jeweils mit dem Substituenten $-CH(CO_2H)CH_2CH_2CO_2H$ substituiert sind, ist in P. Caravan et al., Chem. Rev. 1999, 99, 2293-2352 offenbart. Eine mögliche Verwendung dieser Verbindung zur Herstellung von Konjugaten mit Biomolekülen wird jedoch nicht offenbart. Die WO 97/02051 offenbart makrocyclische Verbindungen, in denen

A ein Rest $-CH(R^4)-CO-NR^2-U^6-$ ist, als Zwischenverbindungen für die Herstellung von Kaskadenpolymeren. Die EP-A-0 565 930 offenbart macrocyclische Verbindungen, in denen A ein Rest $-CH(R_3)-C(O)-NH-(CH_2)_{1-6}-NH-D-$ ist. Eine Erhöhung der Relaxivität durch bestimmte Substituenten wird nicht offenbart. Diese Verbindungen sind dementsprechend bei der Definition der Verbindung der Formel I in Anspruch 1 ausgenommen.

Wenn nicht anders angegeben wird vorliegend unter "Alkylrest" ein gesättigter oder ungesättigter, gradkettiger oder verzweigter oder cyclischer Alkylrest mit der angegebenen Anzahl von Kohlenstoffatomen verstanden. Wenn dieser Rest weitere Gruppen oder Atome enthalten kann, wird darunter vorliegend verstanden, daß die weiteren Gruppen oder Atome zusätzlich zu den bereits vorhandenen Atomen des Restes vorliegen und an einer beliebigen Position des Restes einschließlich der terminalen Positionen eingefügt sein können.

Unter "Aryl" wird vorliegend vorzugsweise Phenyl, Bisphenyl, Pyridyl, Furanyl, Pyrrolyl und Imidazolyl verstanden. Besonders bevorzugt ist Phenyl.

Unter "Kohlenwasserstoffkette", die ganz oder teilweise ringförmig angeordnet sein kann, wird vorliegend vorzugsweise eine Kohlenwasserstoffkette wie beispielsweise eine Alkylkette verstanden, die beispielsweise einen aliphatischen oder aromatischen gegebenenfalls heterocyclischen 5- oder 6-Ring (z.B. Phenyl(en), Pyridyl(en) oder Cyclohexyl (en)) umfassen kann oder daraus besteht.

In der erfindungsgemäßen Verbindung der Formel I sind drei der vier Stickstoffatome des makrocyclischen

Rings mit gegebenenfalls substituierten Essigsäure- bzw. Carboxylatmethylresten substituiert. Diese Reste tragen zur Koordination bzw. zum Ladungsausgleich eines koordinierten Metallions bei. Daher steht Z entweder für ein Wasserstoffatom oder ein Metallionenäquivalent.

Die- Essigsäure- bzw. Carboxylatmethylreste an drei der Stickstoffatome des makrocyclischen Rings können zusätzlich einen Substituenten R aufweisen. Darüber hinaus kann der makrocyclische Ring an vier seiner Kohlenstoffatome einen weiteren Substituenten B aufweisen. Eine Besonderheit der erfindungsgemäßen Verbindungen besteht darin, daß B und R nicht beide gleichzeitig Wasserstoffatome darstellen können, d.h. daß der makrocyclische Ring entweder direkt an seinen Ringatomen und/oder an den Essigsäure- bzw. Carboxylatmethylsubstituenten seiner Stickstoffatome weitere Substituenten aufweisen muß. Durch die geeignete Wahl dieser zusätzlichen Substituenten erfolgt das gewünschte Feintuning der Relaxivität eines unter Verwendung der erfindungsgemäßen Verbindung hergestellten Kontrastmittels.

B kann ein Wasserstoffatom oder ein $C_{1-4}$-Alkylrest sein. Bevorzugte $C_{1-4}$-Alkylreste sind Methyl, Ethyl und iso-Propyl.

Falls in den erfindungsgemäßen Verbindungen der Formel I B ein Wasserstoffatom ist, steht R für einen geraden, verzweigten und/oder cyclischen, gesättigten oder ungesättigten $C_{1-10}$-Alkyl- (vorzugsweise $C_{5-10}$-Alkyl-) oder Arylrest, der gegebenenfalls mit einer Carboxylgruppe, $-SO_3H$ oder $-PO_3H_2$ substituiert ist, und wobei die Alkylkette des $C_{1-10}$-Alkylrestes gegebenenfalls eine Arylgruppe und/oder 1-2 Sauerstoffatome enthält. Als Alkylreste sind geradkettige oder verzweigte, vorzugsweise gesättigte $C_{1-10}$- und insbesondere $C_{1-4}$-Alkylreste, wie Methyl, Ethyl, n-Propyl, iso-Propyl, n-Butyl, iso-Butyl und tert.-Butyl, sowie Cyclohexyl bevorzugt. Alternativ sind geradkettige, verzweigte oder cyclische, vorzugsweise gesättigte $C_{5-10}$-Alkylreste wie Pentyl, Hexyl, Cyclohexyl, Heptyl, Octyl, Nonyl und Decyl, bevorzugt. Der $C_{1-10}$-Alkylrest für R kann gegebenenfalls mit einer Carboxylgruppe, $-SO_3H$ oder $-PO_3H_2$ substituiert sein. Bevorzugte Beispiele derartiger substituierter Alkylgruppen sind $-CH_2-COOH$ und $-C(CH_3)_2-COOH$. Darüber hinaus kann die Alkylkette des $C_{1-10}$-Alkylrestes eine Arylgruppe und/oder 1-2 Sauerstoffatome enthalten. Die Arylgruppe und die Sauerstoffatome können an beliebiger Stelle innerhalb der Alkylkette vorliegen. Die Arylgruppe kann zudem auch endständig an der Alkylkette angeordnet sein und zusammen mit einem Sauerstoffatom eine Aryloxygruppe bilden. Als Arylgruppe eignet sich insbesondere eine Phenylgruppe.

Eine bevorzugte Alkylkette für R, die gegebenenfalls eine Arylgruppe und 1-2 Sauerstoffatome enthält, ist ein Rest der Formel $-(CH_2)_m-(O)_n-(\text{Phyenylen})_p-Y$, worin m eine ganze Zahl von 1-5 ist, n 0 oder 1 ist, p 0 oder 1 ist und Y ein Wasserstoffatom, ein Methoxyrest, eine Carboxylgruppe, $-SO_3H$ oder $-PO_3H_2$ ist. Der Substituent Y befindet sich dabei vorzugsweise in para-Position.

Der Arylrest für R ist vorzugsweise ein Phenylrest, der gegebenenfalls mit einer Carboxylgruppe, $-SO_3H$ oder $-PO_3H_2$ substituiert ist.

Vorzugsweise steht R, wenn B ein Wasserstoffatom ist, für Isopropyl, Isobutyl, tert.-Butyl, einen geradkettigen oder verzweigten $C_{5-10}$-Alkylrest, Cyclohexyl, $-CH_2-COOH$, $-C(CH_3)_2-COOH$, einen Phenylrest oder einen Rest der Formel $-(CH_2)_m-(O)_n-(\text{Phenylen})_p-Y$, worin m eine ganze Zahl von 1 bis 5 ist, n 0 oder 1 ist, p 0 oder 1 ist und Y ein Wasserstoffatom, einen Methoxyrest, eine Carboxylgruppe, $-SO_3H$ oder $-PO_3H_2$ darstellt, und besonders bevorzugt für Isopropyl, Cyclohexyl oder Phenyl.

Der substituierte makrocyclische Ring der Verbindung der Formel I kann mittels einer Gruppe X, die mit einem Biomolekül eine Reaktion eingehen kann, über einen Spacer A an ein Biomolekül angebunden werden.

Der Spacer muß mindestens drei Atome und vorzugsweise mindestens vier Atome in einer Kette zwischen dem Stickstoffatom des makrocyclischen Rings und X aufweisen. Als Kette von Atomen wird dabei die kürzeste Verbindung zwischen dem Stickstoffatom des makrocyclischen Rings und X auch über einen Ring hinweg verstanden. Im Sinne dieser Definition würde beispielsweise eine para-Phenylengruppe als Spacer mit vier Atomen in einer Kette angesehen und eine meta-Phenylengruppe als Spacer mit drei Atomen in einer Kette. Bei der Bestimmung der Länge der Atomkette werden Kohlenstoff-, Stickstoff- und Sauerstoffatome gleichmäßig jeweils als ein Atom gerechnet. Substituenten an diesen Atomen oder Seitenketten gehören nicht zur Anzahl der Atome innerhalb der Kette.

Der Spacer A stellt einen Rest A'-U dar, worin A' an das Stickstoffatom des makrocyclischen Rings und U an X gebunden ist. Hierin ist A'

a) eine Gruppe der Formel

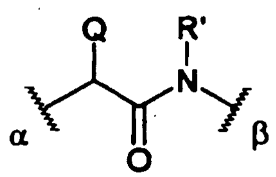

worin Q ein Wasserstoffatom, einen $C_{1-10}$-Alkylrest, der gegebenenfalls mit einer Carboxylgruppe substituiert ist, oder einen Arylrest darstellt, der gegebenenfalls mit einer Carboxylgruppe, einer $C_{1-15}$-Alkoxygruppe, einer Aryloxygruppe oder einem Halogenatom substituiert ist, und R' wie R definiert ist, aber unabhängig gewählt werden kann, oder

b) eine Gruppe der Formel

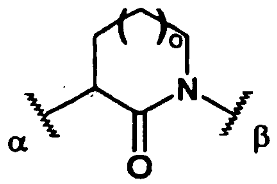

worin o 0 oder 1 ist, und der Ring gegebenenfalls mit einem Benzolring anelliert ist, wobei dieser Benzolring, falls vorhanden, mit einer Methoxy- oder Carboxylgruppe, $-SO_3H$ oder $-PO_3H_2$ substituiert sein kann. In den vorstehenden Gruppen unter a) und b) sind die an den mit 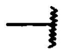 gekennzeichneten Positionen an die benachbarten Gruppen gebunden und die Position α ist an ein Stickstoffatom des makrocyclischen Rings und die Position β an U gebunden.

In der Gruppe der Formel

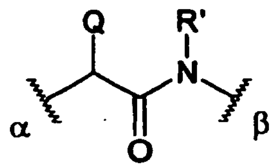

ist Q vorzugsweise ein linearer oder verzweigter $C_{1-10}$-, insbesondere $C_{1-4}$-Alkylrest, wie Methyl, Ethyl oder iso-Propyl, oder ein Cyclohexylrest. Diese Reste können gegebenenfalls mit einer Carboxylgruppe substituiert sein, wobei ein Carboxymethylrest bevorzugt wird. Der bevorzugte Arylrest für Q ist Phenyl. Dieser Arylrest kann mit einer Carboxylgruppe, einer $C_{1-15}$-Alkoxygruppe, einer Aryloxygruppe, wie insbesondere einer Phenoxygruppe oder einem Halogenatom, wie Fluor, Chlor, Brom oder Iod und insbesondere Fluor oder Chlor substituiert sein. Wenn der Arylrest ein Phenylrest ist, ist dieser vorzugsweise in para-Stellung mit einer der genannten Gruppen substituiert. Besonders bevorzugte Gruppen für Q sind Methyl, Phenyl und p-Dodecanoxyphenyl.

R' ist wie vorstehend R definiert, kann aber unabhängig von R gewählt werden. Besonders bevorzugt ist R' ein Wasserstoffatom.

Vorzugsweise ist A' ausgewählt aus $-CH(CO_2H)-$, $-C(CH_3)H-CO-NH-$, -C(Phenyl)H-CO-NH-, -C(p-Dodecanoxyphenyl)H-CO-NH-,

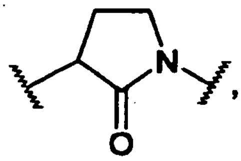, 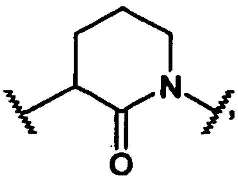, 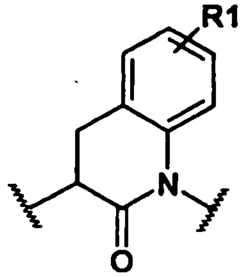 und 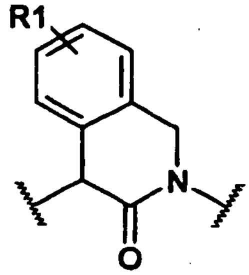

worin $R^1$ $-OCH_3$, $-CO_2H$, $-SO_3H$ oder $-PO_3H_2$ ist.

U ist eine gerade oder verzweigte, gesättigte oder ungesättigte $C_{1-30}$-Kohlenwasserstoffkette, die gegebenenfalls 1-3 Sauerstoffatome, 1-3 Stickstoffatome und/oder 1-3 -NR"-Rest, worin R" wie vorstehend R definiert ist, aber unabhängig gewählt werden kann, enthält und bei der gegebenenfalls 1-3 Kohlenstoffatome als Carbonylgruppen vorliegen, wobei die Kette oder ein Teil der Kette ringförmig angeordnet sein kann. Bevorzugt ist U ein Arylrest oder ein $C_{1-20}$-Alkylrest (vorzugsweise geradlinig oder zumindest teilweise cyclisch und gesättigt) der gegebenenfalls 1-3 Sauerstoffatome, 1-3 - NR"-Reste, 1-2 Phenylenreste und/oder einen Pyridylenrest enthält, bei dem gegebenenfalls 1-3 Kohlenstoffatome als Carbonylgruppen vorliegen, und der gegebenenfalls mit einem Arylrest (z.B. Phenyl) substituiert ist. A' und U müssen zusammen so ausgestaltet sind, daß X über mindestens drei Atome mit dem Stickstoffatom, an das A' gebunden ist, verbunden ist. Die Kette von mindestens drei Atomen ist wie vorstehend zu A definiert.

Der Arylrest für U ist vorzugsweise ein Phenylrest. Der $C_{1-20}$-Alkylrest für U ist vorzugsweise ein linearer, gesättigter $C_{1-10}$-Alkylrest, Cyclohexylrest oder Cyclohexyl-$C_{1-5}$-alkylrest. Die Alkylreste dieser Reste können gegebenenfalls durch 1 Sauerstoffatom, 1 Phenylenrest und/oder 1 Pyridylenrest unterbrochen sein oder einen -CO-NR"-Rest enthalten oder mit Phenyl substituiert sein. Bevorzugt wird U ausgewählt aus $-CH_2-$, $-(CH_2)_5-$, $-(CH_2)_{10}-$, -Phenylen-O-$CH_2$-, -Phenylen-O-$(CH_2)_3$-, -Phenylen-O-$(CH_2)_{10}$-, $-CH_2$-Phenylen-, -Cyclohexylen-O-$CH_2$-, -Phenylen-, -C(Phenyl)H-, $-CH_2$-Pyridylen-O-$CH_2$-, $-CH_2$-Pyridylen-und $-CH_2-CO-NH-CH_2-CH_2-$. In den vorgenannten bevorzugten Gruppen für U sind die Phenylengruppen vorzugsweise in para-Stellung substituiert und bei den Pyridylengruppen handelt es sich vorzugsweise um Pyrid-2,5-ylen- oder Pyrid-2,4-ylen-Gruppen.

Bevorzugte Gruppen für den Spacer A sind:

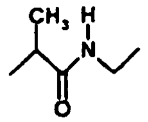, 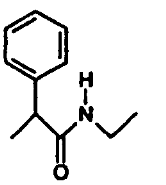,

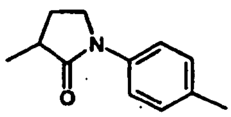,

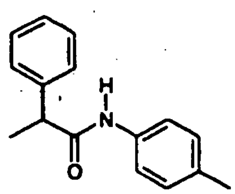, 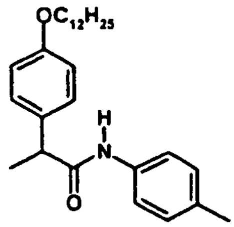,

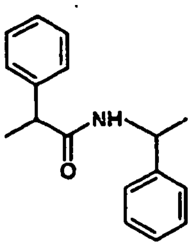 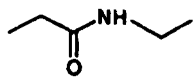, 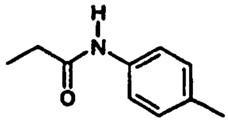,

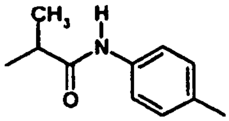, 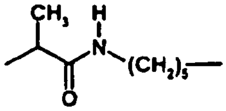 und 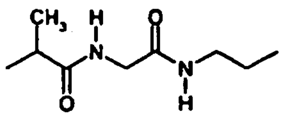.

Über den Spacer A ist eine Gruppe X an den makrocyclischen Ring in den Verbindungen der Formel I angebunden. Bei dieser Gruppe X handelt es sich um eine Gruppe, die mit einem Biomolekül eine Reaktion eingehen kann. Beispielsweise eignet sich hierfür Carboxyl (-COOH), aktiviertes Carboxyl, Amino ($-NH_2$), Isocyanat (-NCO), Isothiocyanat (-NCS), Hydrazin ($-NHNH_2$), Semicarbazid ($-NHCONHNH_2$), Thiosemicarbazid ($-NHCSNHNH_2$), Chloracetamid ($-NHCOCH_2Cl$), Bromacetamid ($-NHCOCH_2Br$), Iodacetamid ($-NHCOCH_2I$), Acylamino, wie beispielsweise Acetylamino ($-NHCOCH_3$), gemischte Anhydride, Azid, Hydroxid, Sulfonylchlorid, Carbodiimid oder eine Gruppe der Formeln

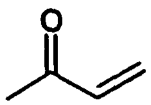, 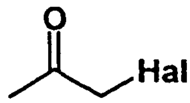 oder 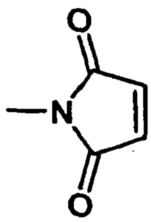

worin Hal ein Halogenatom darstellt.

Unter aktivierter Carboxylgruppe werden vorstehend solche Carboxylgruppen verstanden, die so derivatisiert sind, daß sie die Reaktion mit einem Biomolekül erleichtern. Welche Gruppen zur Aktivierung verwendet werden können, ist bekannt und es kann beispielsweise auf M. und A. Bodanszky, "The Practice of Peptide Synthesis", Springerverlag 1984 verwiesen werden. Beispiele sind Adukte der Carbonsäure mit Carbodiimiden oder aktivierter Ester wie z.B. Hydroxybenzotriazolester. Besonders bevorzugt wird die aktivierte Carboxylgruppe für X ausgewählt aus

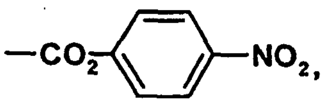 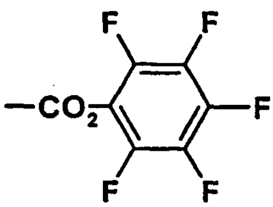 , 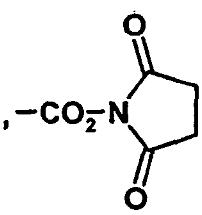 , 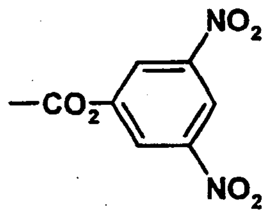

und

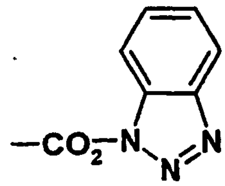

In der Formel I steht Z für ein Wasserstoffatom oder ein Metallionäquivalent. Welches Metallion in der erfindungsgemäßen Verbindung komplexiert vorliegen soll, hängt von der beabsichtigten Anwendung der mit den erfindungsgemäßen Erfindungen hergestellten Konjugate mit einem Biomolekül ab. Entsprechende Konjugate eignen sich beispielsweise für die NMR-Diagnostik, die Radiodiagnostik und Radiotherapie und die Neutroneneinfangtherapie. Besonders bevorzugt werden die Konjugate in der NMR-Diagnostik als Kontrastmittel eingesetzt.

Die Herstellung von Komplexen für die NMR-Diagnostik kann in der Weise erfolgen, wie sie in den Patentschriften EP 71564, EP 130934 und DE-OS 34 01 052 offenbart worden ist. Dazu wird das Metalloxid oder ein Metallsalz (beispielsweise ein Chlorid, Nitrat, Acetat, Carbonat oder Sulfat) des gewünschten Elements in Wasser und/oder einem niederen Alkohol (wie Methanol, Ethanol oder Isopropanol) gelöst oder suspendiert und mit der Lösung oder Suspension der äquivalenten Menge des erfindungsgemäßen Komplexbildners umgesetzt.

Sollen die Komplexbildner zur Herstellung von Radiodiagnostika oder -therapeutika Verwendung finden, kann die Herstellung der Komplexe aus den Komplexbildnern nach den in "Radiotracers for Medical Applications", Vol I, CRC Press, Boca Raton, Florida beschriebenen Methoden erfolgen.

Die erfindungsgemäßen Verbindungen kommen zur Anwendung

1. für die NMR-Diagnostik in Form ihrer Komplexe mit den Ionen der paramagnetischen Elemente mit den Ordnungszahlen 21-29, 42, 44 und 58-70. Geeignete Ionen sind beispielsweise das Chrom(III)-, Eisen(II)-, Cobalt(II), Nickel(II)-, Kupfer(II)-, Praseodym(III)-, Neodym(III)-, Samarium(III)- und Ytterbium(III)-ion. Wegen ihres starken magnetischen Moments sind für die NMR-Diagnostik besonders bevorzugt das Gadolinium(III)-, Terbium(III)-, Dysprosium(III)-, Holmium(III)-, Erbium(III)-, Mangan(II)- und Eisen(III)-ion.

2. für die Radiodiagnostik und Radiotherapie in Form ihrer Komplexe mit den Radioisotopen der Elemente mit den Ordnungszahlen 26, 27, 29, 31, 32, 37-39, 43, 46, 47, 49, 61, 62, 64, 67, 70, 71, 75, 77, 82 und 83.

Die erfindungsgemäßen Verbindungen und insbesondere deren Konjugate mit Biomolekülen erfüllen die vielfältigen Voraussetzungen für die Eignung als Kontrastmittel für die Kernspintomographie. So sind sie hervorragend dazu geeignet, nach oraler oder parenteraler Applikation durch Erhöhung der Signalintensität das mit Hilfe des Kernspintomographen erhaltene Bild in seiner Aussagekraft zu verbessern. Ferner zeigen sie die hohe Wirksamkeit, die notwendig ist, um den Körper mit möglichst geringen Mengen an Fremdstoffen zu belasten, und die gute Verträglichkeit, die notwendig ist, um den nichtinvasiven Charakter der Untersuchungen aufrechtzuerhalten.

Die gute Wasserlöslichkeit und geringe Osmolalität der erfindungsgemäßen Verbindungen und deren Konjugate mit Biomolekülen erlaubt es, hochkonzentrierte Lösungen herzustellen, damit die Volumenbelastung des Kreislaufs in vertretbaren Grenzen zu halten und die Verdünnung durch die Körperflüssigkeit auszugleichen, das heißt NMR-Diagnostika müssen 100- bis 1000fach besser wasserlöslich sein als für die NMR-Spektroskopie. Weiterhin weisen die erfindungsgemäßen Verbindungen nicht nur eine hohe Stabilität in vitro auf, sondern auch eine überraschend hohe Stabilität in vivo, so daß eine Freigabe oder ein Austausch der in den Komplexen nicht kovalent gebundenen - an sich giftigen - Ionen innerhalb der Zeit, in der die neuen Kontrastmittel vollständig wieder ausgeschieden werden, nur äußerst langsam erfolgt.

Ferner können die erfindungsgemäßen Komplexverbindungen vorteilhaft als Suszeptibilitäts-Reagenzien und als shift-Reagenzien für die in vivo-NMR-Spektroskopie verwendet werden.

Die erfindungsgemäßen Verbindungen und deren Konjugate mit Biomolekülen sind aufgrund ihrer günstigen radioaktiven Eigenschaften und der guten Stabilität der in ihnen enthaltenen Komplexverbindungen auch als Radiodiagnostika und Radiotherapeutika geeignet. Details ihrer Anwendung und Dosierung werden z.B. in "Radiotracers for Medical Applications", CRC-Press, Boca Raton, Florida 1983, sowie in Eur. J. Nucl. Med. 17 (1990) 346-364 und Chem. Rev. 93 (1993) 1137-1156 beschrieben.

Für SPECT geeignet sind die Komplexe mit den Isotopen $^{111}In$ und $^{99m}Tc$.

Eine weitere bildgebende Methode mit Radioisotopen ist die Positronen-Emissions-Tomographie, die positronenemittierende Isotope wie z.B. $^{43}Sc$, $^{44}Sc$, $^{52}Fe$, $^{55}Co$, $^{68}Ga$, $^{64}Cu$, $^{86}Y$ und $^{94m}Tc$ verwendet (Heiss, W.D.; Phelps, M.E.; Positron Emission Tomography of Brain, Springer Verlag Berlin, Heidelberg, New York 1983).

Die erfindungsgemäßen Verbindungen und deren Konjugate mit Biomolekülen sind überraschenderweise auch zur Differenzierung von malignen und benignen Tumoren in Bereichen ohne Blut-Hirn-Schranke geeignet. Sie zeichnen sich auch dadurch aus, daß sie vollständig aus dem Körper eliminiert werden und somit gut verträglich sind.

Da sich die erfindungsgemäßen Verbindungen und insbesondere deren Konjugate mit Biomolekülen in malignen Tumoren anreichern (keine Diffusion in gesunde Gewebe, aber hohe Durchlässigkeit von Tumorgefäßen), können sie auch die Strahlentherapie von malignen Tumoren unterstützen. Diese unterscheidet sich von der entsprechenden Diagnostik nur durch die Menge und Art des verwendeten Isotops. Ziel ist dabei, die Zerstörung von Tumorzellen durch energiereiche kurzwellige Strahlung mit einer möglichst geringen Reichweite. Hierzu werden Wechselwirkungen der in den Komplexen enthaltenen Metalle (wie z.B. Eisen oder Gadolinium) mit ionisierenden Strahlungen (z.B. Röntgenstrahlen) oder mit Neutronenstrahlen ausgenutzt. Durch diesen Effekt wird die lokale Strahlendosis am Ort, wo sich der Metallkomplex befindet (z.B. in Tumoren) signifikant erhöht. Um die gleiche Strahlendosis im malignen Gewebe zu erzeugen, kann bei Anwendung solcher Metallkomplexe die Strahlenbelastung für gesunde Gewebe erheblich reduziert und damit belastende Nebenwirkungen für die Patienten vermieden werden. Die erfindungsgemäßen Metallkomplex-Konjugate eignen sich deshalb auch als radiosensibilisierende Substanz bei der Strahlentherapie von malignen Tumoren (z.B. Ausnutzen von Mössbauer-Effekten oder bei Neutroneneinfangtherapie). Geeignete $\beta$-emittierende Ionen sind z.B. $^{46}Sc$, $^{47}Sc$, $^{48}Sc$, $^{72}Ga$, $^{73}Ga$, $^{90}Y$, $^{67}Cu$, $^{109}Pd$, $^{111}Ag$, $^{149}Pm$, $^{153}Sm$, $^{166}Ho$, $^{177}Lu$, $^{186}Re$ und $^{188}Re$. Bevorzugt werden $^{90}Y$, $^{177}Lu$, $^{72}Ga$, $^{153}Sm$ und $^{67}Cu$. Geeignete geringe Halbwertzeiten aufweisende $\alpha$-emittierende Ionen sind z.B. $^{211}At$, $^{211}Bi$, $^{212}Bi$, $^{213}$, Bi und $^{214}Bi$, wobei $^{212}Bi$ bevorzugt ist. Ein geeignetes Photonen- und Elektronenemittierendes Ion ist $^{158}Gd$, das aus $^{157}Gd$ durch Neutroneneinfang erhalten werden kann.

Ist die erfindungsgemäße Verbindung oder deren Konjugat mit einem Biomolekül zur Anwendung in der von R. L. Mills et al. [Nature Vol. 336, (1988), S. 787] vorgeschlagenen Variante der Strahlentherapie bestimmt, so muß sich das Zentralion von einem Mößbauer-Isotop wie beispielsweise $^{57}Fe$ oder $^{151}Eu$ ableiten.

Die Neutralisation eventuell noch vorhandener freier Carboxygruppen erfolgt mit Hilfe anorganischer Basen (z.B. Hydroxyden, Carbonaten oder Bicarbonaten) von z.B. Natrium, Kalium, Lithium, Magnesium oder Calcium und/oder organischer Basen wie unter anderem primärer, sekundärer und tertiärer Amine, wie z.B. Ethanolamin, Morpholin, Glucamin, N-Methyl- und N,N-Dimethylglucamin, sowie basischer Aminosäuren, wie z.B. Lysin, Arginin und Ornithin oder von Amiden ursprüngliche neutraler oder saurer Aminosäuren. Zur Herstellung der neutralen Komplexverbindungen kann man beispielsweise in sauren Komplexsalzen in wäßriger Lösung oder Suspension soviel der gewünschten Base zusetzen, daß der Neutralpunkt erreicht wird. Die erhaltene Lösung kann anschließend im Vakuum zur Trockne eingeengt werden. Häufig ist es von Vorteil, die gebildeten Neutralsalze durch Zugabe von mit Wasser mischbaren Lösungsmitteln, wie z.B. niederen Alkoholen (Methanol, Ethanol, Isopropanol und andere), niederen Ketonen (Aceton und andere), polaren Ethern (Tetrahydrofuran, Dioxan, 1,2-Dimethoxyethan und andere) auszufällen und so leicht zu isolierende und gut zu reinigende Kristallisate zu erhalten. Als besonders vorteilhaft hat es sich erwiesen, die gewünschte Base bereits während der Komplexbildung der Reaktionsmischung zuzusetzen und dadurch einen Verfahrensschritt einzusparen.

Die erfindungsgemäßen Verbindungen der Formel I lassen sich nach dem Fachmann bekannten Verfahren herstellen. Beispielsweise könne die Verbindungen der Formel I durch ein Verfahren erhalten werden, worin eine Verbindung der Formel II

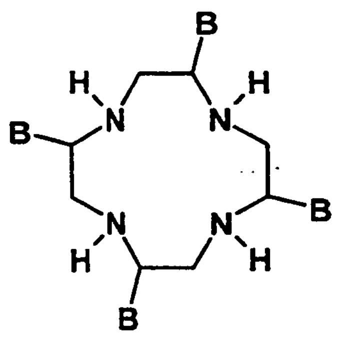

II

worin B wie oben definiert ist gegebenenfalls nach Einführung von Schutzgruppen für die Stickstoffatome mit Nu-A-X' und Nu-CH(R)-$CO_2$Z' umgesetzt wird, wobei A und R wie oben definiert sind und Nu ein Nucleofug ist, X' für X oder eine geschützte Form von X steht und X wie oben definiert ist und Z' für ein Wasserstoffatom, ein Metallionenäquivalent, vorzugsweise von einem Alkali- oder Erdalkalimetall wie insbesondere Natrium oder Kalium, oder eine Schutzgruppe für Carboxyl steht. Anschließend können die gegebenenfalls vorhandenen Schutzgruppen entfernt werden und es kann in an sich bekannter Weise mit mindestens einem Metalloxid oder Metallsalz eines gewünschten Elements umgesetzt werden. Gegebenenfalls können anschließend in den so erhaltenen Komplexen noch vorhandene acide Wasserstoffatome ganz oder teilweise durch Kationen von anorganischen und/oder organischen Basen, Aminosäuren oder Aminosäureamiden substituiert werden.

Drei bevorzugte Verfahrensvarianten werden nachfolgend näher beschrieben:

In der ersten Variante wird zunächst der an den Stickstoffen unsubstituierte Makrocyclus mit der geschützten Einheit AX' umgesetzt. Die Gruppe A trägt dabei als Abgangsgruppe ein Nocleofug. Durch stöchiometrische Reaktionskontrolle reagiert eines der vier Stickstoffatome im Makrocyclus mit der Gruppe A unter Austritt der Abgangsgruppe. Auf diese Weise erhält man einen monofunktionalisierten Makrocyclus, welcher den Rest X in geschützter Form (X') enthält. Im zweiten Reaktionsschritt werden die verbliebenen drei nukleophilen Stickstoffatome des Makrocyclus jeweils mit einer geschützten Carbonsäure umgesetzt, welche in α-Position zur Carboxylgruppe ein Nucleofug trägt. Nach Abspaltung der Schutzgruppen von den Carbonsäurefunktionalitäten wird der Komplex aus paramagnetischem Metallion und Chelatligand durch Zugabe von Metalloxid oder Metallsalz fertiggestellt. Diese Verfahrensvariante ist im folgenden schematisch wiedergegeben, wobei die Reste in den Formeln wie vorstehend definiert sind:

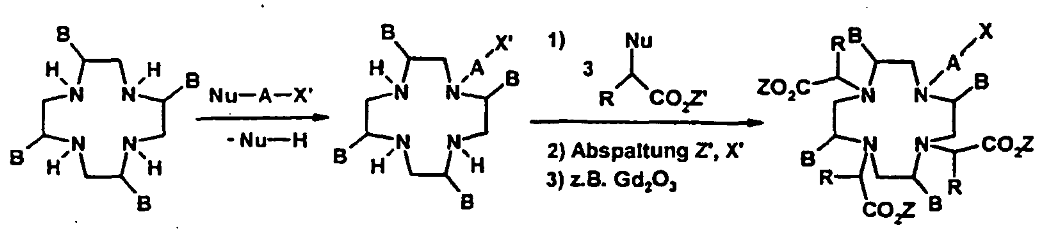


Nu = Nucleofug (z.B. Br, I, O-Triflat, Mesylat, Tosylat, etc). $Z = \frac{1}{3}\ Gd^{3+}$

Z' = Schutzgruppe der Carbonsäure

In einer zweiten Variante kommt als Edukt ein Makrocyclus zum Einsatz, welcher an drei der vier Stickstoffatome bereits geeignete Schutzgruppen SG trägt. Als Schutzgruppen eignen sich hier z.B. tert-Butyl-oxycarbonyl (t-BOC), $COCF_3$, Carbobenzoxy (Cbo) oder Fluorenyl-methoxycarbonyl (FMOC), etc. Durch die Anwesenheit der Schutzgruppen ist nur noch eines der vier Stickstoffatome nukleophil und kann mit A-X' reagieren, das seinerseits wie bei der vorstehenden Variante ein Nucleofug Nu trägt. Nach Verknüpfung beider Moleküle unter Austritt der Abgangsgruppe erfolgt eine Abspaltung der drei Schutzgruppen von den Stickstoffatomen. Es schließt sich die Derivatisierung mit Hilfe der Carbonsäurederivate an, wie sie bereits für die vorstehende Variante beschrieben wurde. Diese zweite Verfahrensvariante ist im folgenden schematisch wiedergegeben, wobei die Reste in den Formeln wie oben definiert sind:

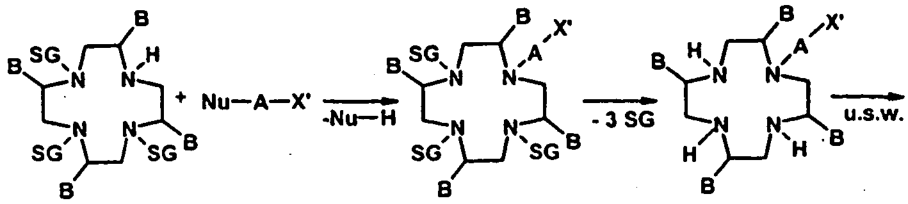


SG = Schutzgruppe (z.B. BOC, Cbo, $COCF_3$, FMOC, etc.)

In der dritten Variante wird zunächst eines der vier Stickstoffatome des Makrocyclus durch eine entsprechende Schutzgruppe SG blockiert. Beispiele für geeignete Schutzgruppen sind Formyl, Benzyl, Boctrityl, etc. Nun erfolgt die Umsetzung an den drei verbleibenden nukleophilen Stickstoffatomen mit entsprechend geschützten Carbonsäurederivaten, die in α-Position ein entsprechendes Nucleofug tragen. Anschließend erfolgt die Abspaltung der zunächst am ersten Stickstoffatom eingeführten Schutzgruppe SG und Derivatisierung mit AX', das seinerseits ebenfalls ein Nucleofug trägt. Diese dritte Verfahrensvariante ist im folgenden schematisch wiedergegeben, wobei die Reste in den Formeln wie oben definiert sind:

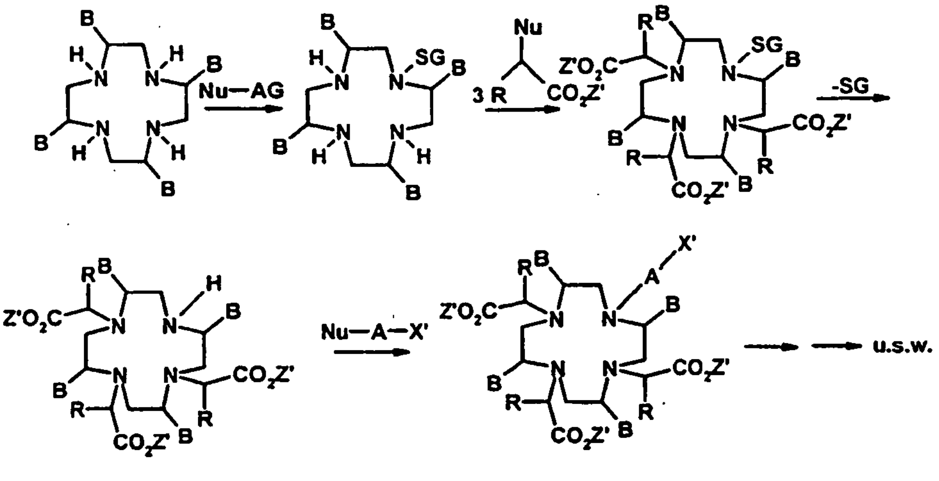


Als Nucleofug dienen vorteilhaft die Reste:

Cl, Br, I, O-Triflat, Mesylat und Tosylat.

Die Umsetzung wird im Gemisch von Wasser und organischen Lösungsmitteln wie:

Isopropanol, Ethanol, Methanol, Butanol, Dioxan, Tetrahydrofuran, Dimethylformamid, Dimethylacetamid, Formamid oder Dichlormethan durchgeführt. Bevorzugt sind ternäre Gemische aus Wasser, Isopropanol und Dichlormethan.

Die Umsetzung wird in einem Temperaturbereich zwischen -10°C und 100°C, vorzugsweise zwischen 0°C und 30°C durchgeführt.

Der Schutz der vorstehend benannten Gruppen kann auf zahlreichen, dem Fachmann bekannten Möglichkeiten erfolgen. Die nachfolgend beschriebenen Ausführungsformen dienen zur Erläuterung dieser Schutzgruppentechniken ohne auf diese Synthesewege beschränkt zu sein.

Als Säureschutzgruppen kommen $C_1$-$C_6$-Alkyl-, $C_6$-$C_{10}$-Aryl- und $C_6$-$C_{10}$-Ar($C_1$-$C_4$)-alkylgruppen sowie Trialkylsilylgruppen in Frage. Bevorzugt werden die Methyl-, Ethyl-, Propyl-, Isopropyl, n-Butyl-, i-Butyl- und die tert-Butylgruppe.

Die Abspaltung dieser Säureschutzgruppen erfolgt nach den dem Fachmann bekannten Verfahren, beispielsweise durch Hydrolyse, Hydrogenolyse, alkalische Verseifung der Ester mit Alkali in wäßrig-alkoholischer Lösung bei Temperaturen von 0 bis 50°C, saure Verseifung mit Mineralsäuren oder im Fall von tert-Butylestern mit Hilfe von Trifluoressigsäure.

Die NH-Gruppen lassen sich in vielfältiger Weise schützen und wieder freilegen. Das N-Trifluoracetylderivat wir durch Kalium- oder Natriumcarbonat in Wasser (H. Newman, J. Org. Chem., 30: 287 (1965), M.A. Schwartz et al., J. Am. Chem. Soc., 95 G12 (1973)) oder einfach durch Ammoniaklösung gespalten (M. Imazama u. F. Eckstein, J. Org. Chem., 44: 2039 (1979)). Ebenfalls milde zu spalten ist das tert-Butyloxycarbonylderivat: es genügt Rühren mit Trifluoressigsäure (B.F. Lundt et al., J. Org. Chem., 43: 2285 (1978)). Sehr groß ist die Gruppe der hydrogenolytisch oder reduzierend zu spaltenden NH-Schutzgruppen: Die N-Benzylgruppe ist bequem mit Wasserstoff/Pd-C zu spalten (W. H. Hartung u. R. Rimonoff, Org. Reactions VII, 262 (1953)), was auch für die Tritylgruppe (L. Zervas et al., J. Am. Chem. Soc., 78: 1359 (1956)) und die Benzyloxycarbonylgruppe gilt (M. Bergmann u. L. Zervas Ber. 65: 1192 (1932)).

Die aktivierten Ester der vorstehend beschriebenen Verbindungen werden wie dem Fachmann bekannt hergestellt. Für den Fall von Isothiocyanaten oder α-Halogenacetaten werden die entsprechenden terminalen Aminvorstufen nach literaturbekannten Methoden mit Thiophosgen oder 2-Halo-Essigsäure-Halogeniden umgesetzt. Auch die Umsetzung mit entsprechend derivatisierten Estern von N-Hydroxysuccinimid wie beispielsweise:

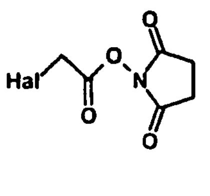

ist möglich (Hal = Halogen).

Allgemein können für diesen Zweck alle üblichen Aktivierungsmethoden für Carbonsäuren verwendet werden, die im Stand der Technik bekannt sind. Das Molekül Nu-A-X' wird bevorzugt zunächst unabhängig synthetisiert. Enthält das Molekül eine Amidgruppe, so wird diese beispielsweise hergestellt, indem eine aktivierte Carbonsäure mit einem Amin umgesetzt wird. Die Aktivierung der Carbonsäure erfolgt nach den üblichen Methoden. Beispiele für geeignete Aktivierungsreagentien sind Dicyclohexylcarbodiimid (DCC), 1-Ethyl-3-(3-dimethylaminopropyl)-carbodiimid-hydrochlorid (EDC), Benzotriazol-1-yloxytris-(dimethylamino)-phosphoniumhexafluorophosphat (BOP) und O-(Benzotriazol-1-yl)-1,1,3,3-tetramethyluroniumhexafluorophosphat (HBTU), vorzugsweise DCC. Auch der Zusatz von O-nukleophilen Katalysatoren, wie z.B. N-Hydroxysuccinimid (NHS) oder N-Hydroxybenzotriazol ist möglich.

Handelt es sich bei der Gruppe X um eine Carbonsäurefunktion, so kann diese in geschützter Form (z.B. in Form des Benzylesters) eingesetzt werden, die Abspaltung der Schutzgruppe kann dann hydrogenolytisch erfolgen.

Um diese Carbonsäurefunktion an eine geeignete funktionelle Gruppe eines geeigneten Biomoleküls zu knüpfen, sollte diese im Regelfall zunächst aktiviert werden. Bevorzugt werden dazu aktivierte Ester intermediär erzeugt, welche dann von einer nukleophilen Gruppe des Biomoleküls angegriffen werden. Auf diese Weise entsteht eine kovalente Verknüpfung zwischen dem Biomolekül und der erfindungsgemäßen Verbindung der Formel I. Bevorzugte aktivierte Ester sind die Ester des N-Hydroxysuccinimids, die Ester des Paranitrophenols oder die Ester des Pentafluorphenols. Soll die Gruppe X in Form eines Isothiocyanats an das Biomolekül geknüpft werden, so wird bevorzugt zunächst ein terminales Amin verwendet, welches, wenn notwendig, mit einer geeigneten Schutzgruppe versehen sein kann. Geeignete Schutzgruppen sind aus der Peptidchemie bekannt. Nach Abspaltung der Schutzgruppe kann durch Umsetzung des primären terminalen Amins mit Thiophosgen das Isothiocyanat erzeugt werden. An dieses können nukleophile Gruppen des Biomoleküls addiert werden.

In einer Ausführungsform stellt die Gruppe X ein Maleinimid dar, welches z.B. selektiv mit Thiolfunktionen des Biomoleküls reagieren kann.

In einer anderen Ausführungsform ist die Gruppe X ein Nukleophil ($NH_2$, SH), welches an einer geeigneten Funktionalität des Biomoleküls angreift (aktivierter Ester, Maleinimid, etc.). Zahlreiche mit Maleinimiden funktionalisierte Biomoleküle sind kommerziell erhältlich.

Die vorliegende Erfindung betrifft darüber hinaus die Verwendung der vorstehend beschriebenen Verbindungen der Formel I zur Herstellung von Konjugaten mit einem Biomolekül.

Die Synthese der Konjugate erfolgt in der Regel derart, daß zunächst ein derivatisierter und funktionalisierter Chelatkomplex erzeugt wird, welcher dann an das Biomolekül geknüpft wird. Es ist aber auch möglich, daß im Falle der Verwendung von synthetisch hergestellten Biomolekülen der erfindungsgemäße Chelatkomplex während der Synthese des Biomoleküls in dieses eingebaut wird. Dies kann beispielsweise während der sequentiellen Synthese von Oligopeptiden am Syntheseroboter erfolgen. Falls erforderlich, können dazu die in der Synthese des entsprechenden Biomoleküls üblichen Schutzgruppen in die erfindungsgemäße Verbindung eingeführt werden. Diese werden dann im Zuge der üblichen Synthesealgorithmen am Synthesizer wieder abgespalten.

Unter "Biomolekül" wird vorliegend jedes Molekül verstanden, das entweder natürlich beispielsweise im Körper auftritt oder mit analoger Struktur synthetisch hergestellt wurde. Darüber hinaus werden hierunter solche Moleküle verstanden, die mit einem biologisch, beispielsweise im Körper auftretenden Molekül oder einer dort auftretenden Struktur in Wechselwirkung treten können, so daß sich beispielsweise die Konjugate an bestimmten, gewünschten Stellen des Körpers anreichern. Unter "Körper" wird vorliegend jeder pflanzliche oder tierische Körper verstanden, wobei tierische und insbesondere menschliche Körper bevorzugt sind.

Biomoleküle sind insbesondere die in Lebewesen auftretenden Moleküle, die als Produkte einer evolutionären Selektion durch geordnetes und komplexes Zusammenwirken spezifische Aufgaben für den Organismus erfüllen und die Grundlage seiner Lebensfunktionen (Stoff- und Formwechsel, Fortpflanzung, Energiehaushalt) ausmachen. In Biomolekülen sind zumeist aus einfachen Bausteinen (Aminosäuren, Nucleobasen, Monosacchariden, Fettsäuren, etc.) größere Moleküle (Proteine, Nucleinsäuren, Polysaccharide, Lipide, etc.) aufgebaut. Entsprechende Makromoleküle werden auch als Biopolymere bezeichnet.

Vorteilhaft kann das Biomolekül beispielsweise ein Polypeptidgerüst aus Aminosäuren mit Seitenketten aufweisen, die mit der reaktiven Gruppe X der erfindungsgemäßen Verbindungen der Formel I eine Reaktion eingehen können. Solche Seitenketten schließen beispielsweise die Carboxylgruppen von Asparaginsäure- und Glutaminsäureresten, die Aminogruppen von Lysinresten, die aromatischen Gruppen von Tyrosin- und Histidinresten und die Sulphhydrylgruppen von Cysteinresten ein.

Eine Übersicht über Biomoleküle mit zahlreichen Beispielen findet sich in dem Skriptum "Chemie der Biomoleküle" der TU-Graz (H. Berthold et al., Institut für Organische Chemie, TU-Graz, 2001), das auch über das Internet unter www.orgc.tu-graz.ac.at eingesehen werden kann. Der Inhalt dieses Dokuments wird durch Bezugnahme in die vorliegende Beschreibung aufgenommen.

Zur Bildung von Konjugaten mit den erfindungsgemäßen Verbindungen sind folgende Biomoleküle besonders geeignet:

Biopolymere, Proteine, wie Proteine, die eine biologische Funktion haben, HSA, BSA, etc., Proteine und Peptide, die sich an bestimmten Stellen im Organismus anreichern (z.B. an Rezeptoren, Zellmembranen, Kanälen etc.), durch Proteasen spaltbare Peptide, Peptide mit synthetischen Sollbruchstellen (z.B. labile Ester, Amide etc.), Peptide, die durch Metalloprotheasen gespalten werden, Peptide mit photospaltbaren Linkern, Peptide mit oxydativen Mitteln (Oxydasen) spaltbaren Gruppen, Peptide mit natürlichen und unnatürlichen Aminosäuren, Glycoproteine (Glycopeptide), Signal-Proteine, antivirale Proteine und Apoktosis, synthetisch modifizierte Biopolymere, wie mit Linkern derivatisierte Biopolymere, modifizierte Metalloproteasen und derivatisierte Oxydase etc., Kohlenhydrate (Mono- bis Polysaccharide), wie derivatisierte Zucker, im Organismus spaltbare Zucker, Cyclodextrine und dessen Derivate, Aminozucker, Chitosan, Polysulfate und Acetylneuraminsäure-Derivate, Antikörper, wie monoklonale Antikörper, Antikörperfragmente, polyklonale Antikörper, Minibodies, Single Chains (auch solche, die mit Linkern zu mehrfachen Fragmenten verknüpft sind), rote Blutkörperchen und andere Blutbestandteile, Cancermarker (z.B. CAA) und Zell-Adhäsions-Stoffe (z.B. Lewis X und Anti-Lewis X-Derivate), DNA und RNA Fragmente, wie derivatisierte DNAs und RNAs (z.B. solche, die durch das SELEX-Verfahren gefunden wurden), synthetische RNA und DNA (auch mit unnatürlichen Basen), PNAs (Hoechst) und Antisense, β-Aminosäuren (Seebach), Vektoramine zur Einschleusung in die Zelle, biogene Amine, Pharmazeutika, onkologische Präparate, synthetische Polymere, die auf ein biologisches Target (z.B. Rezeptor) gerichtet sind, Steroide (natürliche und modifizierte), Prostaglandine, Taxol und dessen Derivate, Endotheline, Alkaloide, Folsäure und deren Derivate, bioaktive Lipide, Fette, Fettsäureester, synthetisch modifizierte Mono-, Di- und Triglyceride, Liposome, die an der Oberfläche derivatisiert sind, Micellen aus natürlichen Fettsäuren oder aus Perfluoralkyl-Verbindungen, Porphyrine, Texaphrine, erweitere Porphyrine, Cytochrome, Inhibitoren, Neuramidasen, Neuropeptide, Immunomodulatoren, wie FK 506, CAPE und Gliotoxin, Endoglycosidasen, Substrate, die durch Enzyme aktiviert werden wie Calmodolin Kinase, Casein-Kinase II, Gluthathion-S-Transferase, Heparinase, Matrix-Metalloprotheasen, β-Insulin-Rezeptor-Kinase, UDP-Galactose 4-Epimerase, Fucosidasen, G-Proteine, Galactosidasen, Glycosidasen, Glycosyltransferasen und Xylosidase, Antibiotika, Vitamin und Vitamin-Analoga, Hormone, DNA-Interkalatoren, Nucleoside, Nucleotide, Lektine, Vitamin B12, Lewis-X und Verwandte, Psoralene, Dientrienantibiotika, Carbacycline, VEGF (vascular endothelial growth factor), Somatostatin und dessen Derivate, Biotin-Derivate, Antihormone, tumorspezifische Proteine und Synthetika, Polymere, die sich in sauren oder basischen Bereichen des Körpers anreichern (pH-gesteuerte Verteilung), Myoglobine, Apomyoglobine etc., Neurotransmitter-Peptide, Tumornecrosefaktoren, Peptide, die sich in entzündetem Gewebe anreichern, Bloodpool-Reagenzien, Anionen und Kationen-Transporterproteine, Polyester (z.B. der Milchsäure), Polyamide und Polyphosphate.

Die meisten der vorgenannten Biomoleküle sind kommerziell beispielsweise bei Merck, Alderich, Sigma, Calibochem oder Bachem erhältlich.

Außerdem können als Biomoleküle alle in der WO 96/23526 und der WO 01/08712 offenbarten "Plasmaproteinbindungsgruppen" bzw. "Zielbindungsgruppen" eingesetzt werden. Der Inhalt dieser beiden Offenlegungsschriften wird daher durch Bezugnahme in die vorliegende Beschreibung aufgenommen.

Die Anzahl der erfindungsgemäßen Verbindungen der Formel I pro Biomolekül ist prinzipiell beliebig, bevorzugt ist jedoch ein molekulares Verhältnis 0,1:1 bis 10:1, insbesondere von 0,5:1 bis 7:1.

Ferner eignen sich die erfindungsgemäßen Verbindungen zur Konjugation an all diejenigen Moleküle, welche im Stand der Technik mit Fluoreszenzfarbstoffen umgesetzt werden, um beispielsweise ihre Lokalisation durch Epifluoreszenzmikroskopie innerhalb der Zelle zu bestimmen. Auch können die Verbindungen mit prinzipiell beliebigen Medikamenten konjugiert werden, um dann nach Verabreichung des Medikaments den Transport innerhalb des Organismus durch die NMR-Technik zu verfolgen. Ferner ist es möglich, daß die Konjugate aus den erfindungsgemäßen Verbindungen und den Biomolekülen weitere zusätzliche Moleküle enthalten, die an die Biomoleküle konjugiert worden sind. Mit dem Begriff "Biomolekül" im Sinne der Erfindung sind also alle Moleküle umfaßt, die in biologischen Systemen vorkommen und alle Moleküle, die biokompatibel sind.

Bevorzugt werden die mit den erfindungsgemäßen Verbindungen erhaltenen Konjugate als Kontrastmittel in der NMR-Diagnostik eingesetzt. Daher sollten die Konjugate wasserlöslich sein. Wenn die mit den erfindungsgemäßen Verbindungen erhaltenen Konjugate als NMR-Kontrastmittel eingesetzt werden sollen, werden sie vorzugsweise in einer Menge von 0,0001-5 mMol/kg Körpergewicht und besonders bevorzugt in einer Menge von 0,005-0,5 mMol/kg Körpergewicht dosiert. Details der Anwendung werden z.B. in H.-J. Weinmann et al., Am. J. of Roentgenology 142, 619 (1984) diskutiert. Durch die überraschend hohe Relaxivität der erfindungsgemäßen Verbindungen bei gleichzeitiger Targetspezifität der mit diesen Verbindungen erhaltenen Konjugate können diese beispielsweise zum Nachweis von Tumoren besonders niedrig dosiert werden.

Details der Anwendungen von Radiotherapeutika werden z.B. in R. W. Kozak et al. TIBTEC, Oktober 1986, 262, diskutiert (s. a. Bioconjugate Chem. 12 (2001) 7-34).

Durch die nachfolgenden Beispiele wird die vorliegende Erfindung näher erläutert, ohne sie darauf einzuschränken.

***Beispiele***

**Beispiel 1**

a) 10-[4-(Benzyloxycarbonyl)-1-methyl-2-oxo-3-azabutyl]-1,4,7-α,α',α"-trimethyl-1,4,7-tris-(benzyloxycarbonylmethyl)-1,4,7,10-tetraazacyclododecan

Zu 27,9 g (162,2 mmol) 1,4,7,10-Tetraazacyclododecan, gelöst in 300 mL Chloroform, gibt man 25 g (81,1 mmol) 2-Brompropionylglycin-benzylester (Beispiel 1e der WO 98/24774) und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Chloro-form/Methanol/aqu. 25 % Ammoniak = 10/5/1). Das so erhaltene 1-[4-(Benzyloxycarbonyl)-1-methyl-2-oxo-3-azabutyl]-1,4,7,10-tetraazacyclododecan (19,6 g; 50 mmol; 62 % d. Th.) und 60 mL (0,35 mol) N-Ethyldiisopropylamin in 200 mL Dichlormethan gibt man zu 62,45 g (0,2 mol) 2-(Trifluormethansulfonyloxy)propansäurebenzylester (Kitazaki et al., Chem. Pharm. Bull. (1999), 47(3), 360) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 32,0 g (73 % d. Th.) eines farblosen kristallinen Pulvers

| Elementaranalyse: | | | |
|---|---|---|---|
| ber.: | C 68,39 | H 7,23 | N 7,98 |
| gef.: | C 67,95 | H 7,41 | N 8,22 |

b) 10-(4-Carboxy-1-methyl-2-oxo-3-azabutyl)-1,4,7-α,α',α"-trimethyl-1,4,7-tris(carboxymethyl)-1,4,7,10-tetraazacyclododecan

26,3 g (30 mmol) der Titelverbindung aus Beispiel 1a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 15,7 g (quantitativ) eines farblosen Pulvers

| Elementaranalyse: | | | |
|---|---|---|---|
| ber.: | C 51,05 | H 7,60 | N 13,53 |
| gef.: | C 50,71 | H 7,83 | N 13,25 |

c) Gd-Komplex des 10-(4-Carboxy-1-methyl-2-oxo-3-azabutyl)-1,4,7-α,α',α"-trimethyl-1,4,7-tris(carboxymethyl)-1,4,7,10-tetraazacyclododecans

10,4 g (20 mmol) des in Beispiel 1b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 10,1 g (69 % d. Th.) eines farblosen Pulvers.
Wassergehalt (Karl-Fischer): 8,3 %

| Elementaranalyse (bezogen auf die wasserfreie Substanz): | | | | |
|---|---|---|---|---|
| ber.: | C 39,33 | H 5,40 | Gd 23,41 | N 10,42 |
| gef.: | C 39,21 | H 5,88 | Gd 22,93 | N 10,11 |

**Beispiel 2**

a) 10-[4-(Benzyloxycarbonyl)-1-methyl-2-oxo-3-azabutyl]-1,4,7-α,α',α''-tris(isopropyl)-1,4,7-tris(benzyloxycarbonylmethyl)-1,4,7,10-tetraazacyclododecan

19,6 g (50 mmol) des in Beispiel 1a als Zwischenprodukt beschriebenen 1-[4-(Benzyloxycarbonyl)-1-methyl-2-oxo-3-azabutyl]-1,4,7,10-tetraazacyclododecans und 60 mL (0,35 mol) N-Ethyldiisopropylamin in 200 mL Dichlormethan gibt man zu 68,1 g (0,2 mol) 2-(Trifluormethansulfonyloxy)-isovaleriansäurebenzylester (Walker et al., Tetrahedron (1997), 53(43), 14591) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 33,7 g (70 % d. Th.) eines farblosen kristallinen Pulvers

| Elementaranalyse: | | | |
|---|---|---|---|
| ber.: | C 69,90 | H 7,86 | N 7,28 |
| gef.: | C 69,77 | H 7,51 | N 7,22 |

b) 10-(4-Carboxy-1-methyl-2-oxo-3-azabutyl)-1,4,7-α,α',α''-tris(isopropyl)-1,4,7-tris(carboxymethyl)-1,4,7,10-tetraazacyclododecan

28,9 g (30 mmol) der Titelverbindung aus Beispiel 2a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 18,0 g (quantitativ) eines farblosen Pulvers

| Elementaranalyse: | | | |
|---|---|---|---|
| ber.: | C 55,89 | H 8,54 | N 11,64 |
| gef.: | C 55,63 | H 8,83 | N 11,31 |

c) Gd-Komplex des 10-(4-Carboxy-1-methyl-2-oxo-3-azabutyl)-1,4,7-α,α',α''-tris(isopropyl)-1,4,7-tris(carboxymethyl)-1,4,7,10-tetraazacyclododecans

12,0 g (20 mmol) des in Beispiel 2b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 12,0 g (72 % d. Th.) eines farblosen Pulvers.
Wassergehalt (Karl-Fischer): 9,1 %

| Elementaranalyse (bezogen auf die wasserfreie Substanz): | | | | |
|---|---|---|---|---|
| ber.: | C 44,49 | H 6,40 | Gd 20,80 | N 9,26 |
| gef.: | C 44,21 | H 6,72 | Gd 20,23 | N 9,11 |

**Beispiel 3**

a) 10-[4-(Benzyloxycarbonyl)-1-methyl-2-oxo-3-azabutyl]-1,4,7-α,α',α''-tris(cyclohexyl)-1,4,7-tris(benzyloxycarbonylmethyl)-1,4,7,10-tetraazacyclododecan

19,6 g (50 mmol) des in Beispiel 1a als Zwischenprodukt beschriebenen 1-[4-(Benzyloxycarbonyl)-1-methyl-2-oxo-3-azabutyl]-1,4,7,10-tetraazacyclododecans und 60 mL (0,35 mol) N-Ethyldiisopropylamin in 200 mL Dichlormethan gibt man zu 76,1 g (0,2 mol) 2-(Trifluormethansulfonyloxy)-2-cyclohexylessigsäurebenzylester (Qabar et al., Tetrahedron Letters (1998), 39(33), 5895) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 41,1 g (76 % d. Th.) eines farblosen kristallinen Pulvers

| Elementaranalyse: | | | |
|---|---|---|---|
| ber.: | C 72,13 | H 8,10 | N 6,47 |
| gef.: | C 71,88 | H 8,21 | N 6,25 |

b) 10-(4-Carboxy-1-methyl-2-oxo-3-azabutyl)-1,4,7-α,α',α''-tris(cyclohexyl)-1,4,7-tris(carboxymethyl)-1,4,7,10-tetraazacyclododecan

32,5 g (30 mmol) der Titelverbindung aus Beispiel 3a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 22,0 g (quantitativ) eines farblosen Pulvers

| Elementaranalyse: | | | |
|---|---|---|---|
| ber.: | C 61,56 | H 8,80 | N 9,70 |
| gef.: | C 61,17 | H 8,98 | N 9,41 |

c) Gd-Komplex des 10-(4-Carboxy-1-methyl-2-oxo-3-azabutyl)-1,4,7-α,α',α''-tris(cyclohexyl)-1,4,7-tris(carboxymethyl)-1,4,7,10-tetraazacyclododecans

14,4 g (20 mmol) des in Beispiel 3b beschriebenen Liganden werden in 150 mL Wasser und 150 mL Isopropanol gelöst und durch Zugabe von 5 mL Essigsäure angesäuert. Es werden 3,6 g (10 mmol) Gadoliniumoxid zugegeben und 8 h am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und zur Trockne eingedampft. Der Rückstand wird mit Ameisensäure aufgenommen und mehrfach unter Zusatz von Dichlormethan zur Trockne eingedampft und anschließend bis zur Gewichtskonstanz im Vakuum getrocknet.
Ausbeute: 12,4 g (65 % d. Th.) eines farblosen Pulvers.
Wassergehalt (Karl-Fischer): 8,0 %

| Elementaranalyse (bezogen auf die wasserfreie Substanz): | | | | |
|---|---|---|---|---|
| ber.: | C 50,72 | H 6,90 | Gd 17,95 | N 7,99 |
| gef.: | C 51,03 | H 7,08 | Gd 17,42 | N 8,11 |

## Beispiel 4

a) 10-[4-(t-Butoxycarbonyl)-1-phenyl-2-oxo-3-azabutyl]-1,4,7-α,α',α''-trimethyl-1,4,7-tris-(benzyloxycarbonylmethyl)-1,4,7,10-tetraazacyclododecan

Zu 27,9 g (162,2 mmol) 1,4,7,10-Tetraazacyclododecan, gelöst in 300 mL Chloroform, gibt man 26,6 g (81,1 mmol) N-[2-Brom-2-phenylacetyl]-glycin-t-butylester (Beispiel 6a der WO 98/24775) und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Chloro-form/Methanoi/aqu. 25 % Ammoniak = 10/5/1). Das so erhaltene 1-[4-(t-Butoxycarbonyl)-1-phenyl-2-oxo-3-azabutyl]-1,4,7,10-tetraazacyclododecan (21,0 g; 50 mmol; 62 % d. Th.) und 60 mL (0,35 mol) N-Ethyldiisopropylamin in 200 mL Dichlormethan gibt man zu 62,45 g (0,2 mol) 2-(Trifluormethansulfonyloxy)propansäurebenzylester (Kitazaki et al., Chem. Pharm. Bull. (1999), 47(3), 360) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel:
Dichlormethan/Methanol: 20/1). Die das Produkt enthaltenden Fraktionen werden vereinigt und eingedampft.
Ausbeute: 34,0 g (75 % d. Th.) eines farblosen kristallinen Pulvers

| Elementaranalyse: | | | |
|---|---|---|---|
| ber.: | C 68,93 | H 7,45 | N 7,73 |
| gef.: | C 69,12 | H 7,57 | N7,60 |

b) 10-(4-(*t*-Butoxycarbonyl-1-phenyl-2-oxo-3-azabutyl)-1,4,7-α,α',α''-trimethyl-1,4,7-tris(carboxy-methyl)-1,4,7,10-tetraazacyclododecan

27,2 g (30 mmol) der Titelverbindung aus Beispiel 4a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 17,5g (quantitativ) eines farblosen Pulvers

| Elementaranalyse: | | | |
|---|---|---|---|
| ber.: | C 55,95 | H 7,13 | N 12,08 |
| gef.: | C 56,21 | H 6,99 | N 11,83 |

c) Gd-Komplex des 10-(4-Carboxy-1-phenyl-2-oxo-3-azabutyl)-1,4,7-α,α',α''-trimethyl-1,4,7-tris(carboxymethyl)-1,4,7,10-tetraazacyclododecans

11,6 g (20 mmol) des in Beispiel 4b beschriebenen *t*-Butylesters werden in sehr wenig Trifluoressigsäure gelöst und 15 min bei Raumtemperatur gerührt. Nach Zugabe von 250 mL Diethylether wird 2 Stunden nachgerührt, der Niederschlag abgesaugt und im Vakuum getrocknet. Der so erhaltene freie Ligand wird in 200 mL Wasser und 80 mL Isopropanol gelöst, mit verdünntem Ammoniak auf pH 7 eingestellt und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 11,6 g (72 % d. Th.) eines farblosen Pulvers.
Wassergehalt (Karl-Fischer): 9,0 %

| Elementaranalyse (bezogen auf die wasserfreie Substanz): | | | | |
|---|---|---|---|---|
| ber.: | C 44,19 | H 5,22 | Gd 21,43 | N 9,54 |
| gef.: | C 43,91 | H 5,27 | Gd 21,09 | N 9,77 |

**Vergleichsbeispiel 5**

a) 4-(Ethoxycarbonylmethoxy)-phenylessigsäuremethylester

10 g (60,2 mmol) Hydroxyphenylessigsäuremethylester (Aldrich) werden in 75 mL Aceton gelöst. Es werden 18,4 g (133 mmol) festes Kaliumcarbonat zugegeben, 17,8 mL (123 mmol) Bromessigsäureethylester innerhalb 15 min unter Rückfluß zugetropft, weitere 4 Stunden bei dieser Temperatur gehalten und über Nacht bei Raumtemperatur gerührt. Es wird vom Niederschlag abfiltriert, die Lösung zur Trockne eingedampft und an Kieselgel chromatographiert (Hexan/Essigester 3: 1). Die das Produkt enthaltenden Fraktionen werden vereinigt und eingedampft.
Ausbeute: 14,6 g (96 % d. Th.)

| Elementaranalyse: | | |
|---|---|---|
| ber.: | C 61,90 | H 6,39 |
| gef.: | C 61,67 | H 6,50 |

b) α-Brom-4-(ethoxycarbonylmethoxy)-phenylessigsäuremethylester

13,5 g (53,5 mmol) der Titelverbindung aus Beispiel 5a werden in 75 mL Tetrachlorkohlenstoff gelöst. Es werden 9,52 g (53,5 mmol) N-Bromsuccinimid und 48 mg Dibenzoylperoxid zugegeben, 5 Stunden unter Rückfluß erhitzt und über Nacht bei Raumtemperatur gerührt. Die Suspension wird zweimal mit Natriumhydrogencarbonat-Lösung und

einmal mit Wasser gewaschen, die organische Phase mit Magnesiumsulfat getrocknet, vom Trocknungsmittel abfiltriert und das Filtrat im Vakuum zur Trockne eingedampft. Der Rückstand wird an Kieselgel chromatographiert (Hexan/ Essigester 3:1). Die das Produkt enthaltenden Fraktionen werden vereinigt und eingedampft.
Ausbeute: 15,4 g (87 % d. Th.)

| Elementaranalyse: | | | |
|---|---|---|---|
| ber.: | C 47,15 | H 4,57 | Br 24,13 |
| gef.: | C 47,01 | H 4,76 | Br 23,70 |

c) 10-[α-(4-(Ethoxycarbonylmethoxy)phenyl)-methoxycarbonylmethyl]-1,4,7-α,α',α''-trimethyl-1,4,7-tris(benzyloxycarbonylmethyl)-1,4,7,10-tetraazacyclododecan

Zu 27,9 g (162,2 mmol) 1,4,7,10-Tetraazacyclododecan, gelöst in 300 mL Chloroform, gibt man 26,9 g (81,1 mmol) der im vorstehenden Beispiel 5b beschriebenen Bromverbindung und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Dichlormethan/Methanol/Triethylamin = 10/5/0,1). Das so erhaltene 1-[α-(4-(Ethoxy-carbonylmethoxy)phenyl)-methoxycarbonylmethyl]-1,4,7,10-tetraazacyclododecan (21,1 g; 50 mmol; 62 % d. Th.) und 60 mL (0,35 mol) N-Ethyldiisopropylamin in 200 mL Dichlormethan gibt man zu 62,45 g (0,2 mol) 2-(Trifluormethansulfonyloxy)propansäure-benzylester (Kitazaki et al., Chem. Pharm. Bull. (1999), 47(3), 360) in 400 mL Dichformethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 34,1 g (75 % d. Th.) eines farblosen kristallinen Pulvers

| Elementaranalyse: | | | |
|---|---|---|---|
| ber.: | C 67,38 | H 7,10 | N 6,16 |
| gef.: | C 67,20 | H 7,33 | N 6,31 |

d) 10-[α-(4-(Ethoxycarbonylmethoxy)phenyl)-methoxycarbonylmethyl]-1,4,7-α,α',α''-tri-methyl-1,4,7-tris(carboxymethyl)-1,4,7,10-tetraazacyclododecan

27,3 g (30 mmol) der Titelverbindung aus Beispiel 5c werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 19,3 g (quantitativ) eines farblosen Pulvers

| Elementaranalyse: | | | |
|---|---|---|---|
| ber.: | C 56,42 | H 7,26 | N 8,77 |
| gef.: | C 56,21 | H 7,56 | N 8,47 |

e) Gd-Komplex des 10-[α-(4-Carboxymethoxyphenyl)-carboxymethyl]-1,4,7-α,α',α''-trimethyl-1,4,7-tris(carboxymethyl)-1,4,7,10-tetraazacyclododecans

13,3 g (20 mmol) der Titelverbindung aus Beispiel 5d werden in 250 mL 2N Natronlauge und 250 mL Tetrahydrofuran aufgenommen und 5 Tage bei 40 °C gerührt. Anschließend wird die wäßrige Phase, mit Amberlite IR-120® ($H^+$-Form) auf pH 7 gestellt, es werden 80 mL Isopropanol zugefügt und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 8,6 g (61 % d. Th.) eines farblosen Pulvers.
Wassergehalt (Karl-Fischer): 9,3 %

| Elementaranalyse (bezogen auf die wasserfreie Substanz): | | | | |
|---|---|---|---|---|
| ber.: | C 43,19 | H 4,97 | Gd 20,94 | N 7,46 |
| gef.: | C 43,22 | H 5,29 | Gd 20,42 | N 7,11 |

**Vergleichsbeispiel 6**

a) 4-(Ethoxycarbonylpropoxy)-phenylessigsäuremethylester

10 g (60,2 mmol) Hydroxyphenylessigsäuremethylester (Aldrich) werden in 75 mL Aceton gelöst. Es werden 18,4 g (133 mmol) festes Kaliumcarbonat zugegeben, 17,8 mL (123 mmol) 4-Brombuttersäureethylester innerhalb 15 min unter Rückfluß zugetropft, weitere 4 Stunden bei dieser Temperatur gehalten und über Nacht bei Raumtemperatur gerührt. Es wird vom Niederschlag abfiltriert, die Lösung zur Trockne eingedampft und an Kieselgel chromatographiert (Hexan/Essigester 3: 1). Die das Produkt enthaltenden Fraktionen werden vereinigt und eingedampft.
Ausbeute: 16,4 g (97 % d. Th.)

| Elementaranalyse: | | |
|---|---|---|
| ber.: | C 64,27 | H 7,19 |
| gef.: | C 64,41 | H 6,92 |

b) α-Brom-[4-(ethoxycarbonylpropoxy)-phenyl]-essigsäuremethylester

15,0 g (53,5 mmol) der Titelverbindung aus Beispiel 6a werden in 75 mL Tetrachlorkohlenstoff gelöst. Es werden 9,52 g (53,5 mmol) N-Bromsuccinimid und 48 mg Dibenzoylperoxid zugegeben, 5 Stunden unter Rückfluß erhitzt und über Nacht bei Raumtemperatur gerührt. Die Suspension wird zweimal mit Natriumhydrogencarbonat-Lösung und einmal mit Wasser gewaschen, die organische Phase mit Magnesiumsulfat getrocknet, vom Trocknungsmittel abfiltriert und das Filtrat im Vakuum zur Trockne eingedampft. Der Rückstand wird an Kieselgel chromatographiert (Hexan/Essigester 3:1). Die das Produkt enthaltenden Fraktionen werden vereinigt und eingedampft.
Ausbeute: 15,9 g (83 % d. Th.)

| Elementaranalyse: | | | |
|---|---|---|---|
| ber.: | C 50,16 | H 5,33 | Br 22,24 |
| gef.: | C 50,33 | H 5,04 | Br 21,94 |

c) 10-[α-(4-(Ethoxycarbonylpropoxy)phenyl)-methoxycarbonylmethyl]-1,4,7-α,α',α''-trimethyl-1,4,7-tris(benzyloxycarbonylmethyl)-1,4,7,10-tetraazacyclododecan

Zu 27,9 g (162,2 mmol) 1,4,7,10-Tetraazacyclododecan, gelöst in 300 mL Chloroform, gibt man 29,1 g (81,1 mmol) der im vorstehenden Beispiel 6b beschriebenen Bromverbindung und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Dichlormethan/Methanol/Triethylamin = 10/5/0,1). Das so erhaltene 1-[α-(4-(Ethoxy-carbonylpropoxy)phenyl)methoxycarbonylmethyl]-1,4,7,10-tetraazacyclododecan (22,5 g; 50 mmol; 62 % d. Th.) und 60 mL (0,35 mol) N-Ethyldiisopropylamin in 200 mL Dichlormethan gibt man zu 62,45 g (0,2 mol) 2-(Trifluormethansulfonyloxy)propansäure-benzylester (Kitazaki et al., Chem. Pharm. Bull. (1999), 47(3), 360) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 30,5 g (65 % d. Th.) eines farblosen kristallinen Pulvers

| Elementaranalyse: | | | |
|---|---|---|---|
| ber.: | C 67,93 | H 7,31 | N 5,98 |
| gef.: | C 67,95 | H 7,22 | N 6,13 |

d) 10-[α-(4-(Ethoxycarbonylpropoxy)phenyl)-methoxycarbonylmethyl]-1,4,7-α,α',α"-tri-methyl-1,4,7-tris(carboxymethyl)-1,4, 7,10-tetraazacyclododecan

28,1 g (30 mmol) der Titelverbindung aus Beispiel 6c werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 20,0 g (quantitativ) eines farblosen Pulvers

| Elementaranalyse: | | | |
|---|---|---|---|
| ber.: | C 57,64 | H 7,56 | N 8,40 |
| gef.: | C 57,43 | H 7,77 | N 8,69 |

e) Gd-Komplex des 10-[α-(4-Carboxypropoxyphenyl)-carboxymethyl]-1,4,7-α,α',α"-trimethyl-1,4,7-tris(carboxymethyl)-1,4,7,10-tetraazacyclododecans

13,3 g (20 mmol) der Titelverbindung aus Beispiel 6d werden in 250 mL 2N Natronlauge und 250 mL Tetrahydrofuran aufgenommen und 5 Tage bei 40 °C gerührt. Anschließend wird die wäßrige Phase mit Amberlite IR-120® ($H^+$-Form) auf pH 7 gestellt, es werden 80 mL Isopropanol zugefügt und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 9,3 g (55 % d. Th.) eines farblosen Pulvers.
Wassergehalt (Karl-Fischer): 8,0 %

| Elementaranalyse (bezogen auf die wasserfreie Substanz): | | | | |
|---|---|---|---|---|
| ber.: | C 44,72 | H 5,31 | Gd 20,19 | N 7,19 |
| gef.: | C 44,31 | H 5,88 | Gd 19,93 | N 7,11 |

**Vergleichsbeispiel 7**

a) 4-(Ethoxycarbonyldecyloxy)-phenylessigsäuremethylester

10 g (60,2 mmol) Hydroxyphenylessigsäuremethylester (Aldrich) werden in 75 mL Aceton gelöst. Es werden 18,4 g (133 mmol) festes Kaliumcarbonat zugegeben, 36,1 g (123 mmol) ω-Bromundecansäureethylester in 50 mL Aceton zugetropft, 8 Stunden unter Rückfluß gekocht und über Nacht bei Raumtemperatur gerührt. Es wird vom Ungelösten abfiltriert, die Lösung zur Trockne eingedampft und an Kieselgel chromatographiert (Hexan/Essigester 3:1). Die das Produkt enthaltenden Fraktionen werden vereinigt und eingedampft.
Ausbeute: 20,3 g (89 % d. Th.)

| Elementaranalyse: | | |
|---|---|---|
| ber.: | C 69,81 | H 9,05 |
| gef.: | C 69,50 | H 8,91 |

b) α-Brom-[4-(ethoxycarbonyldecyloxy)-phenyl]-essigsäuremethylester

20,2 g (53,5 mmol) der Titelverbindung aus Beispiel 7a werden in 75 mL Tetrachlorkohlenstoff gelöst. Es werden 9,52 g (53,5 mmol) N-Bromsuccinimid und 48 mg Dibenzoylperoxid zugegeben, 5 Stunden unter Rückfluß erhitzt und über Nacht bei Raumtemperatur gerührt. Die Suspension wird zweimal mit Natriumhydrogencarbonat-Lösung und einmal mit Wasser gewaschen, die organische Phase mit Magnesiumsulfat getrocknet, vom Trocknungsmittel abfiltriert und das Filtrat im Vakuum zur Trockne eingedampft. Der Rückstand wird an Kieselgel chromatographiert (Hexan/Essigester 3:1). Die das Produkt enthaltenden Fraktionen werden vereinigt und eingedampft.
Ausbeute: 21,0 g (86 % d. Th.)

| Elementaranalyse: | | | |
|---|---|---|---|
| ber.: | C 57,77 | H 7,27 | Br 17,47 |
| gef.: | C 57,95 | H 7,41 | Br 17,02 |

c) 10-[α-(4-(Ethoxycarbonyldecyloxy)phenyl)-methoxycarbonylmethyl]-1,4,7-α,α',α''-trimethyl-1,4,7-tris(benzyloxycarbonylmethyl)-1,4,7,10-tetraazacyclododecan

Zu 27,9 g (162,2 mmol) 1,4,7,10-Tetraazacyclododecan, gelöst in 300 mL Chloroform, gibt man 37,1 g (81,1 mmol) der im vorstehenden Beispiel 7b beschriebenen Bromverbindung und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Dichlormethan/Methanol/Triethylamin = 10/510,1). Das so erhaltene 1-[α-(4-(Ethoxy-carbonyldecyloxy)phenyl)-methoxycarbonylmethyl]-1,4,7,10-tetraazacyclododecan (27,4 g; 50 mmol; 62 % d. Th.) und 60 mL (0,35 mol) N-Ethyldiisopropylamin in 200 mL Dichlormethan gibt man zu 62,45 g (0,2 mol) 2-(Trifluormethansulfonyloxy)propansäure-benzylester (Kitazaki et al., Chem. Pharm. Bull. (1999), 47(3), 360) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 33,6 g (65 % d. Th.) eines farblosen kristallinen Pulvers

| Elementaranalyse: | | | |
|---|---|---|---|
| ber.: | C 69,61 | H 7,98 | N 5,41 |
| gef.: | C 69,75 | H 7,88 | N 5,12 |

d) 10-[α-(4-(Ethoxycarbonyldecyloxy)phenyl)-methoxycarbonylmethyl]-1,4,7-α,α',α''-trimethyl-1,4,7-tris(carboxymethyl)-1,4,7,10-tetraazacyclododecan

31,1 g (30 mmol) der Titelverbindung aus Beispiel 7c werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 23,0 g (quantitativ) eines farblosen Pulvers

| Elementaranalyse: | | | |
|---|---|---|---|
| ber.: | C 61,24 | H 8,43 | N 7,32 |
| gef.: | C 60,96 | H 8,61 | N 7,22 |

e) Gd-Komplex des 10-[α-(4-Carboxydecyloxyphenyl)-carboxymethyl]-1,4,7-α,α',α''-trimethyl-1,4,7-tris(carboxymethyl)-1,4,7,10-tetraazacyclododecans

15,3 g (20 mmol) der Titelverbindung aus Beispiel 7d werden in 250 mL 2N Natronlauge und 250 mL Tetrahydrofuran aufgenommen und 5 Tage bei 40 °C gerührt. Anschließend wird die wäßrige Phase mit Amberlite IR-120® ($H^+$-Form) auf pH 7 gestellt, es werden 80 mL Isopropanol zugefügt und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 11,5 g (60 % d. Th.) eines farblosen Pulvers.
Wassergehalt (Karl-Fischer): 8,5 %

| Elementaranalyse (bezogen auf die wasserfreie Substanz): | | | | |
|---|---|---|---|---|
| ber.: | C 49,30 | H 6,32 | Gd 17,93 | N 6,39 |
| gef.: | C 49,56 | H 6,10 | Gd 17,52 | N 6,63 |

**Vergleichsbeispiel 8**

a) 10-(p-Methoxycarbonylbenzyl)-1,4,7-α,α',α''-trimethyl-1,4,7-tris(benzyloxycarbonyl-methyl)-1,4,7,10-tetraazacyclododecan

Zu 27,9 g (162,2 mmol) 1,4,7,10-Tetraazacyclododecan, gelöst in 300 mL Chloroform, gibt man 18,6 g (81,1 mmol) 4-Brommethyl-benzoesäuremethylester (Aldrich) in 150 mL Chloroform und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Methanol/aqu. 25 % Ammoniak = 8/1). Das so erhaltene 1-(p-Methoxycarbonylbenzyl)-1,4,7,10-tetraazacyclodo-decan (21,6 g; 67,3 mmol; 83 % d. Th.) und 60 mL (0,35 mol) N-Ethyldiisopropylamin in 200 mL Dichlormethan gibt man zu 62,45 g (0,2 mol) 2-(Trifluormethansulfonyloxy)propansäure-benzylester (Kitazaki et al., Chem. Pharm. Bull. (1999), 47(3), 360) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 41,8 g (77 % d. Th.) eines farblosen kristallinen Pulvers

| Elementaranalyse: | | | |
|---|---|---|---|
| ber.: | C 69,95 | H 7,24 | N 6,94 |
| gef.: | C 69,57 | H 7,39 | N 7,12 |

b) 10-(p-Carboxybenzyl)-1,4,7-α,α',α''-trimethyl-1,4,7-tris(carboxymethyl)-1,4,7,10-tetraazacyclododecan

24,2 g (30 mmol) der Titelverbindung aus Beispiel 8a werden in 400 mL Methanol gelöst, mit 100 mL 15 N Natronlauge versetzt, 6 Stunden unter Rückfluß gekocht und über Nacht bei Raumtemperatur gerührt. Nach Eindampfen im Vakuum wird der Rückstand in 200 mL Wasser gelöst und durch Zugabe von IR-120®-Kationenaustauscher ($H^+$-Form) auf pH 7 gestellt. Es wird vom Austauscher abfiltriert im Vakuum zur Trockne eingedampft. Der Rückstand wird ohne weitere Charakterisierung komplexiert. Dünnschicht-System: n-Butanol/aqu. Ammoniak/Ethanol/Wasser 12/6/3/3
Ausbeute: 16 g

c) Gd-Komplex des 10-(p-Carboxybenzyl)-1,4,7-α,α',α''-trimethyl-1,4,7-tris(carboxymethyl)-1,4,7,10-tetraazacyclododecans

11 g (20 mmol) des in Beispiel 8b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 8,9 g (61 % d. Th.) eines farblosen Pulvers.
Wassergehalt (Karl-Fischer): 7,2 %

| Elementaranalyse (bezogen auf die wasserfreie Substanz): | | | | |
|---|---|---|---|---|
| ber.: | C 44,37 | H 5,21 | Gd 23,23 | N 8,28 |
| gef.: | C 44,12 | H 5,46 | Gd 22,93 | N 8,51 |

**Vergleichsbeispiel 9**

a) 10-(p-Methoxycarbonylbenzyl)-1,4,7-α,α',α''-tris(isopropyl)-1,4,7-tris(benzyloxycarbonyl-methyl)-1,4,7,10-tetraazacyclododecan

21,6 g (67,3 mmol) des in Beispiel 8a als Zwischenprodukt beschriebenen 1-(p-Methoxycarbonylbenzyl)-1,4,7,10-tetraazacyclododecans und 60 mL (0,35 mol) N-Ethyldiisopropylamin in 200 mL Dichlormethan gibt man zu 85,1 g (0,25 mol) 2-(Trifluormethan-sulfonyloxy)-isovaleriansäurebenzylester (Walker et al., Tetrahedron (1997), 53(43),

14591) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 48,5 g (81 % d. Th.) eines farblosen kristallinen Pulvers

| Elementaranalyse: | | | |
|---|---|---|---|
| ber.: | C 71,43 | H 7,92 | N 6.29 |
| gef.: | C 71,12 | H 7,79 | N 6,55 |

b) 10-(p-Carboxybenzyl)-1,4,7-α,α',α"-tris(isopropyl)-1,4,7-tris(carboxymethyl)-1,4,7,10-tetraazacyclododecan

26,7 g (30 mmol) der Titelverbindung aus Beispiel 9a werden in 400 mL Methanol gelöst, mit 100 mL 15 N Natronlauge versetzt, 6 Stunden unter Rückfluß gekocht und über Nacht bei Raumtemperatur gerührt. Nach Eindampfen im Vakuum wird der Rückstand in 200 mL Wasser gelöst und durch Zugabe von IR-120®-Kationenaustauscher ($H^+$-Form) auf pH 7 gestellt. Es wird vom Austauscher abfiltriert im Vakuum zur Trockne eingedampft. Der Rückstand wird ohne weitere Charakterisierung komplexiert.
Dünnschicht-System: n-Butanol/aqu. Ammoniak/Ethanol/Wasser 12/6/3/3
Ausbeute: 19 g

c) Gd-Komplex des 10-(p-Carboxybenzyl)-1,4,7-α,α',α"-tris(isopropyl)-1,4,7-tris(carboxymethyl)-1,4,7,10-tetraazacyclododecans

12,6 g (20 mmol) des in Beispiel 9b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 10,9 g (65 % d. Th.) eines farblosen Pulvers.
Wassergehalt (Karl-Fischer): 9,0 %

| Elementaranalyse (bezogen auf die wasserfreie Substanz): | | | | |
|---|---|---|---|---|
| ber.: | C 48,93 | H 6,23 | Gd 20,66 | N 7,36 |
| gef.: | C 48,87 | H 6,01 | Gd 20,22 | N 7,59 |

**Vergleichsbeispiel 10**

a) 10-(p-Methoxycarbonylbenzyl)-1,4,7-α,α',α"-tris(cyclohexyl)-1,4,7-tris(benzyloxycarbonyl-methyl)-1,4,7,10-tetraazacyclododecan

21,6 g (67,3 mmol) des in Beispiel 8a als Zwischenprodukt beschriebenen 1-(p-Methoxycarbonylbenzyl)-1,4,7,10-tetraazacyclododecans und 60 mL (0,35 mol) N-Ethyldiisopropylamin in 200 mL Dichlormethan gibt man zu 95,1 g (0,25 mol) 2-(Trifluormethan-sulfonyloxy)-2-cyclohexylessigsäurebenzylester (Qabar et al., Tetrahedron Letters (1998), 39(33), 5895) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 48,3 g (71 % d. Th.) eines farblosen kristallinen Pulvers

| Elementaranalyse: | | | |
|---|---|---|---|
| ber.: | C 73,63 | H 8,17 | N 5,54 |
| gef.: | C 73,42 | H 8,39 | N 5,75 |

b) 10-(p-Carboxybenzyl)-1,4,7-α,α',α''-tris(cyclohexyl)-1,4,7-tris(carboxymethyl)-1,4,7,10-tetraazacyclododecan

30,3 g (30 mmol) der Titelverbindung aus Beispiel 10a werden in 400 mL Methanol gelöst, mit 100 mL 15 N Natronlauge versetzt, 6 Stunden unter Rückfluß gekocht und über Nacht bei Raumtemperatur gerührt. Nach Eindampfen im Vakuum wird der Rückstand in 200 mL Wasser gelöst und durch Zugabe von IR-120®-Kationenaustauscher ($H^+$-Form) auf pH 7 gestellt. Es wird vom Austauscher abfiltriert im Vakuum zur Trockne eingedampft. Der Rückstand wird ohne weitere Charakterisierung komplexiert. Dünnschicht-System: n-Butanol/aqu. Ammoniak/Ethanol/Wasser 12/6/3/3
Ausbeute: 22,5 g

c) Gd-Komplex des 10-(p-Carboxybenzyl)-1,4,7-α,α',α''-tris(cyclohexyl)-1,4,7-tris(carboxymethyl)-1,4,7,10-tetraazacyclododecans

15,0 g (20 mmol) des in Beispiel 10b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan/Methanoi/Ammoniak: 20/20/1). Die das Produkt enthaltenden Fraktionen werden vereinigt und zur Trockne eingedampft. Der Rückstand wird mit Ameisensäure aufgenommen und mehrfach unter Zusatz von Dichlormethan zur Trockne eingedampft und anschließend bis zur Gewichtskonstanz im Vakuum getrocknet.
Ausbeute: 11,9 g (63 % d. Th.) eines farblosen Pulvers.
Wassergehalt (Karl-Fischer): 7,0 %

Elementaranalyse (bezogen auf die wasserfreie Substanz):

| | | | | |
|---|---|---|---|---|
| ber.: | C 54,52 | H 6,75 | Gd 17,85 | N 6,36 |
| gef.: | C 54,19 | H 6,83 | Gd 17,61 | N 6,69 |

**Vergleichsbeispiel 11**

a) 10-(p-Methoxycarbonylbenzyl)-1,4,7-α,α',α''-triphenyl-1,4,7-tris(benzyloxycarbonyl-methyl)-1,4,7,10-tetraazacyclododecan

21,6 g (67,3 mmol) des in Beispiel 8a als Zwischenprodukt beschriebenen 1-(p-Methoxycarbonylbenzyl)-1,4,7,10-tetraazacyclododecans und 60 mL (0,35 mol) N-Ethyldiisopropylamin in 200 mL Dichlormethan gibt man zu 93,6 g (0,25 mol) 2-(Trifluormethan-sulfonyloxy)-2-phenylessigsäurebenzylester (Qabar et al., Tetrahedron Letters (1998), 39(33), 5895) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 50,8 g (76 % d. Th.) eines farblosen kristallinen Pulvers

Elementaranalyse:

| | | | |
|---|---|---|---|
| ber.: | C 74,98 | H 6,49 | N 5,64 |
| gef.: | C 75,22 | H 6,61 | N 5,47 |

b) 10-(p-Carboxybenzyl)-1,4,7-α,α',α''-triphenyl-1,4,7-tris(carboxymethyl)-1,4,7,10-tetraazacyclododecan

29,8 g (30 mmol) der Titelverbindung aus Beispiel 11a werden in 400 mL Methanol gelöst, mit 100 mL 15 N Natronlauge versetzt, 6 Stunden unter Rückfluß gekocht und über Nacht bei Raumtemperatur gerührt. Nach Eindampfen im Vakuum wird der Rückstand in 200 mL Wasser gelöst und durch Zugabe von IR-120®-Kationenaustauscher ($H^+$-Form) auf pH 7 gestellt. Es wird vom Austauscher abfiltriert im Vakuum zur Trockne eingedampft. Der Rückstand wird ohne weitere Charakterisierung komplexiert. Dünnschicht-System: n-Butanol/aqu. Ammoniak/Ethanol/Wasser 12/6/3/3
Ausbeute: 22,0 g

c) Gd-Komplex des 10-(p-Carboxybenzyl)-1,4,7-α,α',α''-triphenyl-1,4,7-tris(carboxymethyl)-1,4,7,10-tetraazacyclododecans

14,6 g (20 mmol) des in Beispiel 11 b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und zur Trockne eingedampft. Der Rückstand wird mit Ameisensäure aufgenommen und mehrfach unter Zusatz von Dichlormethan zur Trockne eingedampft und anschließend bis zur Gewichtskonstanz im Vakuum getrocknet.
Ausbeute: 13,1 g (70 % d. Th.) eines farblosen Pulvers.
Wassergehalt (Karl-Fischer): 8,1 %

Elementaranalyse (bezogen auf die wasserfreie Substanz):

| | | | | |
|---|---|---|---|---|
| ber.: | C 55,67 | H 4,79 | Gd 18,22 | N 6,49 |
| gef.: | C 55,33 | H 4,97 | Gd 17,92 | N 6,54 |

**Beispiel 12**

a) 10-[4-(t-Butoxycarbonyl)-1-phenyl-2-oxo-3-azabutyl]-1,4,7-α,α',α''-triphenyl-1,4,7-tris-(benzyloxycarbonylmethyl)-1,4,7,10-tetraazacyclododecan

Zu 27,9 g (162,2 mmol) 1,4,7,10-Tetraazacyclododecan, gelöst in 300 mL Chloroform, gibt man 26,6 g (81,1 mmol) N-[2-Brom-2-phenylacetyl]-glycin-t-butylester (Beispiel 6a der WO 98/24775) und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Chloro-form/Methanollaqu. 25 % Ammoniak = 10/5/1). Das so erhaltene 1-[4-(t-Butoxycarbonyl)-1-phenyl-2-oxo-3-azabutyl]-1,4,7,10-tetraazacyclododecan (21,0 g; 50 mmol; 62 % d. Th.) und 60 mL (0,35 mol) N-Ethyldiisopropylamin in 200 mL Dichlormethan gibt man zu 74,9 g (0,2 mol) 2-(Trifluormethansulfonyloxy)-2-phenylessigsäurebenzylester (Qabar et al., Tetrahedron Letters (1998), 39(33), 5895) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 30/1). Die das Produkt enthaltenden Fraktionen werden vereinigt und eingedampft.
Ausbeute: 37,7 g (69 % d. Th.) eines farblosen kristallinen Pulvers

Elementaranalyse:

| | | | |
|---|---|---|---|
| ber.: | C 73,67 | H 6,74 | N 6,41 |
| gef.: | C 73,44 | H 6,43 | N 6,79 |

b) 10-(4-(*t*-Butoxycarbonyl-1-phenyl-2-oxo-3-azabutyl)-1,4,7-α,α',α''-triphenyl-1,4,7-tris(carboxy-methyl)-1,4,7,10-tetraazacyclododecan

32,8 g (30 mmol) der Titelverbindung aus Beispiel 12a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 24,8 g (quantitativ) eines farblosen Pulvers

Elementaranalyse:

| | | | |
|---|---|---|---|
| ber.: | C 67,22 | H 6,74 | N 8,52 |
| gef.: | C 67,00 | H 6,85 | N 8,23 |

c) Gd-Komplex des 10-(4-Carboxy-1-phenyl-2-oxo-3-azabutyl)-1,4,7-α,α',α''-triphenyl-1,4,7-tris(carboxymethyl)-1,4,7,10-tetraazacyclododecans

16,4 g (20 mmol) des in Beispiel 12b beschriebenen *t*-Butylesters werden in sehr wenig Trifluoressigsäure

gelöst und 15 min bei Raumtemperatur gerührt. Nach Zugabe von 250 mL Diethylether wird 2 Stunden nachgerührt, der Niederschlag abgesaugt und im Vakuum getrocknet. Der so erhaltene freie Ligand wird in 200 mL Wasser und 80 mL Isopropanol gelöst, mit verdünntem Ammoniak auf pH 7 eingestellt und durch Zugabe von 5 mL Essigsäure angesäuert. Es werden 3,6 g (10 mmol) Gadoliniumoxid zugegeben und 3 Stunden am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 25/15/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 11,7 g (59 % d. Th.) eines farblosen Pulvers.
Wassergehalt (Karl-Fischer): 7,5 %

| Elementaranalyse (bezogen auf die wasserfreie Substanz): | | | | |
|---|---|---|---|---|
| ber.: | C 54,83 | H 4,82 | Gd 17,09 | N 7,61 |
| gef.: | C 54,91 | H 4,67 | Gd 16,62 | N 7,33 |

**Beispiel 13**

a) 10-[4-(Benzyloxycarbonyl)-2-oxo-3-azabutyl]-1,4,7-α,α',α''-tris(isopropyl)-1,4,7-tris(benzyloxy-carbonylmethyl)-1,4,7,10-tetraazacyclododecan

Zu 34,4 g (0,2 mol) 1,4,7,10-Tetraazacyclododecan, gelöst in 300 mL Chloroform, gibt man 23,2 g (81,1 mmol) 2-Bromacetylglycin-benzylester (Teger-Nilsson et al., WO 93/11152, Seite 38) und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Chloro-form/Methanol/aqu. 25 % Ammoniak = 10/5/1). Das so erhaltene 1-[4-(Benzyloxycarbonyl)-2-oxo-3-azabutyl]-1,4,7,10-tetraazacyclododecan (19,6 g; 50 mmol; 62 % d. Th.) und 60 mL (0,35 mol) N-Ethyldiisopropylamin in 200 mL Dichlormethan gibt man zu 68,1 g (0,2 mol) 2-(Trifluormethansulfonyloxy)-isovaleriansäurebenzylester (Walker et al., Tetrahedron (1997), 53 (43), 14591) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 37,0 g (78 % d. Th.) eines farblosen kristallinen Pulvers

| Elementaranalyse: | | | |
|---|---|---|---|
| ber.: | C 69,67 | H 7,76 | N 7,39 |
| gef.: | C 69,51 | H 7,88 | N 7,39 |

b) 10-(4-Carboxy-2-oxo-3-azabutyl)-1,4,7-α,α',α''-tris(isopropyl)-1,4,7-tris(carboxymethyl)-1,4,7,10-tetraazacyclododecan

28,4 g (30 mmol) der Titelverbindung aus Beispiel 13a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 17,7 g (quantitativ) eines farblosen Pulvers

| Elementaranalyse: | | | |
|---|---|---|---|
| ber.: | C 55,18 | H 8,40 | N 11,92 |
| gef.: | C 54,97 | H 8,70 | N 11,88 |

c) Gd-Komplex des 10-(4-Carboxy-2-oxo-3-azabutyl)-1,4,7-α,α',α''-tris(isopropyl)-1,4,7-tris(carboxymethyl)-1,4,7,10-tetraazacyclododecans

11,8 g (20 mmol) des in Beispiel 13b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak auf pH 7,4 eingestellt und an Kieselgel chromatographiert (Laufmittel: Dichiormethan/Methanol/Ammoniak: 20/20/1). Die das Produkt enthaltenden Fraktionen

werden vereinigt und über eine IR-120®-Kationenaustauschersäule ($H^+$-Form) gegeben. Das saure Eluat wird gefriergetrocknet.
Ausbeute: 12,1 g (75 % d. Th.) eines farblosen Pulvers.
Wassergehalt (Karl-Fischer): 8,0 %

| Elementaranalyse (bezogen auf die wasserfreie Substanz): | | | | |
|---|---|---|---|---|
| ber.: | C 43,71 | H 6,25 | Gd 21,19 | N 9,44 |
| gef.: | C 43,90 | H 6,40 | Gd 20,80 | N 9,33 |

**Beispiel 14**

a) 10-[4-(Benzyloxycarbonyl)-2-oxo-3-azabutyl]-1,4,7-α,α',α''-tris(cyclohexyl)-1,4,7-tris-(benzyloxycarbonylmethyl)-1,4,7,10-tetraazacyclododecan

18,9 g (50 mmol) des in Beispiel 13a als Zwischenprodukt beschriebenen 1-[4-(Benzyloxycarbonyl)-2-oxo-3-azabutyl]-1,4,7,10-tetraazacyclododecans und 60 mL (0,35 mol) N-Ethyldiisopropylamin in 200 mL Dichlormethan gibt man zu 76,1 g (0,2 mol) 2-(Trifluormethansulfonyloxy)-2-cyclohexylessigsäurebenzylester (Qabar et al., Tetrahedron Letters (1998), 39(33), 5895) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan/Methanol: 20/1). Die das Produkt enthaltenden Fraktionen werden vereinigt und eingedampft.
Ausbeute: 38,5 g (72 % d. Th.) eines farblosen kristallinen Pulvers

| Elementaranalyse: | | | |
|---|---|---|---|
| ber.: | C 71,95 | H 8,02 | N 6,56 |
| gef.: | C 71,90 | H 8,21 | N 6,73 |

b) 10-(4-Carboxy-2-oxo-3-azabutyl)-1,4,7-α,α',α''-tris(cyclohexyl)-1,4,7-tris(carboxymethyl)-1,4,7,10-tetraazacyclododecan

32,1 g (30 mmol) der Titelverbindung aus Beispiel 14a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 21,2 g (quantitativ) eines farblosen Pulvers

| Elementaranalyse: | | | |
|---|---|---|---|
| ber.: | C 61,08 | H 8,69 | N 9,89 |
| gef.: | C 61,27 | H 8,55 | N 9,41 |

c) Gd-Komplex des 10-(4-Carboxy-2-oxo-3-azabutyl)-1,4,7-α,α',α''-tris(cyclohexyl)-1,4,7-tris(carboxymethyl)-1,4,7,10-tetraazacyclododecans

14,2 g (20 mmol) des in Beispiel 14b beschriebenen Liganden werden in 150 mL Wasser und 150 mL Isopropanol gelöst und durch Zugabe von 5 mL Essigsäure angesäuert. Es werden 3,6 g (10 mmol) Gadoliniumoxid zugegeben und 8 h am Rückfluß erhitzt. Nach beendeter Komplexierung wird mit Ammoniak erneut auf pH 7,4 eingestellt und an Kieselgel chromatographiert (Laufmittel: Dichlormethan/Methanol/Ammoniak: 20/20/1). Die das Produkt enthaltenden Fraktionen werden vereinigt und zur Trockne eingedampft. Der Rückstand wird mit Ameisensäure aufgenommen und mehrfach unter Zusatz von Dichlormethan zur Trockne eingedampft und anschließend bis zur Gewichtskonstanz im Vakuum getrocknet.
Ausbeute: 13,5 g (71 % d. Th.) eines farblosen Pulvers.
Wassergehalt (Karl-Fischer): 9,0 %

| Elementaranalyse (bezogen auf die wasserfreie Substanz): | | | | |
|---|---|---|---|---|
| ber.: | C 50,15 | H 6,78 | Gd 18,24 | N 8,12 |
| gef.: | C 49,92 | H 6,51 | Gd 18,01 | N 8,31 |

**Beispiel 15**

a) 10-[4-(Benzyloxycarbonyl)-1-methyl-2-oxo-3-azabutyl]-2,5,8,11-tetramethyl-1,4,7,10-tetraazacyclododecan-1,4,7-triessigsäure-tri-t-butylester, Natriumbromid-Komplex

Zu 1,14 g (5 mmol) 2,5,8,11-Tetramethyl-1,4,7,10-tetraazacyclododecan (Petrov et al., DE 19608307; Ranganathan et al., WO 95/31444), gelöst in 10 mL Chloroform, gibt man 0,50 g (1,67 mmol) 2-Brompropionylglycin-benzylester (Beispiel 1e der WO 98/24774) und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak = 1015/1). Zu dem so erhaltenen 1-[4-(Benzyloxycarbonyl)-1-methyl-2-oxo-3-azabutyl]-2,5,8,11-Tetramethyl-1,4,7,10-tetraazacyclododecan (0,70 g; 1,27 mmol; 76 % d. Th.) und 541 mg (5,1 mmol) Natriumcarbonat in 5 mL Acetonitril gibt man 822 mg (4,2 mmol) Bromessigsäure-tert.-butylester und rührt 12 Stunden bei 60°C. Man kühlt auf 0°C und filtriert von den Salzen ab. Das Filtrat wird zur Trockne eingedampft und der Rückstand an Kieselgel chromatographiert (Laufmittel: Methylenchlorid/Methanol= 20:1).
Ausbeute: 964 mg (85 % d. Th.) eines farblosen Feststoffes

| Elementaranalyse: | | | | | |
|---|---|---|---|---|---|
| ber.: | C 56,49 | H 8.01 | N 7,84 | Na 2,57 | Br 8,95 |
| gef.: | C 56,37 | H 7,88 | N 7,61 | Na 2,33 | Br 8,59 |

b) 10-(4-Carboxy-1-methyl-2-oxo-3-azabutyl)-2,5,8,11-tetramethyl-1,4,7,10-tetraazacyclododecan-1,4,7-triessigsäure-tri-tert-butylester (Natriumbromid-Komplex)

893 mg (1,0 mmol) der Titelverbindung aus Beispiel 15a löst man in 10 mL Isopropanol und gibt eine Spatelspitze Palladiumkatalysator (10 % Pd/C) zu. Man hydriert über Nacht bei Raumtemperatur. Es wird vom Katalysator abfiltriert und das Filtrat zur Trockne eingedampft. Der Rückstand wird aus Dioxan umkristallisiert.
Ausbeute: 562 mg (70 % d. Th.) eines kristallinen Feststoffes

| Elementaranalyse: | | | | | |
|---|---|---|---|---|---|
| ber.: | C 52,36 | H8,16 | N 8,72 | Na 2,86 | Br 9,95 |
| gef.: | C 52,51 | H 8,30 | N 8,93 | Na 2,71 | Br 9,44 |

c) Gadolinium-Komplex der 10-(4-Carboxy-1-methyl-2-oxo-3-azabutyl)-2,5,8,11-tetramethyl-1,4,7,10-tetraazacyclododecan-1,4,7-triessigsäure

803 mg (1,0 mmol) der Titelverbindung aus Beispiel 15b werden in 5 mL Trifluoressigsäure gelöst und 3 Stunden bei Raumtemperatur gerührt. Man dampft zur Trockne ein, nimmt den Rückstand in 300 mL Wasser auf und gibt die Lösung auf eine Säule, gefüllt mit Reillex® 425 PVP. Man eluiert mit Wasser. Die produkthaltigen Fraktionen werden vereinigt und zur Trockne eingedampft (446 mg; 0,84 mmol) und erneut in 4 mL Wasser gelöst. Man gibt 152 mg (0,42 mmol) Gadoliniumoxid zu und erwärmt 3 h auf 90°C. Man dampft zur Trockne ein (Vakuum) und kristallisiert den Rückstand aus 90 % aqu. Ethanol um. Die Kristalle werden abgesaugt, einmal mit Ethanol, dann mit Aceton und zum Schluß mit Dimethylether gewaschen und im Vakuumofen bei 130°C getrocknet (24 Stunden).
Ausbeute: 469 mg (65 % d. Th.) eines farblosen kristallinen Pulvers
Wassergehalt: 5 %

| Elementaranalyse (berechnet auf wasserfreie Substanz): | | | | |
|---|---|---|---|---|
| ber.: | C 40,28 | H 5,58 | N 10,21 | Gd 22,93 |
| gef.: | C 40,06 | H 5,75 | N 10,43 | Gd 22,40 |

**Beispiel 16**

Gd-Komplex des 10-[8-(N-Maleimido)-1-methyl-2,5-dioxo-3,6-diazaoctyl]-1,4,7-$\alpha,\alpha',\alpha''$-tris-(isopropyl)-1,4,7-tris(carboxymethyl)-1,4,7,10-tetraazacyclododecans

2,27 g (3 mmol) der in Beispiel 2 beschriebenen Gd-Komplexsäure werden in 15 mL DMF gelöst, unter Eiskühlung mit 380 mg (3,3 mmol) N-Hydroxysuccinimd und 681 mg (3,3 mmol) Dicyclo-hexylcarbodiimid versetzt und 1 Stunde im Eis voraktiviert. Anschließend wird eine Mischung aus 839 mg (3,3 mmol) N-(2-Aminoethyl)maleimid Trifluoracetatsalz (Arano et al., J. Med. Chem., 1996, 39, 3458) und 0,7 mL (4 mmol) N,N-Diisopropylethyl-amin in 10 mL DMF zugegeben und über Nacht bei Raumtemperatur gerührt. Die Reaktionsmischung wird erneut im Eisbad gekühlt, filtriert und das Filtrat im Vakuum zur Trockne eingedampft. Der Rückstand wird an Kieselgel chromatographiert (Laufmittel: Dichlormethan/Methanol: 1/1).
Ausbeute: 997 mg (35 % d. Th.)
Wassergehalt (Karl-Fischer): 7,5 %

Elementaranalyse (bezogen auf die wasserfreie Substanz):

| | | | | |
|---|---|---|---|---|
| ber.: | C 46,51 | H 6,20 | Gd 17,91 | N 11,17 |
| gef.: | C 46,28 | H 6,44 | Gd 17,31 | N 11,26 |

**Beispiel 17**

Gd-Komplex des 10-[8-(N-Maleimido)-1-methyl-2,5-dioxo-3,6-diazaoctyl]-1,4,7-α,α',α''-tris-(cyclohexyl)-1,4,7-tris(carboxymethyl)-1,4,7,10-tetraazacyclododecans

2,63 g (3 mmol) der in Beispiel 3 beschriebenen Gd-komplexsäure werden in 15 mL DMF gelöst, unter Eiskühlung mit 380 mg (3,3 mmol) N-Hydroxysuccinimd und 681 mg (3,3 mmol) Dicyclo-hexylcarbodiimid versetzt und 1 Stunde im Eis voraktiviert. Anschließend wird eine Mischung aus 839 mg (3,3 mmol) N-(2-Aminoethyl)maleimid Trifluoracetatsalz (Arano et al., J. Med. Chem., 1996, 39, 3458) und 0,7 mL (4 mmol) N,N-Diisopropylethyl-amin in 10 mL DMF zugegeben und über Nacht bei Raumtemperatur gerührt. Die Reaktionsmischung wird erneut im Eisbad gekühlt, filtriert und das Filtrat im Vakuum zur Trockne eingedampft. Der Rückstand wird an Kieselgel chromatographiert (Laufmittel: Dichlormethan/Methanol: 1/1). Ausbeute: 1,24 g (39 % d. Th.) Wassergehalt (Karl-Fischer): 6,0 %

Elementaranalyse (bezogen auf die wasserfreie Substanz):

| | | | | |
|---|---|---|---|---|
| ber.: | C 51,74 | H 6,66 | Gd 15,75 | N 9,82 |
| gef.: | C 51,77 | H 6,41 | Gd 15,25 | N 10,02 |

**Beispiel 18**

a) (3-Brom-2-oxo-pyrrolidin-1-yl)essigsäurebenzylester

67,7 g (0,2 mol) Glycinbenzylester Tosylat und 61,2 ml (0,44 mol) Triethylamin werden in 200 mL Methylenchlorid gelöst und bei 0 °C zu einer Lösung von 52,9 g (0,2 mol) 2,4-Dibrombuttersäurechlorid (Gramain et al. Synth. Commun. (1997), (27), 1827) in 200 ml Methylenchlorid innerhalb 45 min zugetropft und 18 h bei Raumtemperatur gerührt. Die Reaktionsmischung wird nun bei 0 °C zu einer Lösung von 400 ml wässriger 32proz. Natriumhydroxid und 2 g Tetrabutylammoniumhydrogencarbonat getropft (ca. 15 min) und 30 min gerührt. Anschließend weden die Phasen getrennt, und die wäßrige Phase dreimal mit je 200 ml Dichlormethan extrahiert. Die organischen Phasen werden über Natriumsulfat getrocknet, die Lösung zur Trockne eingedampft und an Kieselgel chromatographiert (Methylenchlorid). Die das Produkt enthaltenden Fraktionen werden vereinigt und eingedampft.
Ausbeute: 29,3 g (47 % d. Th.)

Elementaranalyse:

| | | | |
|---|---|---|---|
| ber.: | C 50,02 | H 4,52 | N 4,49 |
| gef.: | C 50,34 | H 4,44 | N 4,41 |

b) 10-[1-(Benzyloxycarbonytmethyl)-2-oxo-pyrrolidin-3-yl]-1,4,7-α,α',α''-trimethyl-1,4,7-tris-(benzyloxycarbonylmethyl)-1,4,7,10-tetraazacyclododecan

Zu 28,7 g (165,8 mmol) 1,4,7,10-Tetraazacyclododecan, gelöst in 300 mL Chloroform, gibt man 20,7 g (66,3 mmol) (3-Brom-2-oxo-pyrrolidin-1-yl)essigsäurebenzylester und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak = 10/5/1). Das so erhaltene 1-[1-(Benzyloxycarbonylmethyl)-2-oxo-pyrrolidin-3-yl]-1,4,7,10-tetraazacyclododecan (20,9 g; 51,8 mmol; 78 % d. Th.) und 60 mL (0,35 mol) N-Ethyldiisopropylamin in 200 mL Dichlormethan gibt man zu 62,45 g (0,2 mol) 2-(Trifluormethan-sulfonyloxy) propansäurebenzylester (Kitazaki et al., Chem. Pharm. Bull. (1999), 47(3), 360) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.
Ausbeute: 32,7 g (71 % d. Th.) eines farblosen kristallinen Pulvers

| Elementaranalyse: | | | |
|---|---|---|---|
| ber.: | C 68,82 | H 7,13 | N 7,87 |
| gef.: | C 68,54 | H 7,28 | N 8,01 |

c) 10-[1-(Carboxymethyl)-2-oxo-pyrrolidin-3-yl]-1,4,7-α, α', α''-trimethyl-1,4,7-tris(carboxymethyl)-1,4,7,10-tetraazacyclododecan

26,7 g (30 mmol) der Titelverbindung aus Beispiel 18b werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 15,8 g (quantitativ) eines farblosen Pulvers

| Elementaranalyse: | | | |
|---|---|---|---|
| ber.: | C 52,16 | H 7,42 | N 13,22 |
| gef.: | C 52,32 | H 7,35 | N 13,11 |

d) Gd-Komplex des 10-[1-(Carboxymethyl)-2-oxo-pyrrolidin-3-yl]-1,4,7-α, α', α''-trimethyl-1,4,7-tris(carboxymethyl)-1,4,7,10-tetraazacyclododecans

10,6 g (20 mmol) des in Beispiel 18c beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 9,7 g (67 % d. Th.) eines farblosen Pulvers.
Wassergehalt (Karl-Fischer): 8,3 %

| Elementaranalyse (bezogen auf die wasserfreie Substanz): | | | | |
|---|---|---|---|---|
| ber.: | C 40,40 | H 5,31 | Gd 23,00 | N 10,24 |
| gef.: | C 39,99 | H 5,55 | Gd 22,93 | N 10,45 |

**Beispiel 19**

a) 10-[1-(Benzyloxycarbonylmethyl)-2-oxo-pyrrolidin-3-yl]-1,4,7-α,α',α''-tris(isopropyl)-1,4,7-tris(benzyloxycarbonylmethyl)-1,4,7,10-tetraazacyclododecan

20,2 g (50 mmol) des in Beispiel 18b als Zwischenprodukt beschriebenen 1-[1-(Benzyloxycarbonylmethyl)-2-oxo-pyrrolidin-3-yl]-1,4,7,10-tetraazacyclododecans und 60 mL (0,35 mol) N-Ethyldiisopropylamin in 200 mL Dichlormethan gibt man zu 68,1 g (0,2 mol) 2-(Trifluormethansulfonyloxy)-isovaleriansäurebenzylester (Walker et al., Tetrahedron (1997), 53(43), 14591) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.
Ausbeute: 34,1 g (70 % d. Th.) eines farblosen kristallinen Pulvers

| Elementaranalyse: | | | |
|---|---|---|---|
| ber.: | C 70,27 | H 7,76 | N 7,19 |
| gef.: | C 70,45 | H 7,61 | N 7,11 |

b) 10-[1-(Carboxymethyl)-2-oxo-pyrrolidin-3-yl]-1,4,7-α,α',α''-tris(isopropyl)-1,4,7-tris(carboxymethyl)-1,4,7,10-tetraazacyclododecan

29,2 g (30 mmol) der Titelverbindung aus Beispiel 19a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 18,4 g (quantitativ) eines farblosen Pulvers

| Elementaranalyse: | | | |
|---|---|---|---|
| ber.: | C 56,75 | H 8,38 | N 11,41 |
| gef.: | C 56,89 | H 8,31 | N 11,37 |

c) Gd-Komplex des 10-[1-(Carboxymethyl)-2-oxo-pyrrolidin-3-yl]-1,4,7-α, α', α''-tris(iso-propyl)-1,4,7-tris(carboxymethyl)-1,4,7,10-tetraazacyclododecans

12,3 g (20 mmol) des in Beispiel 19b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 11,9 g (75 % d. Th.) eines farblosen Pulvers.
Wassergehalt (Karl-Fischer): 8,2 %

| Elementaranalyse (bezogen auf die wasserfreie Substanz): | | | | |
|---|---|---|---|---|
| ber.: | C 45,36 | H 6,30 | Gd 20,48 | N 9,12 |
| gef.: | C 45,89 | H 6,22 | Gd 20,23 | N 9,01 |

In analoger Weise erhält man unter Verwendung von 12,3 g (20 mmol) des in Beispiel 19b beschriebenen Liganden und 3,73 g (10 mmol) Dysprosiumoxid anstelle von Gadoliniumoxid den Dy-Komplex des 10-[1-(Carboxymethyl)-2-oxo-pyrrolidin-3-yl]-1,4,7-α, α', α''-tris(iso-propyl)-1,4,7-tris(carboxymethyl)-1,4,7,10-tetraazacyclododecans.
Ausbeute: 11,4 g (71 % d. Th.) eines farblosen Pulvers,
Wassergehalt (Karl-Fischer): 8,0 %

| Elementaranalyse (bezogen auf die wasserfreie Substanz): | | | | |
|---|---|---|---|---|
| ber.: | C 45,05 | H 6,26 | Dy 21,02 | N 9,06 |
| gef.: | C 45,35 | H 6,22 | Dy 20,88 | N 9,04 |

**Beispiel 20**

a) 10-[1-(Benzyloxycarbonylmethyl)-2-oxo-pyrrolidin-3-yl]-1,4,7-α,α',α''-tris(cyclohexyl)-1,4,7-tris(benzyloxycarbonylmethyl)-1,4,7,10-tetraazacyclododecan

20,2 g (50 mmol) des in Beispiel 18b als Zwischenprodukt beschriebenen 1-[1-(Benzyloxycarbonylmethyl)-2-oxo-pyrrolidin-3-yl]-1,4,7,10-tetraazacyclododecans und 60 mL (0,35 mol) N-Ethyldiisopropylamin in 200 mL Dichlormethan gibt man zu 76,1 g (0,2 mol) 2-(Trifluormethansulfonyloxy)-2-cyclohexylessigsäurebenzylester (Qabar et al., Tetrahedron Letters (1998), 39(33), 5895) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.

Ausbeute: 37,2 g (68 % d. Th.) eines farblosen kristallinen Pulvers

| Elementaranalyse: | | | |
|---|---|---|---|
| ber.: | C 72,43 | H 8,01 | N 6,40 |
| gef.: | C 72,55 | H 7,98 | N 6,35 |

b) 10-[1-(Carboxymethyl)-2-oxo-pyrrolidin-3-yl]-1,4,7-α,α',α"-tris(cyclohexyl)-1,4,7-tris(carboxymethyl)-1,4,7,10-tetraazacyclododecan

32,8 g (30 mmol) der Titelverbindung aus Beispiel 20a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 22,0 g (quantitativ) eines farblosen Pulvers

| Elementaranalyse: | | | |
|---|---|---|---|
| ber.: | C 62,19 | H 8,65 | N 9,54 |
| gef.: | C 62,44 | H8,56 | N 9,46 |

c) Gd-Komplex des 10-[1-(Carboxymethyl)-2-oxo-pyrrolidin-3-yl]-1,4,7-α,α',α"-tris(cyclohexyl)-1,4,7-tris(carboxymethyl)-1,4,7,10-tetraazacyclododecans

14,6 g (20 mmol) des in Beispiel 20b beschriebenen Liganden werden in 150 mL Wasser und 150 mL Isopropanol gelöst und durch Zugabe von 5 mL Essigsäure angesäuert. Es werden 3,6 g (10 mmol) Gadoliniumoxid zugegeben und 8 h am Rückfluß erhitzt. Nach beendeter Komplexierung wird mit Ammoniak erneut auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und zur Trockne eingedampft. Der Rückstand wird mit Ameisensäure aufgenommen und mehrfach unter Zusatz von Dichlormethan zur Trockne eingedampft und anschließend bis zur Gewichtskonstanz im Vakuum getrocknet.
Ausbeute: 12,1 g (65 % d. Th.) eines farblosen Pulvers.
Wassergehalt (Karl-Fischer): 7,0 %

| Elementaranalyse (bezogen auf die wasserfreie Substanz): | | | | |
|---|---|---|---|---|
| ber.: | C 51,39 | H 6,81 | Gd 17,70 | N 7,89 |
| gef.: | C 51,64 | H 6,77 | Gd 17,44 | N 7,77 |

**Beispiel 21**

a) (3-Brom-2-oxo-pyrrolidin-1-yl)benzoesäurebenzylester

45,5 g (0,2 mol) 4-Aminobenzoesäurebenzylester und 30,6 ml (0,22 mol) Triethylamin werden in 200 mL Methylenchlorid gelöst und bei 0 °C zu einer Lösung von 52,9 g (0,2 mol) 2,4-Dibrombuttersäurechlorid (Gramain et al. Synth. Commun. (1997), (27), 1827) in 200 ml Methylenchlorid innerhalb 45 min zugetropft und 18 h bei Raumtemperatur gerührt. Die Reaktionsmischung wird nun bei 0 °C zu einer Lösung von 400 ml wässriger 32proz. Natriumhydroxid und 2 g Tetrabutylammoniumhydrogencarbonat getropft (ca. 15 min) und 30 min gerührt. Anschließend weden die Phasen getrennt, und die wäßrige Phase dreimal mit je 200 ml Dichlormethan extrahiert. Die organischen Phasen werden über Natriumsulfat getrocknet, die Lösung zur Trockne eingedampft und an Kieselgel chromatographiert (Methylenchlorid). Die das Produkt enthaltenden Fraktionen werden vereinigt und eingedampft.
Ausbeute: 38,2 g (51 % d. Th.)

| Elementaranalyse: | | | |
|---|---|---|---|
| ber.: | C 57,77 | H 4,31 | N 3,74 |
| gef.: | C 57,99 | H 4,27 | N 3,66 |

b) 10-[1-(4-Benzyloxycarbonylphenyl)-2-oxo-pyrrolidin-3-yl]-1,4,7-α,α',α''-trimethyl-1,4,7-tris-(benzyloxycarbonylmethyl)-1,4,7,10-tetraazacyclododecan

Zu 31,2 g (180 mmol) 1,4,7,10-Tetraazacyclododecan, gelöst in 300 mL Chloroform, gibt man 26,9 g (71,9 mmol) (3-Brom-2-oxo-pyrrolidin-1-yl)benzoesäurebenzylester und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak = 10/5/1). Das so erhaltene 1-[1-(4-Benzyloxycarbonylphenyl)-2-oxo-pyrrolidin-3-yl]-1,4,7,10-tetraazacyclododecan (26,1 g; 56,1 mmol; 78 % d. Th.) und 60 mL (0,35 mol) N-Ethyldiisopropylamin in 200 mL Dichlormethan gibt man zu 62,45 g (0,2 mol) 2-(Trifluormethan- sulfonyloxy) propansäurebenzylester (Kitazaki et al., Chem. Pharm. Bull. (1999), 47(3), 360) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.
Ausbeute: 36,3 g (68 % d. Th.) eines farblosen kristallinen Pulvers

| Elementaranalyse: | | | |
|---|---|---|---|
| ber.: | C 70,64 | H 6,88 | N 7,36 |
| gef.: | C 70,89 | H 6,81 | N 7,29 |

c) 10-[1-(4-Carboxyphenyl)-2-oxo-pyrrolidin-3-yl]-1,4,7-α, α', α''-trimethyl-1,4,7-tris(carboxymethyl)-1,4,7,10-tetraazacyclododecan

28,6 g (30 mmol) der Titelverbindung aus Beispiel 21b werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 17,7 g (quantitativ) eines farblosen Pulvers

| Elementaranalyse: | | | |
|---|---|---|---|
| ber.: | C 56,84 | H 6,98 | N 11,84 |
| gef.: | C 57,04 | H 6,91 | N 11,79 |

d) Gd-Komplex des 10-[1-(4-Carboxyphenyl)-2-oxo-pyrrolidin-3-yl]-1,4,7-α, α', α''-trimethyl-1,4,7-tris(carboxymethyl)-1,4,7,10-tetraazacyclododecans

11,8 g (20 mmol) des in Beispiel 21 c beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 11,1 g (71 % d. Th.) eines farblosen Pulvers.
Wassergehalt (Karl-Fischer): 7,5 %

| Elementaranalyse (bezogen auf die wasserfreie Substanz): | | | | |
|---|---|---|---|---|
| ber.: | C 45,09 | H 5,13 | Gd 21,08 | N 9,39 |
| gef.: | C 45,45 | H 5,11 | Gd 20,78 | N 9,40 |

**Beispiel 22**

a) 10-[1-(4-Benzyloxycarbonylphenyl)-2-oxo-pyrrolidin-3-yl]-1,4,7-α,α',α''-tris(isopropyl)-1,4,7-tris(benzyloxycarbonylmethyl)-1,4,7,10-tetraazacyclododecan

23,3 g (50 mmol) des in Beispiel 21b als Zwischenprodukt beschriebenen 1-[1-(4-Benzyloxycarbonylphenyl)-2-oxo-pyrrolidin-3-yl]-1,4,7,10-tetraazacyclododecans und 60 mL (0,35 mol) N-Ethyldiisopropylamin in 200 mL Dichlormethan gibt man zu 68,1 g (0,2 mol) 2-(Trifluormethansulfonyloxy)-isovaleriansäurebenzylester (Walker et al., Tetrahedron (1997), 53(43), 14591) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.
Ausbeute: 35,3 g (68 % d. Th.) eines farblosen kristallinen Pulvers

| Elementaranalyse: | | | |
|---|---|---|---|
| ber.: | C 71,86 | H 7,49 | N6,76 |
| gef.: | C 71,99 | H 7,46 | N 6,71 |

b) 10-[1-(4-Carboxyphenyl)-2-oxo-pyrrolidin-3-yl]-1,4,7-α,α',α''-tris(isopropyl)-1,4,7-tris(carboxymethyl)-1,4,7,10-tetraazacyclododecan

31,1 g (30 mmol) der Titelverbindung aus Beispiel 22a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 20,2 g (quantitativ) eines farblosen Pulvers

| Elementaranalyse: | | | |
|---|---|---|---|
| ber.: | C 60,43 | H 7.90 | N 10,36 |
| gef.: | C 60,59 | H 7,82 | N 10,31 |

c) Gd-Komplex des 10-[1-(4-Carboxyphenyl)-2-oxo-pyrrolidin-3-yl]-1,4,7-α, α', α''-tris(isopropyl)-1,4,7-tris(carboxymethyl)-1,4,7,10-tetraazacyclododecans

13,5 g (20 mmol) des in Beispiel 22b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 12,4 g (72 % d. Th.) eines farblosen Pulvers.
Wassergehalt (Karl-Fischer): 7,8 %

| Elementaranalyse (bezogen auf die wasserfreie Substanz): | | | | |
|---|---|---|---|---|
| ber.: | C 49,20 | H 6,07 | Gd 18,94 | N 8,44 |
| gef.: | C 49,51 | H 6,04 | Gd 18,71 | N 8,45 |

In analoger Weise erhält man unter Verwendung von 13,5 g (20 mmol) des in Beispiel 22b beschriebenen Liganden und 3,73 g (10 mmol) Dysprosiumoxid anstelle von Gadoliniumoxid den Dy-Komplex des 10-[1-(4-Carboxyphenyl)-2-oxo-pyrrolidin-3-yl]-1,4,7-α, α', α''-tris(isopropyl)-1,4,7-tris(carboxymethyl)-1,4,7,10-tetraazacyclododecans
Ausbeute: 13,0 g (75 % d. Th.) eines farblosen Pulvers.
Wassergehalt (Karl-Fischer): 7,5 %

| Elementaranalyse (bezogen auf die wasserfreie Substanz): | | | | |
|---|---|---|---|---|
| ber.: | C 48,89 | H 6,03 | Dy 19,45 | N 8,38 |
| gef.: | C 49,11 | H 6,04 | Dy 19,22 | N 8,36 |

**Beispiel 23**

a) 10-[1-(4-Benzyloxycarbonylphenyl)-2-oxo-pyrrolidin-3-yl]-1,4,7-α,α',α''-tris(cyclohexyl)-1,4,7-tris(benzyloxycarbonylmethyl)-1,4,7,10-tetraazacyclododecan

23,3 g (50 mmol) des in Beispiel 21b als Zwischenprodukt beschriebenen 1-[1-(4-Benzyloxycarbonylphenyl)-2-oxo-pyrrolidin-3-yl]-1,4,7,10-tetraazacyclododecans und 60 mL (0,35 mol) N-Ethyldiisopropylamin in 200 mL Dichlormethan gibt man zu 76,1 g (0,2 mol) 2-(Trifluormethansulfonyloxy)-2-cyclohexylessigsäurebenzylester (Qabar et al., Tetrahedron Letters (1998), 39(33), 5895) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.
Ausbeute: 41,1 g (71 % d. Th.) eines farblosen kristallinen Pulvers

| Elementaranalyse: | | | |
|---|---|---|---|
| ber.: | C 73,74 | H 7,76 | N 6,06 |
| gef.: | C 73,91 | H 7,69 | N 6,01 |

b) 10-[1-(4-Carboxyphenyl)-2-oxo-pyrrolidin-3-yl]-1,4,7-α,α',α"-tris(cyclohexyl)-1,4,7-tris-(carboxymethyl)-1,4,7,10-tetraazacyclododecan

34,7 g (30 mmol) der Titelverbindung aus Beispiel 23a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 23,8 g (quantitativ) eines farblosen Pulvers

| Elementaranalyse: | | | |
|---|---|---|---|
| ber.: | C 64,88 | H 8,23 | N 8,80 |
| gef.: | C 65,04 | H 8,19 | N 8,70 |

c) Gd-Komplex des 10-[1-(4-Carboxyphenyl)-2-oxo-pyrrolidin-3-yl]-1,4,7-α,α',α"-tris(cyclohexyl)-1,4,7-tris(carboxymethyl)-1,4,7,10-tetraazacyclododecans

15,9 g (20 mmol) des in Beispiel 23b beschriebenen Liganden werden in 150 mL Wasser und 150 mL Isopropanol gelöst und durch Zugabe von 5 mL Essigsäure angesäuert. Es werden 3,6 g (10 mmol) Gadoliniumoxid zugegeben und 8 h am Rückfluß erhitzt. Nach beendeter Komplexierung wird mit Ammoniak erneut auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und zur Trockne eingedampft. Der Rückstand wird mit Ameisensäure aufgenommen und mehrfach unter Zusatz von Dichlormethan zur Trockne eingedampft und anschließend bis zur Gewichtskonstanz im Vakuum getrocknet.
Ausbeute: 12,9 g (65 % d. Th.) eines farblosen Pulvers.
Wassergehalt (Karl-Fischer): 7,0 %

| Elementaranalyse (bezogen auf die wasserfreie Substanz): | | | | |
|---|---|---|---|---|
| ber.: | C 54,35 | H 6,58 | Gd 16,55 | N 7,37 |
| gef.: | C 54,66 | H 6,57 | Gd 16,32 | N 7,32 |

**Beispiel 24**

a) (3-Brom-2-oxo-piperidin-1-yl)essigsäurebenzylester

67,7 g (0.2 mol) Glycinbenzylester Tosylat und 61,2 ml (0,44 mol) Triethylamin werden in 200 mL Methylenchlorid gelöst und bei 0 °C zu einer Lösung von 55,7 g (0,2 mol) 2,5-Dibromvaleriansäurechlorid (Okawara et al. Chem. Pharm. Bull. (1982), (30), 1225) in 200 ml Methylenchlorid innerhalb 45 min zugetropft und 18 h bei Raumtemperatur gerührt. Die Reaktionsmischung wird nun bei 0 °C zu einer Lösung von 400 ml wässriger 32proz. Natriumhydroxid und 2 g Tetrabutylammoniumhydrogencarbonat getropft (ca. 15 min) und 30 min gerührt. Anschließend weden die Phasen getrennt, und die wäßrige Phase dreimal mit je 200 ml Dichlormethan extrahiert. Die organischen Phasen werden über Natriumsulfat getrocknet, die Lösung zur Trockne eingedampft und an Kieselgel chromatographiert (Methylenchlorid). Die das Produkt enthaltenden Fraktionen werden vereinigt und eingedampft.
Ausbeute: 33,2 g (51 % d. Th.)

| Elementaranalyse: | | | |
|---|---|---|---|
| ber.: | C 51,55 | H 4,94 | N 4,29 |
| gef.: | C 51,86 | H 4,91 | N 4,18 |

b) 10-[1-(Benzyloxycarbonylmethyl)-2-oxo-piperidin-3-yl]-1,4,7-α,α',α"-trimethyl-1,4,7-tris-(benzyloxycarbonylmethyl)-1,4,7,10-tetraazacyclododecan

Zu 30,3 g (175 mmol) 1,4,7,10-Tetraazacyclododecan, gelöst in 300 mL Chloroform, gibt man 18,9 g (58 mmol) (3-Brom-2-oxo-piperidin-1-yl)essigsäurebenzylester und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak = 10/5/1). Das so erhaltene 1-[1-(Benzyloxycarbonylmethyl)-2-oxo-piperidin-3-yl]-1,4,7,10-tetraazacyclododecan (20,3 g; 48,6 mmol; 84 % d. Th.) und 60 mL (0,35 mol) N-Ethyldiisopropylamin in 200 mL Dichlormethan gibt man zu 62,45 g (0,2 mol) 2-(Trifluormethan-sulfonyloxy)propansäurebenzylester (Kitazaki et al., Chem. Pharm. Bull. (1999), 47(3), 360) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.
Ausbeute: 32,5 g (74 % d. Th.) eines farblosen kristallinen Pulvers

| Elementaranalyse: | | | |
|---|---|---|---|
| ber.: | C 69,08 | H 7,25 | N 7,75 |
| gef.: | C 69,34 | H 7,19 | N 7,66 |

c) 10-[1-(Carboxymethyl)-2-oxo-piperidin-3-yl]-1,4,7-α, α', α"-trimethyl-1,4,7-tris(carboxymethyl)-1,4,7,10-tetraazacyclododecan

27,1 g (30 mmol) der Titelverbindung aus Beispiel 24b werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 16,3 g (quantitativ) eines farblosen Pulvers

| Elementaranalyse: | | | |
|---|---|---|---|
| ber.: | C 53,03 | H 7,60 | N 12,88 |
| gef.: | C 53,34 | H 7,54 | N 12,79 |

d) Gd-Komplex des 10-[1-(Carboxymethyl)-2-oxo-piperidin-3-yl]-1,4,7-α, α', α"-trimethyl-1,4,7-tris(carboxymethyl)-1,4,7,10-tetraazacyclododecans

10.9 g (20 mmol) des in Beispiel 24c beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 9,6 g (65 % d. Th.) eines farblosen Pulvers.
Wassergehalt (Karl-Fischer): 7,2 %

| Elementaranalyse (bezogen auf die wasserfreie Substanz): | | | | |
|---|---|---|---|---|
| ber.: | C 41,31 | H 5,49 | Gd 22,53 | N 10,04 |
| gef.: | C 41,67 | H 5,48 | Gd 22,21 | N 9,97 |

**Beispiel 25**

a) 10-[1-(Benzyloxycarbonylmethyl)-2-oxo-piperidin-3-yl]-1,4,7-α,α',α''-tris(isopropyl)-1,4,7-tris(benzyloxycarbonylmethyl)-1,4,7,10-tetraazacyclododecan

20,9 g (50 mmol) des in Beispiel 24b als Zwischenprodukt beschriebenen 1-[1-(Benzyloxycarbonylmethyl)-2-oxo-piperidin-3-yl]-1,4,7,10-tetraazacyclododecans und 60 mL (0,35 mol) N-Ethyldiisopropylamin in 200 mL Dichlormethan gibt man zu 68,1 g (0,2 mol) 2-(Trifluormethansulfonyloxy)-isovaleriansäurebenzylester (Walker et al., Tetrahedron (1997), 53(43), 14591) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.
Ausbeute: 36,2 g (73 % d. Th.) eines farblosen kristallinen Pulvers

| Elementaranalyse: | | | |
|---|---|---|---|
| ber.: | C 70,49 | H 7,85 | N 7,09 |
| gef.: | C 70,61 | H 7,83 | N 7,01 |

b) 10-[1-(Carboxymethyl)-2-oxo-piperidin-3-yl]-1,4,7-α,α',α''-tris(isopropyl)-1,4,7-tris(carboxymethyl)-1,4,7,10-tetraazacyclododecan

29,6 g (30 mmol) der Titelverbindung aus Beispiel 25a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 18,8 g (quantitativ) eines farblosen Pulvers

| Elementaranalyse: | | | |
|---|---|---|---|
| ber.: | C 57,40 | H 8,51 | N 11,16 |
| gef.: | C 57,64 | H 8,45 | N 11,09 |

c) Gd-Komplex des 10-[1-(Carboxymethyl)-2-oxo-piperidin-3-yl]-1,4,7-α, α', α''-tris(isopropyl)-1,4,7-tris(carboxymethyl)-1,4,7,10-tetraazacyclododecans

12,6 g (20 mmol) des in Beispiel 25b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 11,7 g (71 % d. Th.) eines farblosen Pulvers.
Wassergehalt (Karl-Fischer): 8,1 %

| Elementaranalyse (bezogen auf die wasserfreie Substanz): | | | | |
|---|---|---|---|---|
| ber.: | C 46,08 | H 6,44 | Gd 20,11 | N 8,96 |
| gef.: | C 46,34 | H 6,41 | Gd 19,99 | N 8,91 |

In analoger Weise erhält man unter Verwendung von 12,6 g (20 mmol) des in Beispiel 25b beschriebenen Liganden und 3,73 g (10 mmol) Dysprosiumoxid anstelle von Gadoliniumoxid den Dy-Komplex des 10-[1-(Carboxymethyl)-2-oxo-piperidin-3-yl]-1,4,7-α,α',α''-tris(isopropyl)-1,4,7-tris(carboxymethyl)-1,4,7,10-tetraazacyclododecans.
Ausbeute: 10,8 g (66 % d. Th.) eines farblosen Pulvers.
Wassergehalt (Karl-Fischer): 7,6 %

| Elementaranalyse (bezogen auf die wasserfreie Substanz): | | | | |
|---|---|---|---|---|
| ber.: | C 45,77 | H 6,40 | Dy 20,64 | N 8,90 |
| gef.: | C 46,01 | H 6,46 | Dy 20,34 | N 8,91 |

**Beispiel 26**

a) 10-[1-(Benzyloxycarbonylmethyl)-2-oxo-piperidin-3-yl]-1,4,7-α,α',α''-tris(cyclohexyl)-1,4,7-tris(benzyloxycarbonyl-methyl)-1,4,7,10-tetraazacyclododecan

20,9 g (50 mmol) des in Beispiel 24b als Zwischenprodukt beschriebenen 1-[1-(Benzyloxycarbonylmethyl)-2-oxo-piperidin-3-yl]-1,4,7,10-tetraazacyclododecans und 60 mL (0,35 mol) N-Ethyldiisopropylamin in 200 mL Dichlormethan gibt man zu 76,1 g (0,2 mol) 2-(Trifluormethansulfonyloxy)-2-cyclohexylessigsäurebenzylester (Qabar et al., Tetrahedron Letters (1998), 39(33), 5895) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.
Ausbeute: 39,8 g (72 % d. Th.) eines farblosen kristallinen Pulvers

Elementaranalyse:

| | | | |
|---|---|---|---|
| ber.: | C 72,60 | H 8,09 | N 6,32 |
| gef.: | C 72,89 | H 7,98 | N 6,27 |

b) 10-[1-(Carboxymethyl)-2-oxo-piperidin-3-yl)-1,4,7-α,α',α''-tris(cyclohexyl)-1,4,7-tris(carboxymethyl)-1,4,7,10-tetraazacyclododecan

33,3 g (30 mmol) der Titelverbindung aus Beispiel 26a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 22,4 g (quantitativ) eines farblosen Pulvers

Elementaranalyse:

| | | | |
|---|---|---|---|
| ber.: | C 62,63 | H 8,76 | N 9,36 |
| gef.: | C 62,77 | H 8,71 | N 9,29 |

c) Gd-Komplex des 10-[1-(Carboxymethyl)-2-oxo-piperidin-3-yl]-1,4,7-α,α',α''-tris(cyclohexyl)-1,4,7-tris(carboxymethyl)-1,4,7,10-tetraazacyclododecans

14,9 g (20 mmol) des in Beispiel 26b beschriebenen Liganden werden in 150 mL Wasser und 150 mL Isopropanol gelöst und durch Zugabe von 5 mL Essigsäure angesäuert. Es werden 3,6 g (10 mmol) Gadoliniumoxid zugegeben und 8 h am Rückfluß erhitzt. Nach beendeter Komplexierung wird mit Ammoniak erneut auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und zur Trockne eingedampft. Der Rückstand wird mit Ameisensäure aufgenommen und mehrfach unter Zusatz von Dichlormethan zur Trockne eingedampft und anschließend bis zur Gewichtskonstanz im Vakuum getrocknet. Ausbeute: 12,9 g (68 % d. Th.) eines farblosen Pulvers.
Wassergehalt (Karl-Fischer): 7,6 %

Elementaranalyse (bezogen auf die wasserfreie Substanz):

| | | | | |
|---|---|---|---|---|
| ber.: | C 51,92 | H 6,93 | Gd 17,43 | N 7,76 |
| gef.: | C 52,09 | H 6,88 | Gd 17,21 | N 7,77 |

**Beispiel 27**

a) (3-Brom-2-oxo-piperidin-1-yl)benzoesäurebenzylester

45,5 g (0,2 mol) 4-Aminobenzoesäurebenzylester und 30,6 ml (0,22 mol) Triethylamin werden in 200 mL Methylenchlorid gelöst und bei 0 °C zu einer Lösung von 55,3 g (0,2 mol) 2,5-Dibromvaleriansäurechlorid (Okawara et al. Chem. Pharm. Bull. (1982), (30), 1225) in 200 ml Methylenchlorid innerhalb 45 min zugetropft und 18 h bei Raumtemperatur gerührt. Die Reaktionsmischung wird nun bei 0 °C zu einer Lösung von 400 ml wässriger 32proz. Natriumhydroxid und 2 g Tetrabutylammoniumhydrogencarbonat getropft (ca. 15 min) und 30 min gerührt. Anschließend weden die Phasen getrennt, und die wäßrige Phase dreimal mit je 200 ml Dichlormethan extrahiert. Die organischen Phasen werden

über Natriumsulfat getrocknet, die Lösung zur Trockne eingedampft und an Kieselgel chromatographiert (Methylenchlorid). Die das Produkt enthaltenden Fraktionen werden vereinigt und eingedampft.
Ausbeute: 38,8 g (50 % d. Th.)

| Elementaranalyse: | | | |
|---|---|---|---|
| ber.: | C 58,78 | H 4,67 | N 3,61 |
| gef.: | C 59,01 | H 4,50 | N 3,59 |

b) 10-[1-(4-Benzyloxycarbonylphenyl)-2-oxo-piperidin-3-yl]-1,4,7-α,α',α''-trimethyl-1,4,7-tris-(benzyloxycarbonylmethyl)-1,4,7,10-tetraazacyclododecan

Zu 31,2 g (180 mmol) 1,4,7,10-Tetraazacyclododecan, gelöst in 300 mL Chloroform, gibt man 26,6 g (68,5 mmol) (3-Brom-2-oxo-piperidin-1-yl)benzoesäurebenzylester und rührt über Nacht bei Raumtemperatur. Man gibt 250 mL Wasser zu, trennt die organische Phase ab und wäscht sie noch jeweils zweimal mit 200 mL Wasser. Man trocknet die organische Phase über Magnesiumsulfat und dampft im Vakuum zur Trockne ein. Der Rückstand wird an Kieselgel chromatographiert (Laufmittel: Chloroform/Methanol/aqu. 25 % Ammoniak = 10/5/1). Das so erhaltene 1-[1-(4-Benzyloxycarbonylphenyl)-2-oxo-piperidin-3-yl]-1,4,7,10-tetraazacyclododecan (27,6 g; 57,5 mmol; 84 % d. Th.) und 60 mL (0,35 mol) N-Ethyldiiso-propylamin in 200 mL Dichlormethan gibt man zu 62,45 g (0,2 mol) 2-(Trifluormethan-sulfonyloxy) propansäurebenzylester (Kitazaki et al., Chem. Pharm. Bull. (1999), 47(3), 360) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 2011). Die das Produkt enthaltenden Fraktionen werden vereinigt und eingedampft.
Ausbeute: 39,4 g (71 % d. Th.) eines farblosen kristallinen Pulvers

| Elementaranalyse: | | | |
|---|---|---|---|
| ber.: | C 70,86 | H 6,99 | N 7,25 |
| gef.: | C 71,11 | H 6,81 | N 7,17 |

c) 10-[1-(4-Carboxyphenyl)-2-oxo-piperidin-3-yl]-1,4,7-α, α', α''-trimethyl-1,4,7-tris(carboxymethyl)-1,4,7,10-tetraazacyclododecan

29,0 g (30 mmol) der Titelverbindung aus Beispiel 27b werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 18,1 g (quantitativ) eines farblosen Pulvers

| Elementaranalyse: | | | |
|---|---|---|---|
| ber.: | C 57,51 | H 7,16 | N 11,56 |
| gef.: | C 57,72 | H 7,11 | N 11,50 |

d) Gd-Komplex des 10-[1-(4-Carboxyphenyl)-2-oxo-piperidin-3-yl]-1,4,7-α, α', α''-trimethyl-1,4,7-tris(carboxymethyl)-1,4,7,10-tetraazacyclododecans

12,1 g (20 mmol) des in Beispiel 27c beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak erneut auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 11,4 g (72 % d. Th.) eines farblosen Pulvers.
Wassergehalt (Karl-Fischer): 7,1 %

| Elementaranalyse (bezogen auf die wasserfreie Substanz): | | | | |
|---|---|---|---|---|
| ber.: | C 45,84 | H 5,31 | Gd 20,69 | N 9,22 |

(fortgesetzt)

| Elementaranalyse (bezogen auf die wasserfreie Substanz): | | | | |
|---|---|---|---|---|
| gef.: | C 45,99 | H 5,26 | Gd 20,55 | N 9,21 |

**Beispiel 28**

a) 10-[1-(4-Benzyloxycarbonylphenyl)-2-oxo-piperidin-3-yl]-1,4,7-α,α',α''-tris(isopropyl)-1,4,7-tris(benzyloxycarbonylmethyl)-1,4,7,10-tetraazacyclododecan

24,0 g (50 mmol) des in Beispiel 27b als Zwischenprodukt beschriebenen 1-[1-(4-Benzyloxycarbonylphenyl)-2-oxo-piperidin-3-yl]-1,4,7,10-tetraazacyclododecans und 60 mL (0,35 mol) N-Ethyldiisopropylamin in 200 mL Dichlormethan gibt man zu 68,1 g (0,2 mol) 2-(Trifluormethansulfonyloxy)-isovaleriansäurebenzylester (Walker et al., Tetrahedron (1997), 53(43), 14591) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.
Ausbeute: 37,8 g (72 % d. Th.) eines farblosen kristallinen Pulvers

| Elementaranalyse: | | | |
|---|---|---|---|
| ber.: | C 72,04 | H 7,58 | N 6,67 |
| gef.: | C 72,32 | H 7,46 | N 6,59 |

b) 10-[1-(4-Carboxyphenyl)-2-oxo-piperidin-3-yl]-1,4,7-α,α',α''-tris(isopropyl)-1,4,7-tris(carboxymethyl)-1,4,7,10-tetraazacyclododecan

31,5 g (30 mmol) der Titelverbindung aus Beispiel 28a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 20,7 g (quantitativ) eines farblosen Pulvers

| Elementaranalyse: | | | |
|---|---|---|---|
| ber.: | C 60,94 | H 8,04 | N 10,15 |
| gef.: | C 60,87 | H 8,05 | N 10,11 |

c) Gd-Komplex des 10-[1-(4-Carboxyphenyl)-2-oxo-piperidin-3-yl]-1,4,7-α, α', α''-tris(iso-propyl)-1,4,7-tris(carboxymethyl)-1,4,7,10-tetraazacyclododecans

13,8 g (20 mmol) des in Beispiel 28b beschriebenen Liganden werden in 200 mL Wasser und 80 mL Isopropanol gelöst und durch Zugabe von 5 mL Essigsäure angesäuert. Es werden 3,6 g (10 mmol) Gadoliniumoxid zugegeben und 3 h am Rückfluß erhitzt. Nach beendeter Komplexierung wird mit Ammoniak auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und über eine IR-120®-Kationenaustauschersäule ($H^+$-Form) gegeben. Das saure Eluat wird gefriergetrocknet.
Ausbeute: 12,0 g (68 % d. Th.) eines farblosen Pulvers.
Wassergehalt (Karl-Fischer): 7,5 %

| Elementaranalyse (bezogen auf die wasserfreie Substanz): | | | | |
|---|---|---|---|---|
| ber.: | C 49,80 | H 6,21 | Gd 18,63 | N 8,30 |
| gef.: | C 49,99 | H 6,17 | Gd 18,51 | N 8,21 |

In analoger Weise erhält man unter Verwendung von 13,8 g (20 mmol) des in Beispiel 28b beschriebenen Liganden und 3,73 g (10 mmol) Dysprosiumoxid anstelle von Gadoliniumoxid den Dy-Komplex des 10-[1-(4-Carboxyphenyl)-2-oxo-piperidin-3-yl]-1,4,7-α, α', α''-tris(isopropyl)-1,4,7-tris(carboxymethyl)-1,4,7,10-tetraazacyclododecans.
Ausbeute: 12,4 g (70 % d. Th.) eines farblosen Pulvers.
Wassergehalt (Karl-Fischer): 7,5 %

| Elementaranalyse (bezogen auf die wasserfreie Substanz): | | | | |
|---|---|---|---|---|
| ber.: | C 49,50 | H 6,17 | Dy 19,13 | N 8,25 |
| gef.: | C 49,77 | H 6,18 | Dy 18,89 | N 8,27 |

**Beispiel 29**

a) 10-[1-(4-Benzyloxycarbonylphenyl)-2-oxo-piperidin-3-yl]-1,4,7-α,α',α"-tris(cyclohexyl)-1,4,7-tris(benzyloxycarbonylmethyl)-1,4,7,10-tetraazacyclododecan

24,0 g (50 mmol) des in Beispiel 27b als Zwischenprodukt beschriebenen 1-[1-(4-Benzyloxycarbonylphenyl)-2-oxo-piperidin-3-yl]-1,4,7,10-tetraazacyclododecans und 60 mL (0,35 mol) N-Ethyldiisopropylamin in 200 mL Dichlormethan gibt man zu 76,1 g (0,2 mol) 2-(Trifluormethansulfonyloxy)-2-cyclohexylessigsäurebenzylester (Qabar et al., Tetrahedron Letters (1998), 39(33), 5895) in 400 mL Dichlormethan und rührt 6 Stunden unter Rückfluß und anschließend über Nacht bei Raumtemperatur. Es wird dreimal mit je 500 mL Wasser extrahiert, die organische Phase über Magnesiumsulfat getrocknet und zur Trockne eingedampft. Der Rückstand wird an Kieselgel chromatographiert (Laufmittel: Dichlormethan /Methanol: 20/1). Die das Produkt enthaltenden Fraktionen werden vereinigt und eingedampft.
Ausbeute: 40,9 g (70 % d. Th.) eines farblosen kristallinen Pulvers

| Elementaranalyse: | | | |
|---|---|---|---|
| ber.: | C 73,88 | H 7,84 | N 5,98 |
| gef.: | C 74,12 | H 7,69 | N 5,89 |

b) 10-[1-(4-Carboxyphenyl)-2-oxo-piperidin-3-yl]-1,4,7-α,α',α"-tris(cyclohexyl)-1,4,7-tris(carboxymethyl)-1,4,7,10-tetraazacyclododecan

35,1 g (30 mmol) der Titelverbindung aus Beispiel 29a werden in 400 mL Isopropanol gelöst, mit 40 mL Wasser versetzt und 3 g Palladiumkatalysator (10 % Pd/C) hinzugegeben. Man hydriert 8 Stunden bei 50° C. Es wird vom Katalysator abfiltriert, das Filtrat im Vakuum zur Trockne eingedampft.
Ausbeute: 24,3 g (quantitativ) eines farblosen Pulvers

| Elementaranalyse: | | | |
|---|---|---|---|
| ber.: | C 65,24 | H 8,34 | N 8,65 |
| gef.: | C 65,48 | H 8,22 | N 8,60 |

c) Gd-Komplex des 10-[1-(4-Carboxyphenyl)-2-oxo-piperidin-3-yl]-1,4,7-α,α',α"-tris(cyclohexyl)-1,4,7-tris(carboxymethyl)-1,4,7,10-tetraazacyclododecans

16,2 g (20 mmol) des in Beispiel 29b beschriebenen Liganden werden in 150 mL Wasser und 150 mL Isopropanol gelöst und durch Zugabe von 5 mL Essigsäure angesäuert. Es werden 3,6 g (10 mmol) Gadoliniumoxid zugegeben und 8 h am Rückfluß erhitzt. Nach beendeter Komplexierung wird mit Ammoniak erneut auf pH 7,4 eingestellt und an Kieselgel chromatographiert (Laufmittel: Dichlormethan /Methanol /Ammoniak: 20/20/1). Die das Produkt enthaltenden Fraktionen werden vereinigt und zur Trockne eingedampft. Der Rückstand wird mit Ameisensäure aufgenommen und mehrfach unter Zusatz von Dichlormethan zur Trockne eingedampft und anschließend bis zur Gewichtskonstanz im Vakuum getrocknet.
Ausbeute: 13,6 g (68 % d. Th.) eines farblosen Pulvers.
Wassergehalt (Karl-Fischer): 7,5 %

| Elementaranalyse (bezogen auf die wasserfreie Substanz): | | | | |
|---|---|---|---|---|
| ber.: | C 54,81 | H 6,69 | Gd 16,31 | N 7,26 |
| gef.: | C 55,11 | H 6,57 | Gd 16,09 | N 7,24 |

**Beispiele 30-90**

Die Beispiele 30-90 beschreiben ,Konjugate der vorstehend beschriebenen Gadoliniumkomplexe mit Biomolekülen. Die Konjugate wurden nach den folgenden allgemeinen Arbeitsvorschriften I-IV hergestellt. Die Ergebnisse sind in Tabelle 1 zusammengefaßt. Hierin steht "AAV" für allgemeine Arbeitsvorschrift, "ACTH" für adrenocorticotropes Hormon, und "RP-18" bezeichnet eine "reversed phase" stationäre Chromatographiephase. Die Anzahl der Komplexe pro Biomolekül wurde mittels ICP (inductively coupled plasma atomic emission spectroscopy) bestimmt.

**Allgemeine Arbeitsvorschrift (AAV) I: Albumin-Amid-Konjugate**

3 mmol der Gd-komplexsäure werden in 15 mL DMF gelöst, unter Eiskühlung mit 380 mg (3,3 mmol) N-Hydroxysuccinimd und 681 mg Dicyclohexylcarbodiimid versetzt und 1 Stunde im Eis voraktiviert. Die Aktivestermischung wird innerhalb von 30 Minuten in eine Lösung von 16,75 g (0,25 mmol) Rinderserumalbumin (BSA) in 150 mL Phosphatpuffer (pH 7,4) eingetropft und 2 Stunden bei Raumtemperatur gerührt. Die Ansatzlösung wird filtriert, das Filtrat über eine AMICON® YM30 (cut off 30.000 Da) ultrafiltriert, das Retentat über eine Sephadex® G50 -Säule chromatographiert und die Produktfraktionen gefriergetrocknet.

**Allgemeine Arbeitsvorschrift (AAV) II: Albumin-Maleimid-Konjugate**

0,0438 mmol des Gd-komplexmaleimids in 1 mL DMF werden zu 0,84 g (0,0125 mmol) Rinderserumalbumin (BSA), gelöst in 15 mL Phosphatpuffer (pH 7,4), gegeben und eine Stunde bei Raumtemperatur gerührt. Die Ansatzlösung wird filtriert, das Filtrat über eine AMICON® YM30 (cut off 30.000 Da) ultrafiltriert, das Retentat über eine Sephadex® G50 - Säule chromatographiert und die Produktfraktionen gefriergetrocknet.

**Allgemeine Arbeitsvorschrift (AAV) III: Herstellung von Amid-Konjugaten**

3 mmol der Gd-komplexsäure werden in 15 mL DMF gelöst, unter Eiskühlung mit 380 mg (3,3 mmol) N-Hydroxysuccinimd und 681 mg Dicyclohexylcarbodiimid versetzt und 1 Stunde im Eis voraktiviert. Die Aktivestermischung wird in eine Lösung von 2,5 mmol Aminkomponente in 15-150 mL DMF eingetropft und über Nacht bei Raumtemperatur gerührt. Die Ansatzlösung wird filtriert und an Kieselgel chromatographiert.

**Allgemeine Arbeitsvorschrift (AAV) IV: Herstellung von Maleimido-SH-Konjugaten**

3 mmol des Gd-komplexmaleimids in 15 mL DMF werden zu 2,5 mmol SH-Komponente in 15-150 mL DMF eingetropft und eine Stunde bei Raumtemperatur gerührt. Die Ansatzlösung wird an Kieselgel chromatographiert.

**Tabelle 1**

| Beispiel | Edukt Gd-Komplex (Beispiel Nr.) | konjugiert mit | (Herkunft) | AAV | Anzahl Komplexe pro Biomolekül (ICP) | Bemerkungen | Ausbeute (%) |
|---|---|---|---|---|---|---|---|
| 30 | 1 | BSA | Sigma | I | 3,7 | - | quant. |
| 31 | 2 | BSA | Sigma | I | 6,1 | - | quant. |
| 32 | 3 | BSA | Sigma | I | 2,9 | - | quant. |
| 33 | 4 | BSA | Sigma | I | 3,5 | - | quant. |
| 34* | 5 | BSA | Sigma | I | 4,2 | - | quant. |
| 35* | 6 | BSA | Sigma | I | 6,5 | - | quant. |
| 36* | 7 | BSA | Sigma | I | 5,0 | - | quant. |
| 37 | 16 | BSA | Sigma | II | 0,71 | - | quant. |
| 38 | 17 | BSA | Sigma | II | 0,55 | - | quant. |
| 39* | 8 | BSA | Sigma | I | 3,0 | - | quant. |
| 40* | 9 | BSA | Sigma | I | 4,7 | - | quant. |
| 41* | 10 | BSA | Sigma | I | 5,1 | - | quant. |
| 42* | 11 | BSA | Sigma | I | 2,7 | - | quant. |
| 43 | 12 | BSA | Sigma | I | 4,0 | - | quant. |
| 44 | 13 | BSA | Sigma | I | 3,3 | - | quant. |
| 45 | 14 | BSA | Sigma | I | 5,8 | - | quant. |
| 46 | 15 | BSA | Sigma | I | 4,6 | - | quant. |
| 47 | 18 | BSA | Sigma | I | 3,7 | - | quant. |
| 48 | 19 | BSA | Sigma | I | 4,1 | - | quant. |
| 49 | 20 | BSA | Sigma | I | 2,8 | - | quant. |
| 50 | 21 | BSA | Sigma | I | 3,5 | - | quant. |
| 51 | 22 | BSA | Sigma | I | 3,3 | - | quant. |
| 52 | 23 | BSA | Sigma | I | 2,9 | - | quant. |
| 53 | 24 | BSA | Sigma | I | 4,0 | - | quant. |

(fortgesetzt)

| Beispiel | Edukt Gd-Komplex (Beispiel Nr.) | konjugiert mit | (Herkunft) | AAV | Anzahl Komplexe pro Biomolekül (ICP) | Bemerkungen | Ausbeute (%) |
|---|---|---|---|---|---|---|---|
| 54 | 25 | BSA | Sigma | I | 3,5 | - | quant. |
| 55 | 26 | BSA | Sigma | I | 3,0 | - | quant. |
| 56 | 27 | BSA | Sigma | I | 3,9 | - | quant. |
| 57 | 28 | BSA | Sigma | I | 3,1 | - | quant. |
| 58 | 29 | BSA | Sigma | I | 3,4 | - | quant. |
| 59* | 11 | (D-Lys16)-ACTH (1-24 human) | BACHEM | I | 2,0 | - | quant. |
| 60 | 12 | ACTH (1-17) | BACHEM | I | 1,7 | - | quant. |
| 61 | 14 | H-β-Ala-Phe | BACHEM | III | 1,0 | wurde an RP-18 gereinigt | 95 |
| 62* | 8 | Anti-Inflamatory Peptide 2 | BACHEM | I | 1,0 | - | quant. |
| 63* | 9 | L-Carnosin | BACHEM | III | 1,0 | wurde an RP-18 gereinigt | 97 |
| 64 | 16 | Homoglutathion | BACHEM | IV | 1,0 | wurde an RP-18 gereinigt | 94 |
| 65 | 17 | Guanyl-Cys-OH | BACHEM | IV | 1,0 | wurde an RP-18 gereinigt | 93 |
| 66* | 8 | H-DL-d-Hydroxy-DL-Lys-OH | BACHEM | III | 1,0 | wurde an RP-18 gereinigt | 85 |
| 67* | 7 | H-β-Ala-Lys-OH | BACHEM | III | 1,0 | wurde an RP-18 gereinigt | 87 |
| 68 | 16 | H-Arg-Gly-Asp-Cys-OH | BACHEM | III | 1,0 | wurde an RP-18 gereinigt | 91 |
| 69* | 9 | H-Asp-Leu-Trp-Gln-Lys-OH | BACHEM | III | 1,0 | wurde an RP-18 gereinigt | 94 |
| 70 | 12 | H-Ala-His-Lys-OH | BACHEM | III | 2,0 | wurde an RP-18 gereinigt | 91 |
| 71 | 13 | Endothelin-2 (Human) | BACHEM | I | 0,87 | - | quant. |
| 72 | 14 | Human Serumalbumin | BACHEM | I | 5,1 | - | quant. |
| 73* | 7 | Human Serumalbumin | BACHEM | I | 3,1 | - | quant. |
| 74* | 8 | Human Serumalbumin | BACHEM | I | 2,3 | - | quant. |
| 75 | 17 | Thioguanosin | Aldrich | IV | 1,0 | wurde an RP-18 gereinigt | 96 |
| 76* | 5 | 6-Aminopenicilinsäure | Aldrich | III | 1,0 | wurde an RP-18 gereinigt | 92 |

(fortgesetzt)

| Beispiel | Edukt Gd-Komplex (Beispiel Nr.) | konjugiert mit | (Herkunft) | AAV | Anzahl Komplexe pro Biomolekül (ICP) | Bemerkungen | Ausbeute (%) |
|---|---|---|---|---|---|---|---|
| 77* | 11 | 4-Aminopteroylglutaminsäure | Aldrich | III | 1,0 | wurde an RP-18 gereinigt | 65 |
| 78 | 4 | 2-Amino-purinthiol | Aldrich | IV | 1,0 | wurde an RP-18 gereinigt | 94 |
| 79 | 12 | 5-Azacytidin | Aldrich | III | 1,0 | wurde an RP-18 gereinigt | 96 |
| 80 | 17 | 4,5-Diamino-2,6-dimercaptopyrimidin | Aldrich | IV | 1,0 | wurde an RP-18 gereinigt | 71 |
| 81 | 13 | Mitomycin C | Aldrich | III | 1,0 | wurde an RP-18 gereinigt | 81 |
| 82 | 12 | Muraminsäure | Aldrich | III | 1,0 | wurde an RP-18 gereinigt | 92 |
| 83* | 6 | Puromycin | SIGMA | III | 1,0 | wurde an RP-18 gereinigt | 90 |
| 84* | 11 | Doxorubicin | SIGMA | III | 1,0 | wurde an RP-18 gereinigt | 89 |
| 85 | 12 | Spectinomycin | SIGMA | III | 1,0 | wurde an RP-18 gereinigt | 88 |
| 86 | 4 | Streptomycin | SIGMA | III | 1,0 | wurde an RP-18 gereinigt | 62 |
| 87 | 14 | Neomycin B | SIGMA | III | 1,0 | wurde an RP-18 gereinigt | 52 |
| 88* | 8 | Nystatin | SIGMA | III | 1,0 | wurde an RP-18 gereinigt | 72 |
| 89 | 3 | Hygromycin | SIGMA | III | 1,0 | wurde an RP-18 gereinigt | 71 |
| 90 | 2 | Ampicillin | SIGMA | III | 1,0 | wurde an RP-18 gereinigt | 42 |

* Vergleichsbeispiel

**Beispiel 91**

In diesem Beispiel wurden die Relaxivitäten der Konjugate aus den Beispielen 30-38 mit den Relaxivitäten von zwei Vergleichssubstanzen verglichen. Als Vergleichssubstanzen wurden Gd-DTPA (1) mit der Formel:

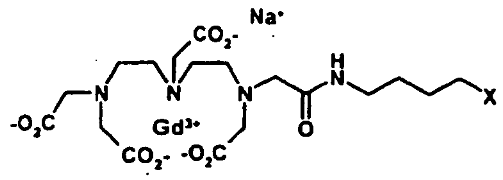

und Gd-GlyMeDOTA (2) mit der Formel:

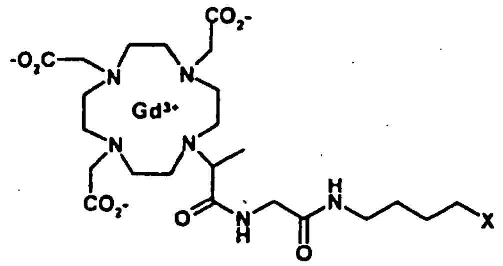

die jeweils mit Rinderserumalbumin (BSA) umgesetzt waren, eingesetzt.

Die Messungen erfolgten jeweils in wäßriger Lösung und in Plasma bei +37°C und einer Frequenz von 20 MHz. Die Ergebnisse sind in der nachfolgenden Tabelle 2 zusammengefaßt, wobei die angegebenen Relaxivitäten pro Mol Gadolinium aus den Meßwerten berechnet wurden:

**Tabelle 2**

| Beispiel | Gd-Komplex (aus Beispiel) | Anzahl Gd/BSA | $R_1$ ($H_2O$) (L/mmol·s) | $R_1$ (Plasma) (L/mmol·s) |
|---|---|---|---|---|
| 30 | 1 | 3,7 | 22,1 | 25,3 |
| 31 | 2 | 6,1 | 29,8 | 35,7 |
| 32 | 3 | 2,9 | 38,2 | 51,5 |
| 33 | 4 | 3,5 | 27,1 | 29,7 |
| 34* | 5 | 4,2 | 20,0 | 22,4 |
| 35* | 6 | 6,5 | 23,2 | 25,8 |
| 36* | 7 | 5,0 | 31,1 | 37,4 |
| 37 | 16 | 0,71 | 38,0 | 38,3 |
| 38 | 17 | 0,55 | 40,6 | 41,4 |
| Vergleichssubstanz 1 | Gd-DTPA | 36 | 13,39 | 13,97 |
| Vergleichssubstanz 2 | Gd-GlyMeDOTA | - | 18,3 | 20,8 |
| * Vergleichsbeispiel | | | | |

Dieses Beispiel zeigt, daß die mit den erfindungsgemäßen Verbindungen hergestellten Konjugate trotz ihrer geringen Anzahl an Gadoliniumatomen pro Biomolekül überraschend eine höhere Relaxivität als die Vergleichssubstanzen aufweisen. Gegenüber Vergleichssubstanz 2 konnte die Relaxivität durch die spezielle Ligandierung des makrocyclischen Rings gesteigert werden.

**Patentansprüche**

**1.** Verbindungen der Formel I

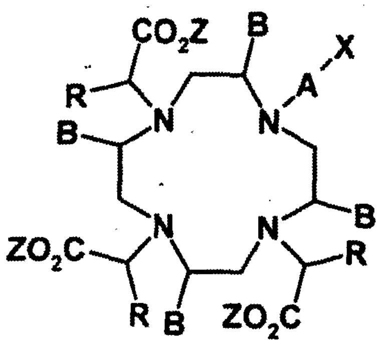

I

worin
Z ein Wasserstoffatom darstellt oder mindestens zwei Z ein Metallionenäquivalent darstellen,
B ein Wasserstoffatom oder einen $C_{1-4}$-Alkylrest darstellt,
R ein Wasserstoffatom oder einen geraden, verzweigten oder cyclischen, gesättigten oder ungesättigten $C_{1-10}$-Alkyl- oder Arylrest darstellt, der gegebenenfalls mit einer Carboxylgruppe, $-SO_3H$ oder $-PO_3H_2$ substituiert ist, und wobei die Alkylkette des $C_{1-10}$-Alkylrestes gegebenenfalls eine Arylgruppe und/oder 1-2 Sauerstoffatome enthält,
mit der Maßgabe, daß die Reste B und R nicht beide gleichzeitig Wasserstoffatome darstellen,
A einen Rest A'-U darstellt, worin A' an das Stickstoffatom des makrocyclischen Rings und U an X gebunden ist, und wobei A'

a) eine Gruppe der Formel

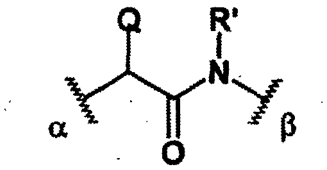

worin Q ein Wasserstoffatom, einen $C_{1-10}$-Alkylrest, der gegebenenfalls mit einer Carboxylgruppe substituiert ist, oder einen Arylrest darstellt, der gegebenenfalls mit einer Carboxylgruppe, einer $C_{1-15}$-Alkoxygruppe, einer Aryloxygruppe oder einem Halogenatom substituiert ist, und R' wie vorstehend R definiert ist, aber unabhängig gewählt werden kann, oder
b) eine Gruppe der Formel

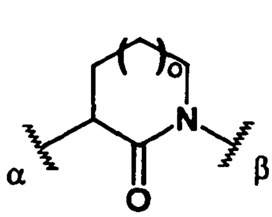

worin o 0 oder 1 ist, und der Ring gegebenenfalls mit einem Benzolring anelliert ist, wobei dieser Benzolring, falls vorhanden, mit einer Methoxy- oder Carboxylgruppe, $-SO_3H$ oder - $PO_3H_2$ substituiert sein kann, darstellt, wobei

in den Gruppen unter a) und b) die an den mit 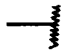 **gekennzeichneten** Positionen an die benachbarten Gruppen gebunden sind und worin die Position α an ein Stickstoffatom des makrocyclischen Rings und die Position β an U

gebunden ist und

U eine gerade oder verzweigte, gesättigte oder ungesättigte $C_{1-30}$-Kohlenwasserstoffkette darstellt, die gegebenenfalls 1-3 Sauerstoffatome, 1-3 Stickstoffatome und/oder 1-3 -NR"-Reste, worin R" wie vorstehend R definiert ist, aber unabhängig gewählt werden kann, enthält und bei der gegebenenfalls 1-3 Kohlenstoffatome als Carbonylgruppen vorliegen, wobei die Kette oder ein Teil der Kette ringförmig angeordnet sein kann mit der Maßgabe, daß A' und U zusammen so ausgestaltet sind, daß X über mindestens 3 Atome mit dem Stickstoffatom, an das A' gebunden ist, verbunden ist; und

X eine Gruppe darstellt, die mit einem Biomolekül eine Reaktion eingehen kann, sowie deren Salze, mit der Maßgabe, daß

a) wenn B ein Wasserstoffatom ist und R ein Methyl- oder Ethylrest ist, der gegebenenfalls mit einer Carboxygruppe substituiert ist, A nicht den Rest -CH($R^4$)-CO-N$R^2$-$U^6$- darstellt, worin $R^2$ für ein Wasserstoffatom, einen Methyl- oder einen Ethylrest, der gegebenenfalls mit 1 Carboxygruppe substituiert ist, $R^4$ für eine geradkettige, verzweigte, gesättigte oder ungesättigte $C_1$-$C_{30}$-Alkylkette, die gegebenenfalls durch 1-10 Sauerstoffatome, 1 Phenylen-, 1 Phenylenoxygruppen unterbrochen und/oder gegebenenfalls durch 1-5 Hydroxy-, 1-3 Carboxy-, 1 Phenylgruppe(n) substituiert ist, und $U^6$ für eine gegebenenfalls 1-5 Imino-, 1-3 Phenylen-, 1-3 Phenylenoxy-, 1-3 Phenylenimino-, 1-5 Amid-, 1-2 Hydrazid-, 1-5 Carbonyl-, 1-5 Ethylenoxy-, 1 Harnstoff-, 1 Thioharnstoff-, 1-2 Carboxyalkylimino-, 1-2 Estergruppen, 1-10 Sauerstoff-, 1-5 Schwefel- und/oder 1-5 Stickstoff-Atom(e) enthaltende und/oder gegebenenfalls durch 1-5 Hydroxy-, 1-2 Mercapto-, 1-5 Oxo-, 1-5 Thioxo-, 1-3 Carboxy-, 1-5 Carboxyalkyl-, 1-5 Ester- und/oder 1-3 Aminogruppe(n) substituierte geradkettige, verzweigte, gesättigte oder ungesättigte $C_1$-$C_{20}$-Alkylengruppe, wobei die gegebenenfalls enthaltenen Phenylengruppen durch 1-2 Carboxy-, 1-2 Sulfon- oder 1-2 Hydroxygruppen substituiert sein können, stehen, und

b) wenn B ein Wasserstoffatom ist und R ein $C_{1-4}$-Alkyrest ist, A nicht den Rest

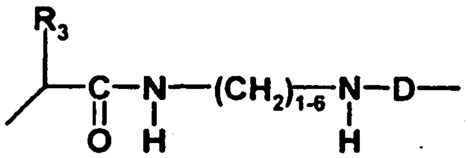

darstellt, worin $R_3$ ein Wasserstoffatom oder ein $C_{1-4}$-Alkylrest ist, D eine gesättigte oder ungesättigte, geradkettige oder verzweigte $C_{1-4}$-Alkylengruppe ist, die gegebenenfalls mit einer Carbonylgruppe unterbrochen oder substituiert sein kann, und D an X gebunden ist.

**2.** Verbindungen nach Anspruch 1, worin R ein Wasserstoffatom, ein geradkettiger oder verzweigter $C_{1-10}$-Alkylrest, ein Cyclohexylrest, $-CH_2-COOH$, $-C(CH_3)_2-COOH$, ein Phenylrest oder ein Rest der Formel $-(CH_2)_m-(O)_n-$(Phenylen)$_p$-Y ist, worin m eine ganze Zahl von 1 bis 5 ist, n 0 oder 1 ist, p 0 oder 1 ist und Y ein Wasserstoffatom, einen Methoxyrest, eine Carboxylgruppe, $-SO_3H$ oder $-PO_3H_2$ darstellt.

**3.** Verbindungen nach Anspruch 2, worin, wenn B ein Wasserstoffatom ist, R ein Isopropylrest, ein Isobutylrest, ein tert.-Butylrest, ein geradkettiger oder verzweigter $C_{5-10}$-Alkylrest, ein Cyclohexylrest, $-CH_2-COOH$, $-C(CH_3)_2-COOH$, ein Phenylrest oder ein Rest der Formel $-(CH_2)_m-(O)_n-$(Phenylen)$_p$-Y ist, worin m eine ganze Zahl von 1 bis 5 ist, n 0 oder 1 ist, p 0 oder 1 ist und Y ein Wasserstoffatom, einen Methoxyrest, eine Carboxylgruppe, $-SO_3H$ oder $-PO_3H_2$ darstellt.

**4.** Verbindungen nach Anspruch 3, worin, wenn B ein Wasserstoffatom ist, R ein Isopropyl-, Cyclohexyl- oder Phenylrest ist.

**5.** Verbindungen nach einem der vorhergehenden Ansprüche, worin für A' die Gruppe der Formel

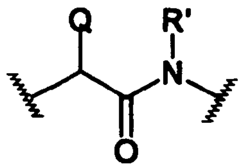

ausgewählt ist aus -$C(CH_3)H$-CO-NH-, -C(Phenyl)H-CO-NH- und -C(p-Dodecanoxyphenyl)H-CO-NH-.

6. Verbindungen nach einem der vorhergehenden Ansprüche, worin für A' die Gruppe der Formel

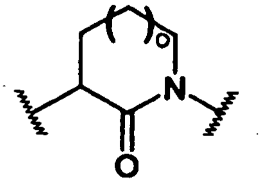

ausgewählt ist aus:

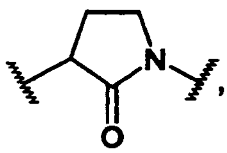, 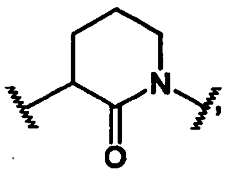, 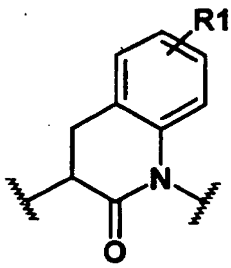 und 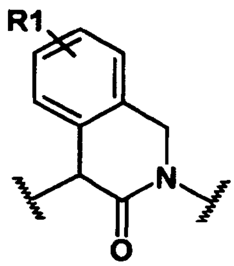

wobei $R^1$ -$OCH_3$, -$CO_2H$, -$SO_3H$ oder -$PO_3H_2$ ist.

7. Verbindungen nach einem der vorhergehenden Ansprüche, worin U ausgewählt ist aus -$CH_2$-, -$(CH_2)_5$-, -$(CH_2)_{10}$-, -Phenylen-O-$CH_2$-, -Phenylen-O-$(CH_2)_3$-, -Phenylen-O-$(CH_2)_{10}$-, -$CH_2$-Phenylen-, -Cyclohexylen-O-$CH_2$-, -Phenylen-, -C(Phenyl)H-, -$CH_2$-Pyridylen-O-$CH_2$-, -$CH_2$-Pyridylen- und -$CH_2$-CO-NH-$CH_2$-$CH_2$-.

8. Verbindungen nach einem der vorhergehenden Ansprüche, worin X ausgewählt ist aus der Gruppe bestehend aus Carboxyl, aktiviertes Carboxyl, Amino, Isocyanat, Isothiocyanat, Hydrazin, Semicarbazid, Thiosemicarbazid, Chloracetamid, Bromacetamid, Iodacetamid, Acylamino, gemischten Anhydriden, Azid, Hydroxid, Sulfonylchlorid, Carbodiimid und Resten der Formeln

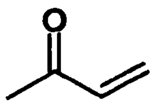, 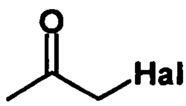 oder 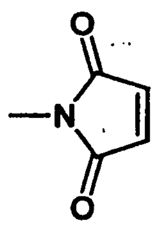

worin Hal ein Halogenatom ist.

9. Verbindungen nach Anspruch 8, worin die aktivierte Carboxylgruppe ausgewählt ist aus

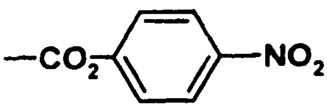, 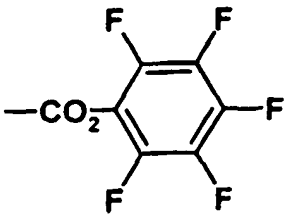, 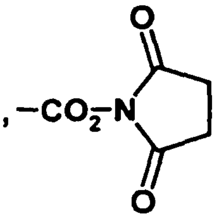, 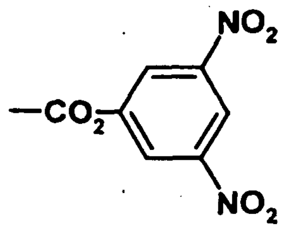

und

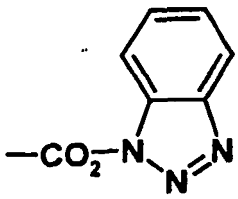

**10.** 10-(4-Carboxy-1-methyl-2-oxo-3-azabutyl)-1,4,7-α,α',α"-trimethyl-1,4,7-tris(carboxymethyl)-1,4,7,10-tetraazacyclododecan, 10-(4-Carboxy-1-methyl-2-oxo-3-azabutyl)-1,4,7-α,α',α"-tris(isopropyl)-1,4,7-tris(carboxymethyl)-1,4,7,10-tetraazacyclododecan, 10-(4-Carboxy-1-methyl-2-oxo-3-azabutyl)-1,4,7-α,α',α"-tris(cyclohexyl)-1,4,7-tris(carboxymethyl)-1,4,7,10-tetraazacyclododecan, 10-(4-(*t*-Butoxycarbonyl-1-phenyl-2-oxo-3-azabutyl)-1,4,7-α,α',α"-trimethyl-1,4,7-tris(carboxymethyl)-1,4,7,10-tetraazacyclododecan, 10-(4-(*t*-Butoxycarbonyl-1-phenyl-2-oxo-3-azabutyl)-1,4,7-α,α',α"-triphenyl-1,4,7-tris(carboxymethyl)-1,4,7,1 0-tetraazacyclododecan, 10-(4-Carboxy-2-oxo-3-azabutyl)-1,4,7-α,α',α"-tris(isopropyl)-1,4,7-tris(carboxymethyl)-1,4,7,10-tetraazacyclododecan, 10-(4-Carboxy-2-oxo-3-azabutyl)-1,4,7-α,α',α"-tris(cyclohexyl)-1,4,7-tris(carboxymethyl)-1,4,7,10-tetraazacyclododecan, 10-(4-Carboxy-1-methyl-2-oxo-3-azabutyl)-2,5,8,11-tetramethyl-1,4,7,10-tetraazacyclododecan-1,4,7-triessigsäure-tri-tert-butylester, 10-[8-(N-Maleimido)-1-methyl-2,5-dioxo-3,6-diazaoctyl]-1,4,7-α,α',α"-tris-(isopropyl)-1,4,7-tris(carboxymethyl)-1,4,7,10-tetraazacyclododecan und 10-[8-(N-Maleimido)-1-methyl-2,5-dioxo-3,6-diazaoctyl]-1,4,7-α,α',α"-tris-(cyclohexyl)-1,4,7-tris(carboxymethyl)-1,4,7,10-tetraazacyclododecan.

**11.** Verbindungen nach einem der vorhergehenden Ansprüche, worin mindestens zwei der Reste Z für ein Metallionenäquivalent eines radioaktiven oder paramagnetischen Elements der Ordnungszahlen 21-29, 31, 32, 37-39, 42-44, 46, 47, 49, 58-71, 75, 77, 82 oder 83 stehen.

**12.** Verwendung von Verbindungen der Formel I

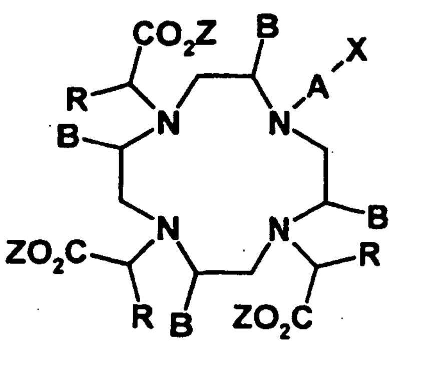

I

,

worin Z, B, R, A und X wie in Anspruch 1 definiert sind, mit der Maßgabe, daß B und R nicht gleichzeitig Wasserstoffatome darstellen und wenn B ein Wasserstoffatom ist und R ein $C_{1-4}$ -Alkyrest ist, A nicht den Rest

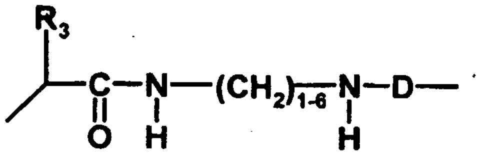

darstellt, worin $R_3$ ein Wasserstoffatom oder ein $C_{1-4}$-Alkylrest ist, D eine gesättigte oder ungesättigte, geradkettige oder verzweigte $C_{1-4}$-Alkylengruppe ist, die gegebenenfalls mit einer Carbonylgruppe unterbrochen oder substituiert sein kann, und D an X gebunden ist, zur Herstellung eines Konjugats mit einem Biomolekül.

**13.** Verwendung nach Anspruch 12, worin das Biomolekül ausgewählt ist aus der Gruppe bestehend aus Biopolymeren, Proteinen, synthetisch modifizierten Biopolymeren, Kohlenhydraten, Antikörpern, DNA und RNA Fragmenten, β-Aminosäuren, Vektoraminen zur Einschleusung in die Zelle, biogenen Aminen, Pharmazeutika, onkologischen Präparaten, synthetischen Polymeren, die auf ein biologisches Target gerichtet sind, Steroiden, Prostaglandinen, Taxol und dessen Derivaten, Endothelinen, Alkaloiden, Folsäure und dessen Derivaten, bioaktiven Lipiden, Fetten, Fettsäureestern, synthetisch modifizierten Mono-, Di- und Triglyceriden, Liposomen, die an der Oberfläche derivatisiert sind, Micellen aus natürlichen Fettsäuren oder aus Perfluoralkyl-Verbindungen, Porphyrinen, Texaphrinen, erweiterten Porphyrinen, Cytochromen, Inhibitoren, Neuramidasen, Neuropeptiden, Immunomodulatoren, Endoglycosidasen, Substraten, die durch die Enzyme Calmodulin Kinase, Casein-Kinase II, Gluthathion-S-Transferase, Heparinase, Matrix Methalloprotheasen, β-Insulin-Receptor-Kinase, UDP-Galactose 4-Epimerase, Fucosidasen, G-Proteine, Galactosidasen, Glycosidasen, Glycosyltransferasen und Xylosidase attackiert werden, Antibiotika, Vitaminen und Vitamin-Analoga, Hormonen, DNA-Interkalatoren, Nucleosiden, Nucleotiden, Lektinen, Vitamin B12, Lewis-X und Verwandten, Psoralenen, Dientrienantibiotika, Carbacyclinen, VEGF, Somatostatin und dessen Derivaten, Biotin-Derivaten, Antihormonen, tumorspezifischen Proteinen und Synthetika, Polymeren, die sich in sauren oder basischen Bereichen des Körpers anreichern, Myoglobinen, Apomyoglobinen, Neurotransmitterpeptiden, Tumornekrose-Faktoren, Peptiden, die sich in entzündeten Geweben anreichern, Bloodpool-Reagentien, Anionen und Kationen-Transporter Proteinen, Polyestern, Polyamiden und Polyphosphaten.

**14.** Verfahren zur Herstellung von Verbindungen der Formel I

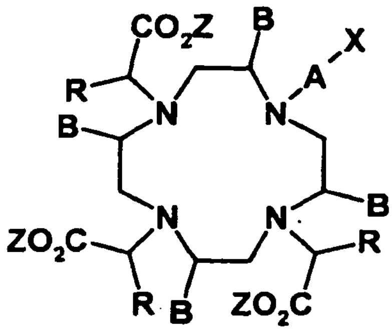

I

worin Z, B, R, A und X wie in Anspruch 1 definiert sind, mit der Maßgabe, daß B und R nicht gleichzeitig Wasserstoffatome darstellen und wenn B ein Wasserstoffatom ist und R ein $C_{1-4}$-Alkyrest ist, A nicht den Rest

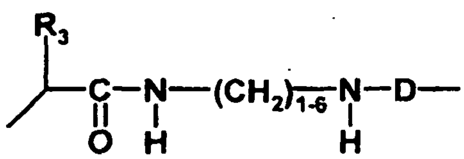

darstellt, worin $R_3$ ein Wasserstoffatom oder ein $C_{1-4}$-Alkylrest ist, D eine gesättigte oder ungesättigte, geradkettige oder verzweigte $C_{1-4}$-Alkylengruppe ist, die gegebenenfalls mit einer Carbonylgruppe unterbrochen oder substituiert

sein kann, und D an X gebunden ist, worin eine Verbindung der Formel II

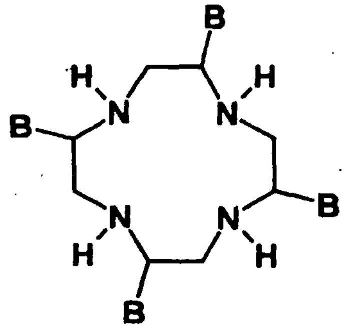

II

worin B wie in Anspruch 1 definiert ist gegebenenfalls nach Einführung von Schutzgruppen für die Stickstoffatome mit Nu-A-X' und Nu-CH(R)-$CO_2$Z' umgesetzt wird, wobei A und R wie in Anspruch 1 definiert sind und Nu ein Nucleofug ist, X' für X oder eine geschützte Form von X steht und X wie in Anspruch 1 definiert ist und Z' für ein Wasserstoffatom, ein Metallionenäquivalent oder eine Schutzgruppe für Carboxyl steht, anschließend die gegebenenfalls vorhandenen Schutzgruppen entfernt werden und wenn gewünscht in an sich bekannter Weise mit mindestens einem Metalloxid oder Metallsalz eines gewünschten Elements umgesetzt wird und gegebenenfalls anschließend in den so erhaltenen Komplexen noch vorhandene acide Wasserstoffatome ganz oder teilweise durch Kationen von anorganischen und/oder organischen Basen, Aminosäuren oder Aminosäureamiden substituiert werden.

## Claims

1. Compounds of formula I

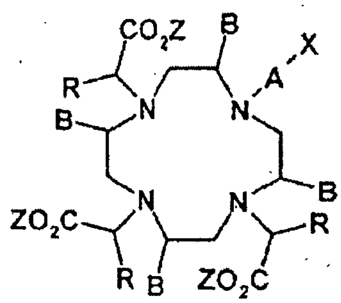

I

wherein
Z represents a hydrogen atom or at least two Zs represent a metal ion equivalent,
B represents a hydrogen atom or a $C_{1-4}$-alkyl radical,
R represents a hydrogen atom or a straight, branched or cyclic, saturated or unsaturated $C_{1-10}$-alyl or aryl radical, which optionally is substituted with a carboxyl group, -$SO_3$H or -$PO_3H_2$, and wherein the alkyl chain of the $C_{1-10}$-alkyl radical optionally contains an aryl group and/or 1-2 oxygen atoms, provided that radicals B and R do not both represent hydrogen atoms simultaneously,
A represents a radical A'-U, wherein A' is bonded to the nitrogen atom of the macrocyclic ring and U is bonded to X, and wherein A' represents

a) a group of formula

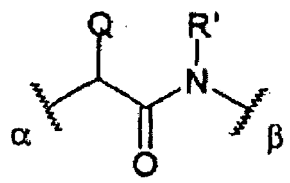

wherein Q represents a hydrogen atom, a $C_{1-10}$-alkyl radical, which optionally is substituted with a carboxyl group, or an aryl radical, which is optionally substituted with a carboxyl group, a $C_{1-15}$-alkoxy group, an aryloxy group or a halogen atom, and R' is defined as R above, but can be selected independently, or

b) a group of formula

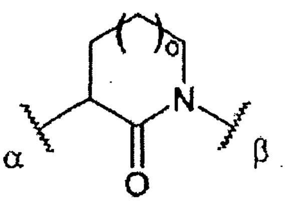

wherein o is 0 or 1, and the ring optionally is annellated with a benzene ring, wherein this benzene ring, if present, can be substituted with a methoxy or carboxyl group, $-SO_3H$ or $-PO_3H_2$, wherein in the groups under a) and b), the positions that are marked 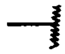 are bonded to the adjacent groups, and wherein position $\alpha$ is bonded to U, and U represents a straight or branched, saturated or unsaturated $C_{1-30}$-hydrocarbon chain that optionally contains 1-3 oxygen atoms, 1-3 nitrogen atoms and/or 1-3 -NR" radicals, wherein R" is defined as R above but can be selected independently, and wherein optionally 1-3 carbon atoms are present as carbonyl groups, wherein the chain or part of the chain can be arranged concentrically, provided that A' and U together are configured in such a way that X is bonded via at least 3 atoms with the nitrogen atom to which A' is bonded; and

X represents a group that can react with a biomolecule, as well as salts thereof, provided that

a) if B is a hydrogen atom and R is a methyl or ethyl radical, which optionally is substituted with a carboxy group, A does not represent the radical $-CH(R^4)-CO-NR^2-U^6-$, wherein $R^2$ stands for a hydrogen atom, a methyl or an ethyl radical, which optionally is substituted with 1 carboxy group, $R^4$ stands for a straight-chain, branched, saturated or unsaturated $C_1$-$C_{30}$-alkyl chain, which optionally is interrupted by 1-10 oxygen atoms, 1 phenylene group, or 1 phenylenoxy group, and/or optionally is substituted by 1-5 hydroxy groups, 1-3 carboxy groups or 1 phenyl group, and $U^6$ stands for a straight-chain, branched, saturated or unsaturated $C_1$-$C_{20}$-alkylene group that optionally contains 1-5 imino groups, 1-3 phenylene groups, 1-3 phenylenoxy groups, 1-3 phenylenimino groups, 1-5 amide groups, 1-2 hydrazide groups, 1-5 carbonyl groups, 1-5 ethylenoxy groups, 1 urea group, 1 thiourea group, 1-2 carboxyalkylimino groups, 1-2 ester groups, 1-10 oxygen atoms, 1-5 sulfur atoms and/or 1-5 nitrogen atoms and/or is optionally substituted by 1-5 hydroxy groups, 1-2 mercapto groups, 1-5 oxo groups, 1-5 thioxo groups, 1-3 carboxy groups, 1-5 carboxyalkyl groups, 1-5 ester groups and/or 1-3 amino groups, wherein the optionally contained phenylene groups can be substituted by 1-2 carboxy groups, 1-2 sulfone groups or 1-2 hydroxy groups, and

b) if B is a hydrogen atom and R is a $C_{1-4}$-alkyl radical, A does not represent the radical

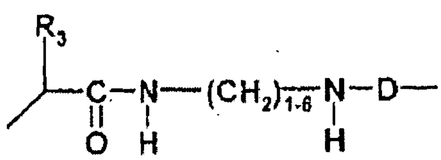

wherein $R_3$ is a hydrogen atom or a $C_{1-4}$-alkyl radical, D is a saturated or unsaturated, straight-chain or branched $C_{1-4}$-alkylene group, which optionally can be interrupted by or substituted with a carbonyl group, and D is bonded to X.

**2.** Compounds according to claim 1, wherein R is a hydrogen atom, a straight-chain or branched $C_{1-10}$-alkyl radical, a cyclohexyl radical, $-CH_2-COOH$, $-C(CH_3)_2-COOH$, a phenyl radical or a radical of formula $-(CH_2)_m-(O)_n-$(phenylene)$_p$-Y, wherein m is an integer from 1 to 5, n is 0 or 1, p is 0 or 1, and Y represents a hydrogen atom, a methoxy radical, a carboxyl group, $-SO_3H$ or $-PO_3H_2$.

**3.** Compounds according to claim 2, wherein, if B is a hydrogen atom, R is an isopropyl radical, an isobutyl radical, a tert-butyl radical, a straight-chain or branched $C_{5-10}$-alkyl radical, a cyclohexyl radical, $-CH_2-COOH$, $-C(CH_3)_2-COOH$, a phenyl radical or a radical of formula $-(CH_2)_m-(O)_n-$(phenylene)$_p$-Y, wherein m is an integer from 1 to 5, n is 0 or 1, p is 0 or 1, and Y represents a hydrogen atom, a methoxy radical, a carboxyl group, $-SO_3H$ or $-PO_3H_2$.

**4.** Compounds according to claim 3, wherein, if B is a hydrogen atom, R is an isopropyl, cyclohexyl or phenyl radical.

**5.** Compounds according to any one of the preceding claims, wherein for A' the group of formula

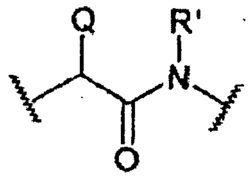

is selected from $-C(CH_3)H-CO-NH-$, -C(phenyl)H-CO-NH- and -C(p-dodecanoxyphenyl)H-CO-NH-.

**6.** Compounds according to any one of the preceding claims, wherein for A' the group of formula

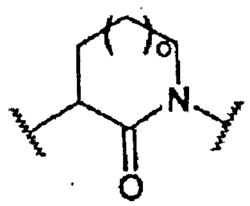

is selected from:

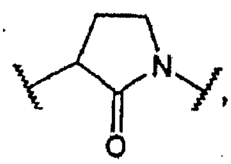, 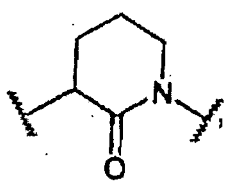, 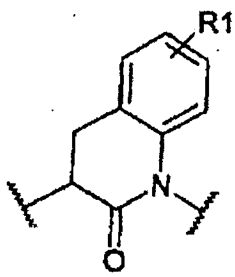 and 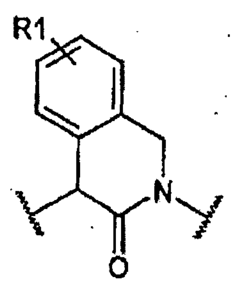

wherein $R^1$ is $-OCH_3$, $-CO_2-H$, $-SO_3H$ or $-PO_3H_2$.

**7.** Compounds according to any one of the preceding claims, wherein U is selected from $-CH_2-$, $-(CH_2)_5-$, $-(CH_2)_{10}-$, -phenylene-O-$CH_2$-, -Phenylene-O-$(CH_2)_3$, -phenylene-O-$(CH_2)_{10}$-, -$CH_2$-phenylene-, -cyclohexylene-O-$CH_2$-, -phenylene-, -C(phenyl)H-, -$CH_2$-pyridylene-O-$CH_2$-, -$CH_2$-pyridylene- and -$CH_2$-CO-NH-$CH_2$-$CH_2$-.

**8.** Compounds according to any one of the preceding claims, wherein X is selected from the group consisting of carboxyl, activated carboxyl, amino, isocyanate, isothiocyanate, hydrazine, semicarbazide, thiosemicarbazide, chloroacetamide, bromoacetamide, iodoacetamide, acylamino, mixed anhydrides, azide, hydroxide, sulfonyl chloride, carbodiimide and radicals of formulas

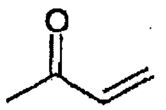,
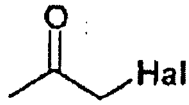
or
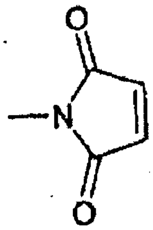

wherein Hal is a halogen atom.

**9.** Compounds according to claim 8, wherein the activated carboxyl group is selected from

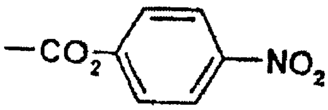,
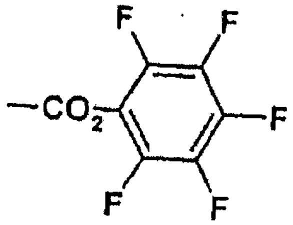,
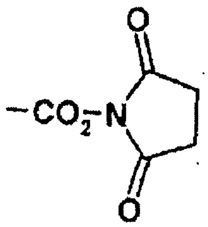,
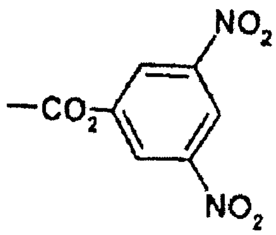

and

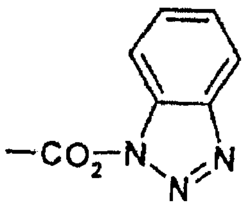.

**10.** 10-(4-Carboxy-1-methyl-2-oxo-3-azabutyl)-1,4,7-α,α',α"-trimethyl-1,4,7-tris(carboxymethyl)-1,4,7,10-tetraazacyclododecane, 10-(4-carboxy-1-methyl-2-oxo-3-azabutyl)-1,4,7-α,α',α"-tris(isopropyl)-1,4,7-tris(carboxymethyl)-1,4,7,10-tetraazacyclododecane, 10-(4-carboxy-1-methyl-2-oxo-3-azabutyl)-1,4,7-α,α',α"-tris(cyclohexyl)-1,4,7-tris(carboxymethyl)-1,4,7,10-tetraazacyclododecane, 10-(4-(*t*-butoxycarbony)-1-phenyl-2-oxo-3-azabutyl)-1,4,7-α, α',α"-trimethyl-1,4,7-tris(carboxymethyl)-1,4,7,10-tetraazacyclododecane, 10-(4-(*t*-butoxycarbonyl-1-phenyl-2-oxo-3-azabutyl)-1,4,7-α,α',α"-triphenyl-1,4,7-tris(carboxymethyl)-1,4,7,10-tetraazacyclododecane, 10-(4-carboxy-2-oxo-3-azabutyl)-1,4,7-α,α',α"-tris(isopropyl)-1,4,7-tris(carboxymethyl)-1,4,7,10-tetraazacyclododecane, 10-(4-carboxy-2-oxo-3-azabutyl)-1,4,7-α, α', α"-tris (cyclohexyl)-1,4,7-tris (carboxymethyl)-1,4,7,10-tetraazacyclododecane, 10-(4-carboxy-1-methyl-2-oxo-3-azabutyl)-2,5,8,11-tetramethyl-1,4,7,10-tetraazacyclododecane-1,4,7-triacetic acid-tri-tert-butyl ester, 10-[8-(N-maleimido)-1-methyl-2,5-dioxo-3,6-diazaoctyl]-1,4,7-α,α',α"-tris-(isopropyl)-1,4,7-tris(carboxymethyl)-1,4,7,10-tetraazacyclododecane and 10-[8-(N-maleimido)-1-methyl-2,5-dioxo-3,6-diazaoctyl]-1,4,7-α,α',α"-tris-(cyclohexyl)-1,4,7-tris(carboxymethyl)-1,4,7,10-tetraazacyclododecane.

**11.** Compounds according to any one of the preceding claims, wherein at least two of radicals Z stand for a metal ion equivalent of a radioactive or paramagnetic element of atomic numbers 21-29, 31, 32, 37-39, 42-44, 46, 47, 49, 58-71, 75, 77, 82 or 83.

**12.** Use of the compounds of formula I

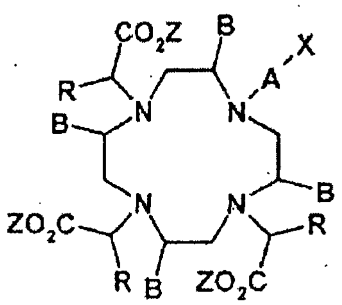

I

wherein Z, B, R, A and X are defined as in claim 1, provided that B and R do not represent hydrogen atoms simultaneously and, if B is a hydrogen atom and R is a $C_{1-4}$-alkyl radical, A does not represent the radical

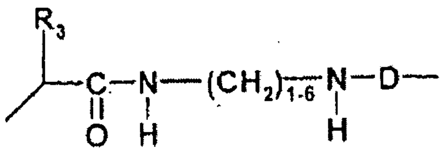

wherein $R_3$ is a hydrogen atom or a $C_{1-4}$-alkyl radical, D is a saturated or unsaturated, straight-chain or branched $C_{1-4}$-alkylene group, which optionally is interrupted by or substituted with a carbonyl group, and D is bonded to X, for the production of a conjugate with a biomolecule.

**13.** Use according to claim 12, wherein the biomolecule is selected from the group consisting of biopolymers, proteins, synthetically modified biopolymers, carbohydrates, antibodies, DNA and RNA fragments, β-amino acids, vector amines for transfer into the cell, biogenic amines, pharmaceutical agents, oncological preparations, synthetic polymers, which are directed to a biological target, steroids, prostaglandins, taxol and derivatives thereof, endothelins, alkaloids, folic acid and derivatives thereof, bioactive lipids, fats, fatty acid esters, synthetically modified mono-, di- and triglycerides, liposomes, which are derivatized on the surface, micelles of natural fatty acids or perfluoroalkyl compounds, porphyrins, texaphrines, expanded porphyrins, cytochromes, inhibitors, neuramidases, neuropeptides, immunomodulators, endoglycosidases, substrates that are attacked by the enzymes calmodulin kinase, casein-kinase II, glutathione-S-transferase, heparinase, matrix-metalloproteases, β-insulin-receptor-kinase, UDP-galactose 4-epimerase, fucosidases, G-proteins, galactosidases, glycosidases, glycosyltransferases and xylosidase, antiobiotics, vitamins and vitamin analogs, hormones, DNA intercalators, nucleosides, nucleotides, lectins, vitamin B12, Lewis-X and related substances, psoralens, dienetriene antibiotics, carbacyclins, VEGF, somatostatin and derivatives thereof, biotin derivatives, antihormones, tumor-specific proteins and synthetic agents, polymers that accumulate in acidic or basic areas of the body, myoglobins, apomyoglobins, neurotransmitter peptides, tumor necrosis factors, peptides that accumulate in inflamed tissues, blood-pool reagents, anion and cation-transporter proteins, polyesters, polyamides and polyphosphates.

**14.** Process for the production of compounds of formula I

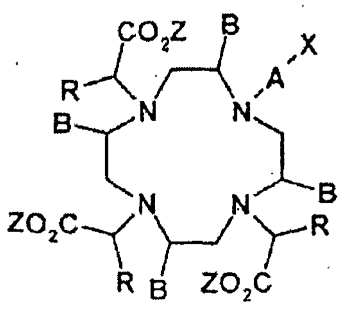

I

wherein Z, B, R, A and X are defined as in claim 1, provided that B and R do not represent hydrogen atoms simultaneously and if B is a hydrogen atom and R is a $C_{1-4}$-alkyl radical, A does not represent the radical

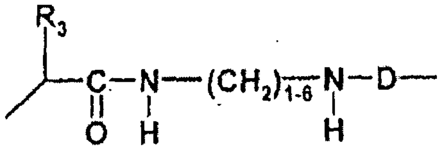

wherein $R_3$ is a hydrogen atom or a $C_{1-4}$-alkyl radical, D is a saturated or unsaturated, straight-chain or branched $C_{1-4}$-alkylene group, which optionally is interrupted by or substituted with a carbonyl group, and D is bonded to X, wherein a compound of formula II

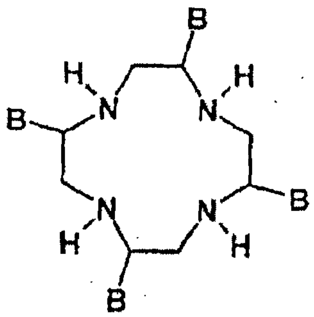

II

wherein B is defined as in claim 1, is reacted with Nu-A-X' and Nu-CH(R)-$CO_2$-Z' optionally after protective groups for the nitrogen atoms are introduced, wherein A and R are defined as in claim 1, and Nu is a nucleofuge, X' stands for X or a protected form of X, and X is defined as in claim 1, Z' stands for a hydrogen atom, a metal ion equivalent or a protective group for carboxyl, then the optionally present protective groups are removed, and, if desired, reacted in a way that is known in the art with at least one metal oxide or metal salt of a desired element and optionally then still present acid hydrogen atoms are completely or partially substituted in the thus obtained complexes by cations of inorganic and/or organic bases, amino acids or amino acid amides.

## Revendications

**1.** Composés de formule I :

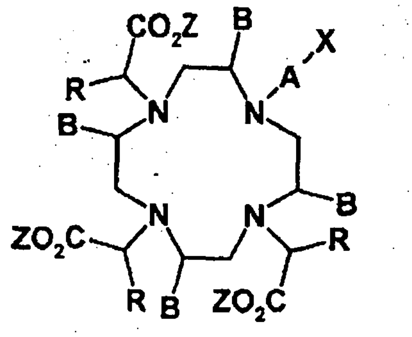

I

dans laquelle :

Z représente un atome d'hydrogène ou au moins deux éléments Z représentent un équivalent d'ion métallique,
B représente un atome d'hydrogène ou un radical alkyle en $C_1$-$C_4$,
R représente un atome d'hydrogène ou un radical alkyle ou aryle en $C_1$-$C_{10}$ linéaire, ramifié ou cyclique, saturé ou insaturé, qui est éventuellement substitué par un groupement carboxyle, $-SO_3H$ ou $-PO_3H_2$ et dans lequel la chaîne alkyle du radical alkyle en $C_1$-$C_{10}$ contient éventuellement un groupement aryle et/ou 1 à 2 atomes d'oxygène, à condition que les radicaux B et R ne représentent pas tous deux simultanément des atomes d'hydrogène,
A représente un radical A'-U, dans lequel A' est lié à l'atome d'azote du cycle macrocyclique et U est lié à X, et dans lequel A' représente

a) un groupement de formule

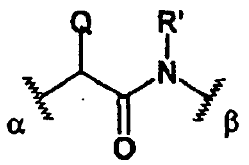

dans laquelle Q représente un atome d'hydrogène, un radical alkyle en $C_1$-$C_{10}$, qui est éventuellement substitué par un groupement carboxyle, ou un radical aryle, qui est éventuellement substitué par un groupement carboxyle, un groupement alcoxy en $C_1$-$C_{15}$, un groupement aryloxy ou un atome d'halogène, et R' est défini comme R précédemment, mais peut être choisi indépendamment, ou
b) un groupement de formule

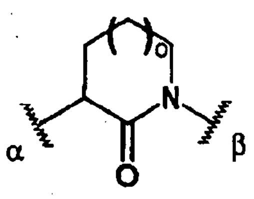

dans laquelle O est égal à 0 ou 1 et le cycle est éventuellement condensé avec un cycle benzène, ce cycle benzène pouvant être substitué, le cas échéant, par un groupement méthoxy ou carboxyle, $-SO_3H$ ou $-PO_3H_2$, et dans

laquelle, dans les groupements cités en a) et b), ceux qui se trouvent dans les positions **caractérisées par** sont liés aux groupements adjacents et la position α est liée à un atome d'azote du cycle macrocyclique et la position β est liée à U, et
U représente une chaîne hydrocarbonée en $C_1$-$C_{30}$ linéaire ou ramifiée, saturée ou insaturée, qui contient éventuellement 1 à 3 atomes d'oxygène, 1 à 3 atomes d'azote et/ou 1 à 3 radicaux -NR", où R" est défini comme R

précédemment, mais peut être choisi indépendamment, et dans laquelle éventuellement 1 à 3 atomes de carbone sont présents sous la forme de groupements carbonyle, laquelle chaîne ou partie de la chaîne peut se présenter sous forme cyclique, à condition que A' et U soient aménagés conjointement de sorte que X soit lié par au moins 3 atomes à l'atome d'azote, auquel est lié A' ; et
X représente un groupement qui peut réagir avec une biomolécule, ainsi qu'avec ses sels, à condition que

a) si B est un atome d'hydrogène et R un radical méthyle ou éthyle, qui est éventuellement substitué par un groupement carboxy, A ne représente pas le radical -CH($R^4$)-CO-N$R^2$-$U^6$-, dans lequel $R^2$ représenté un atome d'hydrogène, un radical méthyle ou un radical éthyle, qui est éventuellement substitué par 1 groupement carboxy, $R^4$ représente une chaîne alkyle en $C_{1-}C_{30}$ linéaire, ramifiée, saturée ou insaturée, qui est éventuellement interrompue par 1 à 10 atomes d'oxygène, 1 groupement phénylène, 1 groupement phénylèneoxy et/où qui est éventuellement substituée par 1 à 5 groupements hydroxy, 1 à 3 groupements carboxy, 1 groupement phényle, et $U^6$ représente un groupement alkylène en $C_1$-$C_{20}$ linéaire, ramifié, saturé ou insaturé, contenant éventuellement 1 à 5 groupements imino, 1 à 3 groupements phénylène, 1 à 3 groupements phénylèneoxy, 1 à 3 groupements phénylèneimino, 1 à 5 groupements amide, 1 à 2 groupements hydrazide, 1 à 5 groupements carbonyle, 1 à 5 groupements éthylèneoxy, 1 groupement urée, 1 groupement thiourée, 1 à 2 groupements carboxyalkylimino, 1 à 2 groupements ester, 1 à 10 atomes d'oxygène, 1 à 5 atomes de soufre et/ou 1 à 5 atomes d'azote et/ou substitué éventuellement par 1 à 5 groupements hydroxy, 1 à 2 groupements mercapto, 1 à 5 groupements oxo, 1 à 5 groupements thioxo, 1 à 3 groupements carboxy, 1 à 5 groupements carboxyalkyle, 1 à 5 groupements ester et/ou 1 à 3 groupements amino, les groupements phénylène éventuellement contenus pouvant être substitués par 1 à 2 groupements carboxy, 1 à 2 groupements sulfone ou 1 à 2 groupements hydroxy, et
b) si B est un atome d'hydrogène et R un radical alkyle en $C_1$-$C_4$, A ne représente pas le radical

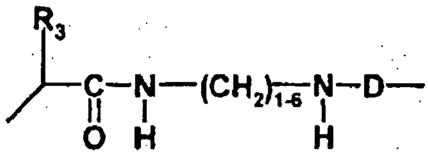

dans lequel $R_3$ est un atome d'hydrogène ou un radical alkyle en $C_{1-}C_4$, D est un groupement alkylène en $C_{1-}C_4$ saturé ou insaturé, linéaire ou ramifié, qui peut éventuellement être interrompu ou substitué par un groupement carbonyle, et D est lié à X.

2. Composés selon la revendication 1, dans lesquels R est un atome d'hydrogène, un radical alkyle en $C_{1-C10}$ linéaire ou ramifié, un radical cyclohexyle, -$CH_2$-COOH, -C($CH_3$)$_2$-COOH, un radical phényle ou un radical de formule -($CH_2$)$_m$-(O)$_n$-(phénylène)$_p$-Y, dans laquelle m est un nombre entier de 1 à 5, n est égal à 0 ou 1, p est égal à 0 ou 1 et Y représente un atome d'hydrogène, un radical méthoxy, un groupement carboxyle, -$SO_3$H ou -$PO_3H_2$.

3. Composés selon la revendication 2, dans lesquels, si B est un atome d'hydrogène, R représente un radical isopropyle, un radical isobutyle, un radical tert-butyle, un radical alkyle en $C_{5-}C_{10}$ linéaire ou ramifié, un radical cyclohexyle, -$CH_2$-COOH, -C($CH_3$)$_2$-COOH, un radical phényle ou un radical de formule -($CH_2$)$_m$-(O)$_n$-(phénylène)$_p$-Y, dans laquelle m est un nombre entier de 1 à 5, n est égal à 0 ou 1, p est égal à 0 ou 1 et Y représente un atome d'hydrogène, un radical méthoxy, un groupement carboxyle, -$SO_3$H ou -$PO_3H_2$.

4. Composés selon la revendication 3, dans lesquels, si B est un atome d'hydrogène, R est un radical isopropyle, cyclohexyle ou phényle.

5. Composés selon l'une quelconque des revendications précédentes, dans lesquels on choisit pour A' le groupement de formule :

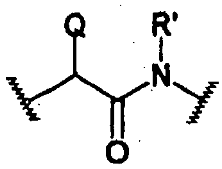

parmi les composés suivants : $-C(CH_3)H-CO-NH-$, -C(phényl)H-CO-NH- et -C(p-dodécanoxyphényl)H-CO-NH-.

**6.** Composés selon l'une quelconque des revendications précédentes, dans lesquels on choisit pour A' le groupement de formule :

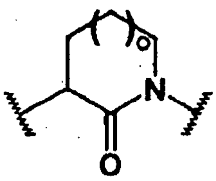

parmi les composés suivants :

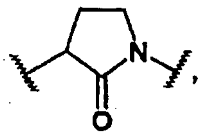, 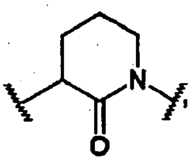, 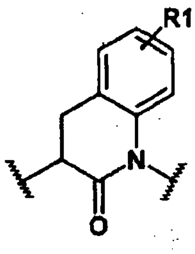 und 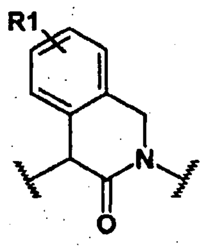

$R^1$ représentant $-OCH_3$, $-CO_2H$, $-SO_3H$ ou $-PO_3H_2$.

**7.** Composés selon l'une quelconque des revendications précédentes, dans lesquels U est choisi parmi $-CH_2-$, $-(CH_2)_5-$, $-(CH_2)_{10}-$, -phénylène-O-$CH_2$-, -phénylène-O-$(CH_2)_3$-, -phénylène-O-$(CH_2)_{10}$-, -$CH_2$-phénylène-, -cyclohexylène-O-$CH_2$-, -phénylène-, -C(phényl)H-, -$CH_2$-pyridylène-O-$CH_2$-, -$CH_2$-pyridylène- et $-CH_2-CO-NH-CH_2-CH_2-$.

**8.** Composés selon l'une quelconque des revendications précédentes, dans lesquels X est choisi dans le groupe constitué des groupements suivants : carboxyle, carboxyle activé, amino, isocyanate, isothiocyanate, hydrazine, semicarbazide, thiosemicarbazide, chloro-acétamide, bromo-acétamide, iodo-acétamide, acylamino, anhydrides mixtes, azide, hydroxyde, chlorure de sulfonyle, carbodiimide et des radicaux de formules suivantes :

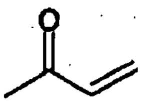, 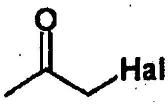 oder 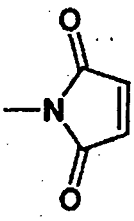

dans lesquelles Hal est un atome d'halogène.

**9.** Composés selon la revendication 8, dans lesquels le groupement carboxyle activé est choisi parmi les groupements suivants :

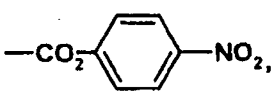, 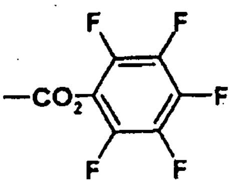, 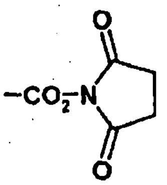, 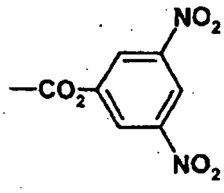

et

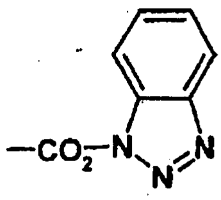

**10.** 10-(4-Carboxy-1-méthyl-2-oxo-3-azabutyl)-1,4,7-α,α',α"-triméthyl-1,4,7-tris(carboxyméthyl)-1,4,7,10-tétraazacyclododécane, 10-(4-carboxy-1-méthyl-2-oxo-3-azabutyl)-1,4,7-α,α',α"-tris(isopropyl)-1,4,7-tris(carboxyméthyl)-1,4,7,10-tétraazacyclododécane, 10-(4-carboxy-1-méthyl-2-oxo-3-azabutyl)-1,4,7-α,α',α"-tris(cyclohexyl)-1,4,7-tris(carboxyméthyl)-1,4,7,10-tétraazacyclododécane, 10-(4-(t-butoxycarbonyl-1-phényl-2-oxo-3-azabutyl)-1,4,7-α,α',α"-triméthyl-1,4,7-tris(carboxyméthyl)-1,4,7,10-tétraazacyclododécane, 10-(4-(t-butoxycarbonyl-1-phényl-2-oxo-3-azabutyl)-1,4,7-α,α',α"-triphényl-1,4,7-tris(carboxyméthyl)-1,4,7,10-tétraazacyclododécane, 10-(4-carboxy-2-oxo-3-azabutyl)-1,4,7-α,α',α"-tris(isopropyl)-1,4,7-tris(carboxyméthyl)-1,4,7,10-tétraazacyclododécane, 10-(4-carboxy-2-oxo-3-azabutyl)-1,4,7-α,α',α"-tris(cyclohexyl)-1,4,7-tris(carboxyméthyl)-1,4,7,10-tétraazacyclododécane, 10-(4-carboxy-1-méthyl-2-oxo-3-azabutyl)-2,5,8,11-tétraméthyl-1,4,7,10-tétraazacyclododécane-1,4,7-tri-tert-butylester triacétique, 10-[8-(N-maléimido)-1-méthyl-2,5-dioxo-3,6-diazaoctyl]-1,4,7-α,α',α"-tris-(isopropyl)-1,4,7-tris(carboxyméthyl)-1,4,7,10-tétraazacyclododécane et 10-[8-(N-maléimido)-1-méthyl-2,5-dioxo-3,6-diazaoctyl]-1,4,7-α,α',α"-tris-(cyclohexyl)-1,4,7-tris(carboxyméthyl)-1,4,7,10-tétraazacyclododécane.

**11.** Composés selon l'une quelconque des revendications précédentes, dans lesquels au moins deux des radicaux Z représentent un équivalent d'ion métallique d'un élément radioactif ou paramagnétique de numéros atomiques 21 à 29, 31, 32, 37 à 39, 42 à 44, 46, 47, 49, 58 à 71, 75, 77, 82 ou 83.

**12.** Utilisation de composés de formule I :

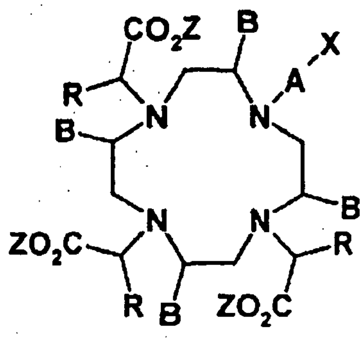

I

,

dans laquelle Z, B, R, A et X sont définis comme dans la revendication 1, à condition que B et R ne représentent pas simultanément des atomes d'hydrogène et que, si B est un atome d'hydrogène et R un radical alkyle en $C_1$-$C_4$, A ne représente pas le radical :

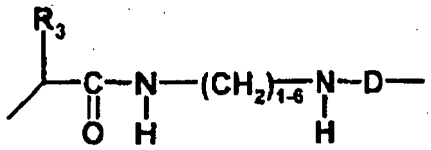

dans lequel $R_3$ est un atome d'hydrogène ou un radical alkyle en $C_1$-$C_4$, D est un groupement alkylène en $C_1$-$C_4$ saturé ou insaturé, linéaire ou ramifié, qui peut éventuellement être interrompu ou substitué par un groupement carbonyle, et D est lié à X, pour la fabrication d'un conjugué avec une biomolécule.

**13.** Utilisation selon la revendication 12, dans laquelle la biomolécule est choisie dans le groupe constitué de biopolymères, de protéines, de biopolymères modifiés par synthèse, d'hydrates de carbone, d'anticorps, de fragments d'ADN et d'ARN, d'acides β-aminés, d'amines vecteurs pour introduction dans la cellule, d'amines biogéniques, de produits pharmaceutiques, de préparations oncologiques, de polymères synthétiques, qui visent une cible biologique, de stéroïdes, de prostaglandines, de taxol et de ses dérivés, d'endothélines, d'alcaloïdes, d'acide folique et de ses dérivés, de lipides bioactifs, de graisses, d'esters d'acides gras, de mono-, di- et triglycérides modifiés par synthèse, de liposomes qui sont dérivatisés en surface, de micelles d'acides gras naturels ou de composés de perfluoroalkyle, de porphyrines, de texaphrines, de porphyrines élargies, de cytochromes, d'inhibiteurs, de neuramidases, de neuropeptides, d'immuno-modulateurs, d'endoglycosidases, de substrats, qui sont attaqués par les enzymes calmoduline kinase, caséine-kinase II, gluthathion-S-transférase, héparinase, les métalloprotéases de matrice, la kinase du récepteur β de l'insuline, l'UDP-galactose 4-épimérase, les fucosidases, les protéines G, les galactosidases, les glycosidases, les glycosyle-transférases et les xylosidases, d'antibiotiques, de vitamines et d'analogues de vitamines, d'hormones, d'intercalaires d'ADN, de nucléosides, de nucléotides, de lectines, de vitamine B12, de Lewis X et apparentés, de psoralènes, d'antibiotiques de diènetriène, de carbacyclines, de facteur de croissance endothéliale vasculaire (VEGF), de somatostatine et de ses dérivés, de dérivés de biotine, d'anti-hormones, de protéines et de produits de synthèse spécifiques aux tumeurs, de polymères qui s'accumulent dans les zones acides ou basiques du corps, de myoglobines, d'apomyoglobines, de peptides neurotransmetteurs, de facteurs de nécrose tumorale, de peptides qui s'accumulent dans les tissus enflammés, de réactifs de compartiments sanguins, de protéines de transport d'anions et de cations, de polyesters, de polyamides et de polyphosphates.

**14.** Procédé de fabrication de composés de formule I :

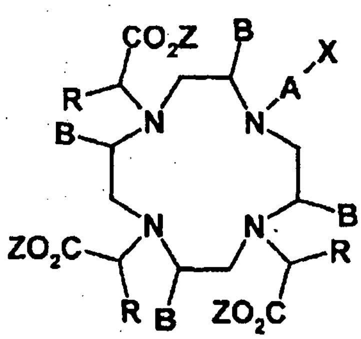

I

dans laquelle Z, B, R, A et X sont définis comme dans la revendication 1, à condition que B et R ne représentent pas simultanément des atomes d'hydrogène et que, si B est un atome d'hydrogène et R un radical alkyle en $C_1$-$C_4$, A ne représente pas le radical :

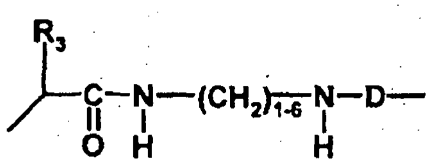

dans lequel $R_3$ est un atome d'hydrogène ou un radical alkyle en $C_1$-$C_4$, D est un groupement alkylène en $C_1$-$C_4$ saturé ou insaturé, linéaire ou ramifié, qui peut éventuellement être interrompu ou substitué par un groupement carbonyle, et D est lié à X, dans lequel on fait réagir un composé de formule II :

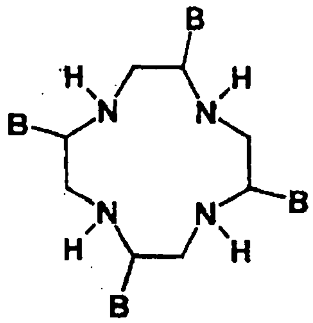

II

dans laquelle B est défini comme dans la revendication 1 et réagit éventuellement, après introduction de groupements protecteurs pour les atomes d'azote, avec Nu-A-X' et Nu-CH(R)-$CO_2$Z', où A et R sont définis comme dans la revendication 1 et Nu est un nucléofuge, X' représente X ou une forme protégée de X et X est défini comme dans la revendication 1 et Z' représente un atome d'hydrogène, un équivalent d'ion métallique ou un groupement protecteur pour le carboxyle, puis on élimine les groupements protecteurs éventuellement présents et on fait réagir, si on le souhaite, de manière connue en soi avec au moins un oxyde métallique ou un sel métallique d'un élément souhaité et on substitue ensuite éventuellement, totalement ou partiellement, dans les complexes ainsi obtenus les atomes d'hydrogène acides encore présents par des cations de bases inorganiques et/ou organiques, des acides aminés ou des amides d'acides aminés.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9531444 A
- WO 0108712 A
- WO 9702051 A
- EP 0565930 A
- WO 9905145 A
- WO 9905128 A
- WO 9517910 A
- WO 9221379 A
- EP 0481420 A
- EP 0353450 A
- EP 71564 A
- EP 130934 A
- DE OS3401052 A
- WO 9623526 A
- WO 9824774 A
- WO 9824775 A
- WO 9311152 A, Teger-Nilsson
- DE 19608307, Petrov


### In der Beschreibung aufgeführte Nicht-Patentliteratur


- **P. CARAVAN et al.** *Chem. Rev.,* 1999, vol. 99, 2293-2352
- **M.D. OGAN et al.** *Invest. Radiol.,* 1987, vol. 22, 665-671
- **U. SCHMIEDL et al.** *Radiology,* 1987, vol. 162, 205-210
- **M. P. LOWE et al.** *J. Am. Chem. Soc.,* 2001, vol. 123, 7601
- **M. P. LOWE et al.** *Inorganica Chemica Acta,* 2001, vol. 317, 163
- **M. WOODS et al.** *J. Am. Chem. Soc.,* 2000, vol. 122, 9781
- *Chemical Journal of Chinese Universities,* 1996, vol. 17, 1179
- **S.I. KANG et al.** *Inorg. Chem.,* 1993, vol. 32, 2912
- **M. UND A. BODANSZKY.** The Practice of Peptide Synthesis. Springerverlag, 1984
- Radiotracers for Medical Applications. CRC Press, vol. I
- Radiotracers for Medical Applications. CRC-Press, 1983
- *Eur. J. Nucl. Med.,* 1990, vol. 17, 346-364
- *Chem. Rev.,* 1993, vol. 93, 1137-1156
- **HEISS, W.D. ; PHELPS, M.E.** Positron Emission Tomography of Brain. Springer Verlag, 1983
- **R. L. MILLS et al.** *Nature,* 1988, vol. 336, 787
- **H. NEWMAN.** *J. Org. Chem.,* 1965, vol. 30, 287
- **M.A. SCHWARTZ et al.** *J. Am. Chem. Soc.,* 1973, vol. 95, G12
- **M. IMAZAMA ; F. ECKSTEIN.** *J. Org. Chem.,* 1979, vol. 44, 2039
- **B.F. LUNDT et al.** *J. Org. Chem.,* 1978, vol. 43, 2285
- **W. H. HARTUNG ; R. RIMONOFF.** *Org. Reactions,* 1953, vol. II, 262
- **L. ZERVAS et al.** *J. Am. Chem. Soc.,* 1956, vol. 78, 1359
- **M. BERGMANN ; L. ZERVAS.** *Ber.,* 1932, vol. 65, 1192
- **H. BERTHOLD et al.** Institut für Organische Chemie. *TU-Graz,* 2001
- **H.-J. WEINMANN et al.** *Am. J. of Roentgenology,* 1984, vol. 142, 619
- **R. W. KOZAK et al.** *TIBTEC,* Oktober 1986, 262
- *Bioconjugate Chem.,* 2001, vol. 12, 7-34
- **KITAZAKI et al.** *Chem. Pharm. Bull.,* 1999, vol. 47 (3), 360
- **WALKER et al.** *Tetrahedron,* 1997, vol. 53 (43), 14591
- **QABAR et al.** *Tetrahedron Letters,* 1998, vol. 39 (33), 5895
- **ARANO et al.** *J. Med. Chem.,* 1996, vol. 39, 3458
- **GRAMAIN et al.** *Synth. Commun.,* 1997, vol. 27, 1827
- **KITAZAKI et al.** *Chem. Pharm. Bull.,* vol. 47 (3), 360
- **OKAWARA et al.** *Chem. Pharm. Bull.,* 1982, vol. 30, 1225